# EUROPEAN PATENT APPLICATION

(11) **EP 4 563 620 A1**
(43) Date of publication of application: **04.06.2025**
(21) Application number: 23845447.4
(22) Date of filing: 21.07.2023
(51) Int. Cl.: C08G 69/08, C08G 65/333, A61L 24/10, A61K 39/395, A61K 47/34, C07K 16/46, C12P 21/08

(54) **THREE-ARM POLYETHYLENE GLYCOL DERIVATIVE CONTAINING AMINO ACID RESIDUES**

(30) Priority: 28.07.2022 CN 202210901488
(71) Applicant: Xiamen Sinopeg Biotech Co., Ltd., Xiamen, Fujian 361100 (CN)
(72) Inventor: WENG, Wengui, Xiamen, Fujian 361100 (CN); LIU, Chao, Xiamen, Fujian 361100 (CN); WANG, Ailan, Xiamen, Fujian 361100 (CN); CHEN, Dandan, Xiamen, Fujian 361100 (CN); LIN, Sheng, Xiamen, Fujian 361100 (CN); ZHU, Qi, Xiamen, Fujian 361100 (CN)
(74) Representative: Algemeen Octrooi- en Merkenbureau B.V.
(86) International application number: PCT/CN2023/108573
(87) International publication number: WO 2024/022237

(57) **Abstract**

The present application discloses a three-arm polyethylene glycol derivative containing amino acid moieties (Formula 1), wherein nᵢ is the degree of polymerization and is an integer from 30 to 1000; R₂ is C₁₋₆ alkyl; L_{1A}, L_{1B}, and L₂ are divalent linking groups; Lys is a lysine residue; AA₁ and AA₂ are residues of amino acids or amino acid derivatives; p is an integer from 1 to 5, with -(AA₁)ₚ- representing a divalent residue formed by p AA₁ units; q is 0 or 1, indicating the absence or presence of AA₂; L₃ is a linking bond or divalent linking group; R₀₁ is H or a functional group capable of reacting with bio-related substances, optionally protected; and m is 1 or 2. This derivative is degradable within cells, prevents vacuole formation at higher molecular weights, and can modify drugs or bio-related substances, offering high biocompatibility, low toxicity, and excellent drug activity retention.

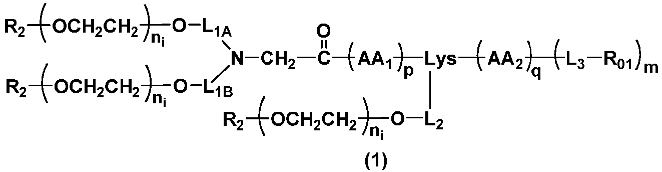

## Description

### TECHNICAL FIELD

The present invention relates to a polyethylene glycol derivative, especially a three-arm polyethylene glycol derivative containing amino acid residues, which is stable in the systemic circulation, degradable within cells, and can be used for modifying drugs or bio-related substances.

### BACKGROUND

Polyethylene glycol (PEG) is widely used in the surface modification of drugs or drug carriers. Under physiological conditions, the hydrated films formed by PEG can effectively protect the modified substances, prevent the modified substances from rapid degradation or clearance, and effectively reduce immunogenicity and systemic toxicity of the modified substances. Moreover, PEG modification can effectively address the poor water solubility of some drugs. Polyethylene glycol-modified drugs or bio-related substances are generally obtained by coupling reactions that form covalent bonds between the active groups of functionalized polyethylene glycol derivatives and drug molecules (including protein drugs and organic small molecule drugs), peptides, carbohydrates, lipids, oligonucleotides, affinity ligands, cofactors, liposomes, biological materials, etc.

In 1990, Adagen, the first PEGylated protein drug developed by Enzon, was approved by FDA for marketing. To date, various types of PEGylated drugs have been approved for sale or are in clinical stages. Overall, the safety of PEGylated drugs has been clinically validated. However, higher molecular weight PEG may induce the formation of cell vacuoles, and most PEG-associated vacuolation occurs when the molecular weight of PEG is ≥30 kDa (Toxicol. Pathol. 2015, 43, 959-983). Research data suggest that PEG-associated vacuolation may be reversible; however, the recovery periods in most nonclinical toxicology studies are usually too short to fully confirm this reversibility. Larger vacuoles or multiple vacuoles may take longer to disappear during the absence of treatment. The formation of vacuoles in phagocytes caused by PEG or PEGylated drugs is considered a normal physiological response. The PEG taken up by phagocytes may eventually be cleared from the body through organs such as the liver, kidneys, or spleen, but it may also persist throughout the cells' entire life cycle. The lifespan of Kupffer cells is as long as 5 weeks to 14 months, suggesting that the cell vacuoles caused by high molecular weight PEG may persist in the human body for an extended period, and its long-term effects remain unclear. Although reducing the molecular weight of PEG can effectively prevent the generation of cell vacuoles, it may also diminish the protective effect of PEG on the modified drugs.

In recent years, multi-arm polyethylene glycols, such as three-arm polyethylene glycol, four-arm polyethylene glycol, six-arm polyethylene glycol, eight-arm polyethylene glycol, etc., have begun to establish a presence in the market due to their advantages in structure and drug loading capacity. Traditional linear structures, whether monofunctional or bifunctional, can improve solubility and reduce toxic side effects of drug molecules; however, these drug molecules may be embedded in PEG chains, resulting in significantly diminished activity. Compared with linear structures, polyethylene glycol derivatives with multi-arm branched structures can simultaneously achieve high solubility, low toxic side effects, low antigenicity and high retention of drug activity. In addition, the system viscosity of multi-arm structures is greatly reduced, facilitating improved pharmacokinetics. Branched polyethylene glycol can form an umbrella-shaped protective layer on the drug's surface, further enhancing the stability of the drug during systemic circulation. However, in the presence of multi-arm PEG with higher molecular weight, the formation of cell vacuoles is also exacerbated.

To address the aforementioned issues, it is necessary to develop monofunctional and bifunctional multi-arm polyethylene glycol derivatives that are stable in the systemic circulation and degradable within cells.

### SUMMARY

The present invention provides novel three-arm monofunctional and bifunctional polyethylene glycol derivatives for the modification of drugs or bio-related substances. The three-arm polyethylene glycol derivatives of the present invention demonstrate stability in systemic circulation while being degradable intracellularly, offering advantages for the modified substances, including excellent protection, prolonged systemic circulation time, low toxicity, good water solubility, intracellular degradability, and high retention of drug activity.

The aforementioned objectives of the present invention are achieved through the following embodiments:

### An embodiment of the present invention is:

A three-arm polyethylene glycol derivative having the structure represented by general formula (1): 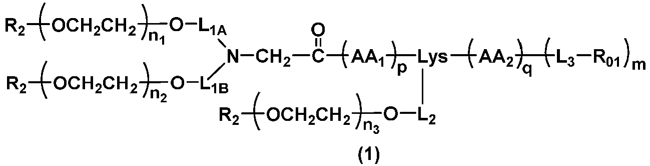
or a pharmaceutically acceptable salt, tautomer, stereoisomer or solvate thereof;
wherein,
n₁, n₂, and n₃ are the degrees of polymerization of the polyethylene glycol chains, each being independently an integer in the range of 30 to 1000;
R₂ is, at each occurrence, independently a C₁₋₆ alkyl group;
L_{1A}, L_{1B}, and L₂ are each independently a divalent linking group;
Lys is a residue of lysine;
AA₁ and AA₂ are each independently a residue of amino acid or amino acid derivative; p is an integer in the range of 1 to 5, and -(AA₁)ₚ- represents a divalent residue formed by the combination of p units of AA₁; q is 0 or 1, indicating the absence of AA₂ or the presence of one AA₂;
L₃ is a linking bond or a divalent linking group;
R₀₁ is H, a functional group capable of reacting with bio-related substances, or a protected form of the functional group; m is 1 or 2; when m is 2, the structures of two L₃ are identical or different, and the structures of two R₀₁ are identical or different;
the three-arm polyethylene glycol derivative is monodisperse or polydisperse.

### The present invention also provides another embodiment:

A bio-related substance modified with a three-arm polyethylene glycol derivative, having the structure represented by general formula (5):

P-L-D (5)

wherein, P is a residue of any aforementioned three-arm polyethylene glycol derivative, D is a residue of bio-related substance, and L is a divalent linking group formed by reacting the functional group of the three-arm polyethylene glycol derivative with the bio-related substance;
the bio-related substance is selected from the group consisting of drugs, proteins, polypeptides, oligopeptides, protein mimics, protein fragments, enzymes, antigens, antibodies, antibody fragments, receptors, aptamers of gene-related substances, polysaccharides, proteoglycans, glycoproteins, lipid compounds, hormones, vitamins, vesicles, liposomes, dyes, fluorescent substances, targeting factors, cytokines, neurotransmitters, extracellular matrix substances, plant or animal extracts, viruses, vaccines, cells, and micelles; preferably, the bio-related substance is selected from the group consisting of small molecule drugs, nucleic acids, steroids, phospholipids, and glycolipids; more preferably, the bio-related substance is selected from the group consisting of small molecule drugs, nucleosides, nucleotides, oligonucleotides, antisense oligonucleotides, polynucleotides, and steroids; furthermore, the small molecule drugs are preferably selected from the group consisting of flavonoids, terpenoids, carotenoids, saponins, steroids, sterols, quinones, anthraquinones, fluoroquinones, coumarins, alkaloids, porphyrins, polyphenols, macrolides, monobactams, phenylpropanoid phenols, anthracyclines, and aminoglycosides.

### Compared with the prior art, the present invention offers the following advantageous effects:

The present invention provides a novel three-arm polyethylene glycol derivative with a monofunctional or bifunctional asymmetric three-arm PEG structure, incorporating both carbon-branched and nitrogen-branched moieties, providing more flexibility in the modification of drugs or bio-related substances. The three-arm PEG of the present invention not only improves the biocompatibility and in vivo stability of drugs or bio-related substances but also, compared with nitrogen-branched V-shape PEGs and carbon-branched multi-arm PEGs lacking amino acid residues in the prior art, contains an oligopeptide residue composed of amino acid residues, which imparts intracellular degradability as well as the ability to inhibit the formation of cell vacuoles even at higher molecular weights (30 kDa and above). Additionally, the three-arm polyethylene glycol derivative of the present invention can be used for modifying drugs or bio-related substances to realize advantages such as excellent protection, prolonged systemic circulation time, high biocompatibility, low toxicity, high retention of drug activity, etc.

### DETAILED DESCRIPTION

The present application provides a detailed description for specific embodiments; however, it should be understood that the description is illustrative rather than restrictive. Any changes or modifications that fall within the scope of the present invention will be obvious to those skilled in the art.

If a reference cited herein contains a description that differs from the description of the present invention, the description of present invention shall prevail. This principle applies to all references cited throughout the entire specification.

### 1.1 Terminology

In the present invention, unless otherwise described, all technical and scientific terms used herein have the same meanings as commonly understood by those of ordinary skill in the art. The disclosures of all patents and other publications cited herein are incorporated by reference in their entireties. In the event that any description or interpretation of a term herein conflicts with any document incorporated herein by reference, the following description or interpretation of the term shall prevail. Unless otherwise specified, each term has the following meanings.

In the present invention, unless otherwise specified, any two objects are independent from each other. For example, "L₁ and L₂ are selected from linking bonds or alkylene groups" indicates that L₁ and L₂ are each independently a linking bond or an alkylene group. When multiple levels of selections (including preferred selections) are present, the selections for any two objects may be from the same level or from different levels. For example, "L_{A} and L_{B} are each independently selected from A, B, and C" indicates that both L_{A} and L_{B} can be A, or that L_{A} is A while L_{B} is B₁ (B₁ is a subclass of B). For example, "L_{A} is preferably A (level 1 preferred selection), more preferably selected from A₁~A₃ (level 2 preferred selection), and most preferably selected from A₁₁~A₁₃ (level 3 preferred selection); L_{B} is preferably B (level 1 preferred selection), more preferably selected from B₁~B₃ (level 2 preferred selection), and most preferably selected from B₁₁~B₁₃ (level 3 preferred selection)" indicates that a preferred situation can be, that A is selected from A₁~A₃ (level 2 preferred selection) while B is selected from B₁₁~B₁₃ (level 3 preferred selection), or that A and B are both selected from their respective level 3 preferred selections.

In the present invention, the phrase "each independently" may be used not only to describe different objects but also, when combined with "at each occurrence" to describe the same object occurring at different instances. For example, "the divalent group is selected from the group consisting of -NR_{c}C(=O)-, -C(=O)NR_{c}-, -NR_{c}C(=O)NRₑ-, -OC(=O)NR_{c}-, -NR_{c}C(=O)O-, -SC(=O)NR_{c}-, -NR_{c}C(=O)S-, -C(Rₑ)=N-NRₑ-, and -NR_{c}-N=C(R_{c})-; wherein, R_{c} is, at each occurrence, independently a hydrogen atom or a C₁₋₁₂ alkyl group" indicates that R_{c}, whether present in different or the same divalent linking group as mentioned, is each independently selected; for example, the two R_{c} groups may be the identical or different in "-NR_{c}-N=C(R_{c})-" (e.g., one R_{c} is a methyl group while the other R_{c} is a hydrogen atom or an ethyl group); for example, any R_{c} in "-NR_{c}C(=O)NR_{c}-" may be the same as or different from the R_{c} in "-NR_{c}C(=O)O-".

In the present invention, a "combination" of listed items refers to one composed of any two or more of the aforementioned listed items. In any "combination", any listed item may appear once or multiple times, in the same or different forms. For example, "L is selected from the group consisting of a linking bond, a hydrocarbylene group, -O-, -S-, -C(=O)-, -NH-, and combinations thereof" indicates that L can be any individual item from the list or a combination of any two or more of the listed items. Such combinations may include, for example, -CH₂-O-CH₂- (a combination of one -O- and two hydrocarbylene groups, wherein the hydrocarbylene groups are specifically two methylene groups), -CH₂-NH-CH₂CH₂- (a combination of one -NH- and two hydrocarbylene groups, wherein the hydrocarbylene groups are specifically one methylene group and one ethylene group), -S-S- (a combination of two -S-), -OC(=O)- (a combination of one -O- and one -C(=O)-), etc.

In the present invention, unless otherwise specified, the terms "include", "contain", and similar expressions in the specification and claims herein shall be interpreted as "include(s) but is/are not limited to" or "include(s) without limitation" in an open and inclusive manner.

In the present invention, when a certain object "includes but not be limited to" a certain range, it indicates that the object may be selected from but not limited to the range, while those explicitly excluded by the present invention shall not be selected, with the smooth implementation of the present invention serving as the screening criterion.

In the present invention, numerical ranges may be denoted by a hyphen (e.g., 1-6), a tilde (e.g., 1~6), or the word "to" (e.g., 1 to 6). Unless otherwise specified, all numerical ranges, including but not limited to those represented by integer, non-integer, percentage, and fractional ranges, include both endpoints. For example, the "integer in the range of 1-3" refers to the group consisting of 1, 2, and 3. Unless otherwise specified, a numerical range for an average value refers to the group consisting of all integers and non-integers within the range. For example, "the average number of EO units is selected from 22~100" indicates that the average number of EO units may be any integer (e.g., 22) or non-integer (e.g., 22.1) within the range. Unless otherwise specified, a numerical range for the number of groups refers to the group consisting of all integers within the range. For example, -(CH₂)₁₋₄- refers to the group consisting of -CH₂-, -(CH₂)₂-, -(CH₂)₃-, and -(CH₂)₄-. For example, 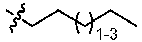 refers to the group consisting of 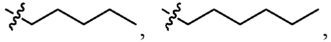 and

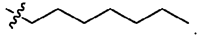

In the present invention, when referring to the molecular weight of polymers, the terms "about" and "approximately" generally indicate a numerical range of ±10%, which in some cases can be extended to ±15%, but not exceeding ±20%. For example, "approximately 10 kDa" generally refers to 9~11 kDa, and in some cases may refer to 8.5~11.5 kDa, but not exceeding 8~12 kDa. For example, when the molecular weight of a certain PEG component is about 5 kDa, it is allowed to be in the range of 5 kDa±10%, i.e., 4500~5500 Da.

In the present invention, with respect to percentages, the terms "about" and "approximately" generally indicate a numerical range of ±0.5*N when the given value (without "%") is accurate to N (including but not limited to 1, 0.1, 0.01, 0.001, 0.0001, etc.). For example, "approximately 1%" refers to the numerical range of 1±0.5% (i.e., 0.5%-1.5%), and "approximately 2.2%" refers to the numerical range of 2.2±0.05% (i.e., 2.15%-2.25%, wherein 2.2 is accurate to 0.1, and "approximately" refers to ±0.5*0.1, i.e., ±0.05).

In the present invention, unless otherwise specified, when a divalent linking group connects other groups, either of its two connecting ends can be selected. For example, when an amide bond is used as a divalent linking group between GroupA and GroupB, the specific configuration can be GroupA-C(=O)NH-GroupB or GroupA-NHC(=O)-GroupB.

In the present invention, a linking bond may be denoted by " 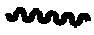 ". For example, 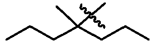 represents the monovalent group -CCH₃(CH₂CH₂CH₃)₂, and 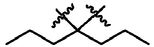 represents the divalent group -C(CH₂CH₂CH₃)₂-, while 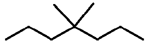 represents the compound (CH₃)₂C(CH₂CH₂CH₃)₂.

In the present invention, with respect to a linking bond or group extending from a cyclic structure, when not marked on a specific ring atom but instead pointing towards the interior of the ring, it indicates that the linking bond or group can extend from any appropriate ring atom. Moreover, when the marked ring is part of a fused ring structure, the linking bond or group may extend from any appropriate ring atom of the fused ring structure. For example, in 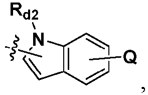 the linking bond and the group Q, both marked by " 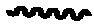 ", can extend from two different carbon ring atoms except the nitrogen atom and the bridgehead carbon atoms.

In the present invention, the range of the number of carbon atoms of a group can be denoted in the subscript of "C", and unless otherwise specified, the number of carbon atoms includes no contribution from substituents. For example, C₁₋₁₂ indicates "having 1 to 12 carbon atoms". For example, the C₁₋₁₀ alkylene group represents any alkylene group having the number of carbon atoms in the range of 1 to 10, which can be any selected from the group consisting of C₁, C₂, C₃, C₄, C₅, C₆, C₇, C₈, C₉, and C₁₀ alkylene groups, with a linear, branched, or ring-containing structure. For example, the "substituted C₁₋₁₂ alkyl group" refers to the group obtained by replacing at least one hydrogen atom in the C₁₋₁₂ alkyl group with a substituent, wherein the number of carbon atoms in the substituent is not particularly limited.

In the present invention, unless otherwise specified, a structure having isomers may refer to any of its isomers. For example, a structure having *cis*/*trans* isomers may refer to either the *cis* or *trans* form. For example, a structure having E/Z isomers may refer to either the *E* or *Z* form. For example, a structure having optical activity may refer to either the levorotatory or dextrorotatory form.

In the present invention, if there is any inconsistency between a structure and its name, the structure shall take precedence.

In the present invention, the molecular weight of polyethylene glycol and its derivatives refers to the average molecular weight by default. Unless otherwise specified, the average molecular weight generally refers to the number average molecular weight (Mₙ). The molecular weight of polyethylene glycol chain can also be expressed by the "degree of polymerization" which is, specifically, the number of repeat units (oxyethylene units). Unless otherwise specified, the degree of polymerization refers to the average degree of polymerization and by default the number average degree of polymerization.

In the present invention, the degree of polymerization of polyethylene glycol chain is represented by n; (i is a natural number selected from 1, 2, 3, ...). nᵢ of the same value in the same structure can be used interchangeably; for example, when n₂≈n₃, n₂ can be represented by n₃, or vice versa.

In the present invention, the phrase "any appropriate" in expressions such as "any appropriate linking group", "any appropriate reactive group", etc., indicates that the described subject adheres to the basic principles of chemical structures and does not impede the effective implementation of the present invention. Chemical structures described in this manner can be regarded as having a clear and defined scope.

In the present invention, the terms "stable" and "degradable", as applied to a group, are a pair of relative concepts.

In the present invention, the term "degradable" means that a linking group can be transformed into at least two independent residues through the cleavage of chemical bonds. If a linking group remains in a complete form even with its structure altered after chemical changes, then it is still within the scope of being "stable". The environments for applying the term "degradable" are not particularly limited, preferably physiological environments in vivo or simulated physiological environments in vitro. The physiological environments are not particularly limited, including but not limited to serum, heart, liver, spleen, lungs, kidneys, bones, muscles, fat, brain, lymph nodes, small intestine, gonads, etc., which may involve intracellular conditions or extracellular matrices, and may also involve normal physiological tissues or pathologic tissues (e.g., tumors, inflamed tissues, etc.). The simulated physiological environment in vitro is not particularly limited, including but not limited to physiological saline, buffer solutions, culture media, etc. The degradation is not particularly limited with respect to the rate, such as rapid degradation via enzymolysis, slow degradation in physiological environments, etc. The physiological environments in vivo include physiological conditions during treatment, such as ultraviolet radiation, hyperthermia, etc. The degradation can occur under conditions including but not limited to light, heat, low temperature, enzymes, redox reactions, acidity, basicity, physiological environment in vivo, simulated physiological environment in vitro, etc., preferably light, heat, enzymes, redox reactions, acidity, basicity, etc. The light includes but is not limited to visible light, ultraviolet light, infrared light, near-infrared light, mid-infrared light, etc. The heat refers to temperatures above normal physiological temperatures, generally above 37°C and below 45°C, with a further preference for temperatures below 42°C. The low temperature refers to temperatures below physiological temperatures of human body, preferably below 25°C, more preferably ≤10°C, with specific examples such as refrigeration temperatures, freezer temperatures, temperatures for liquid nitrogen treatment, 2~10°C, 4~8°C, 4°C, 0°C, -20±5°C, etc. The enzymes are not particularly limited, and all enzymes that can be physiologically generated are included, e.g., peptidases, proteases, lyases, etc. The redox reactions are not particularly limited, e.g., the redox transformation between a mercapto group and a disulfide bond, and hydrogenation-reduction transformations. The acidity and basicity primarily refer to the pH conditions of internal sites such as normal tissues, pathologic tissues, organs or tissues undergoing treatment, etc.; for example, the acidic condition in the stomach, and the acidic condition in tumor sites. The degradation can also occur through metabolism in vivo (e.g., physiological actions, enzymatic reactions, redox reactions, etc.), in specific in vivo sites under microenvironmental stimulations (e.g., acidity and basicity), or under clinical therapeutic stimulations (e.g., light, heat, low temperature), etc. It should be noted that a linking group which is cleavable only under some conditions that are extreme for living organisms (e.g., strong acidity, strong basicity, high temperatures such as above 100°C) is not included in the scope of degradable linking groups of the present invention. For example, although an ether bond can be cleaved under strong acid conditions such as hydrobromic acid, it is always classified as a stable linking group in the present invention.

In the present invention, the term "stable" indicates that a linking group can remain complete as a linking group (i.e., stably covalently bonded to adjacent groups), allowing chemical changes that do not compromise the overall integrity of the linking group. The chemical changes are not particularly limited, including but not limited to isomerization, oxidation, reduction, ionization, protonation, deprotonation, substitution reactions, etc. The conditions under which the linking group remains stable are not particularly limited, including but not limited to light, heat, low temperature, enzymes, redox reactions, neutrality, acidity, basicity, physiological environment in vivo, simulated physiological environment in vitro, etc., preferably light, heat, enzymes, redox reactions, acidity, basicity, etc. The stable existence described herein indicates that, under conditions without particular stimulations (e.g., pH in particular sites; light, heat, and low temperature during treatment; etc.), the stable bonding can be maintained in the metabolic cycle in vivo, and the molecular weight is not reduced due to bond cleavage (as long as the overall integrity is maintained).

In the present invention, linking groups are not absolutely "stable" or "degradable". For example, an amide bond is much more stable than an ester bond under acidic or basic conditions, and "stable" linking groups of the present invention include the amide bond. However, the peptide bond, for example, is a type of amide bond formed via the dehydration condensation of the α-carboxyl group of one amino acid molecule with the α-amino group of another amino acid molecule, which can be cleaved under specific enzymatic conditions and, therefore, is also classified as a "degradable" linking group. Similarly, carbamate groups, thiocarbamate groups, etc., can act as either stable or degradable linking groups. More commonly, carbamate groups, thiocarbamate groups, etc., tend to degrade slowly, while non-peptide amide bonds can remain stable in the metabolic cycle in vivo. For example, a common ester bond can degrade under acidic or basic conditions, while an ester bond within a special structure can also degrade under UV light conditions. For example, even though some linking groups can degrade in the presence of specific enzymes, they may still be regarded as stable if their circulation in clinical use (e.g., site-directed administration) does not pass through, or passes minimally through, environments where such enzymes are present.

In the present invention, unless otherwise specified, all compounds represented by general formulas should be regarded as including salts thereof. The term "salt" is selected from the group consisting of acid addition salts formed with inorganic and/or organic acids, base addition salts formed with inorganic and/or organic bases, and combinations thereof. When a compound represented by a general formula contains both a basic moiety (such as, but not limited to, pyridine or imidazole) and an acidic moiety (such as, but not limited to, carboxylic acid), a zwitterion ("inner salt") can be formed and included in the term "salt". The "salt" can be pharmaceutically acceptable (i.e., non-toxic, physiologically acceptable) or otherwise. The salts of compounds represented by general formulas can be formed by reacting the compounds with a certain amount (e.g., an equivalent) of acid or base in a medium such as a medium for salt precipitation or an aqueous medium, followed by lyophilization. Exemplary acid addition salts include acetates, adipates, alginates, ascorbates, aspartates, benzoates, benzenesulfonates, bisulfates, borates, butyrates, citrates, camphorates, camphorsulfonates, cyclopentanepropionates, digluconates, dodecyl sulfates, ethanesulfonates, fumarates, glucoheptanoates, glycerophosphates, hemi sulfates, heptanoates, hexanoates, hydrochlorides, hydrobromides, hydroiodides, 2-hydroxyethanesulfonates, lactates, maleates, methanesulfonates, 2-naphthalenesulfonates, nicotinates, nitrates, oxalates, pectinates, persulfates, 3-phenylpropionates, phosphates, picrates, pivalates, propionates, salicylates, succinates, sulfates, sulfonates, tartrates, thiocyanates, toluenesulfonates, undecanoates, etc. Exemplary base addition salts include ammonium salts, alkali metal salts (e.g., sodium, lithium, and potassium salts), alkaline earth metal salts (e.g., calcium and magnesium salts), salts with organic bases (e.g., organic amines), and salts with amino acids (e.g., arginine, lysine). Basic nitrogen-containing groups may be quaternized with agents such as lower alkyl halides (e.g., methyl, ethyl, propyl, and butyl chlorides, bromides, and iodides), dialkyl sulfates (e.g., dimethyl, diethyl, dibutyl, and diamyl sulfates), long chain halides (e.g., decyl, lauryl, tetradecyl, and stearyl chlorides, bromides, and iodides), aralkyl halides (e.g., benzyl and phenethyl bromides), etc. The acid addition salts and base addition salts are both preferably pharmaceutically acceptable salts and are considered, for the purpose of this disclosure, equivalent to the free forms of the corresponding compounds represented by general formulas.

In the present invention, heteroatoms are not particularly limited, including but not limited to O, S, N, P, Si, F, Cl, Br, I, B, etc.

In the present invention, relative to a compound, a group formed after losing part of atoms or groups of the compound is also termed a residue.

In the present invention, groups with a valence state of 2 or greater are collectively referred to as "linking groups". A linking group may consist of only a single atom, such as an ether group (-O-) or a thioether group (-S-). Particularly, a "linking bond" only acts as a linker and contains no atoms, which is not strictly classified as a group; however, when the definition of a group includes a "linking bond", it indicates that the group may be absent, with only the connection present.

In the present invention, regarding the valence of a group, the term "multivalent" indicates that the valence is at least 3.

In the present invention, the term "group" for divalent groups can be replaced with the term "bond", without altering the meanings. For example, a divalent ether group can be termed an ether bond (-O-), a divalent ester group can be termed an ester bond (-OC(=O)- or -C(=O)O-), and a divalent carbamate group can be termed a carbamate bond (-OC(=O)NH- or -NHC(=O)O-).

In the present invention, when the number of atoms in a substituent is 1, the substituent can also be termed a "substituent atom".

In the present invention, when a compound or group is "substituted", it means that the compound or group contains one or more substituents.

In the present invention, unless otherwise specified, the term "amino group" can be used interchangeably with "amine group", including monovalent, divalent, trivalent, and quadrivalent neutral or cationic groups, either substituted or unsubstituted. For example, the -NH₂ in CH₃-NH₂ can be termed an "amino group", "amine group", or "primary amine group". For example, the -NH- in CH₃-NH-CH₃ can be termed a "secondary amine group" or "secondary amino group", wherein the -NH-CH₃ can also be interpreted as a methyl-substituted amine group.

In the present invention, the term "amine group" includes but is not limited to primary amine groups, secondary amine groups, tertiary amine groups, and quaternary ammonium ions. Examples include -NRₜRₜ and -N⁺RₜRₜRₜ, wherein each Rₜ is independently a hydrogen atom or any hydrocarbon group.

In the present invention, when the valence is not specified, a "hydrocarbon group" could be a monovalent, divalent, trivalent, ..., or n-valent hydrocarbon group, wherein n is the highest possible valence of the hydrocarbon group.

In the present invention, the term "hydrocarbylene group" refers to a divalent hydrocarbon group.

In the present invention, a secondary amine bond and a hydrazine bond refer to "-NH-" and "-NH-NH-", respectively, wherein both ends are capped by carbon atoms of hydrocarbon groups, e.g., the -NH- in -CH₂-NH-CH₂- and the -NH-NH- in -CH₂-NH-NH-CH₂-. As a counterexample, -C(=O)-NH- is termed an amide bond and is not considered to contain a secondary amine bond.

In the present invention, the term "functional group" preferably refers to a reactive group, a protected reactive group, or a precursor of a reactive group, etc. The number of functional groups is at least 3 for a polyfunctional compound; for example, a polyol contains at least 3 hydroxyl groups, a polythiol contains at least 3 thiol groups, etc. What should be noted is that polyfunctional compounds may contain heterofunctional groups of different types. For example, tris(hydroxymethyl)aminomethane is a triol containing also an amino group, and citric acid is a tricarboxylic acid containing also a hydroxyl group.

In the present invention, unless otherwise specified, reactive groups involved in preparation methods also include their protected forms, and the protected forms may be deprotected at any appropriate step during the actual preparation process to obtain the corresponding reactive forms.

In the present invention, ring atoms refer to the atoms constituting the ring skeleton.

In the present invention, unless otherwise specified, the source of an amino acid is not particularly limited, which can be a natural source, a non-natural source, or a mixture of both. In the present invention, unless otherwise specified, the structural type of an amino acid is not particularly limited, which can be L-type, D-type, or a mixture of both.

In the present invention, the definitions and illustrations of the skeletons of amino acids, the skeletons of amino acid derivatives, and the skeletons of cyclic monosaccharides in documents including CN104877127A, WO/2016/206540A, CN106967213A, CN108530637A, CN108530617A, and all cited documents are hereby incorporated by reference. Unless otherwise specified, the skeletons also refer to residues. Wherein, an amino acid residue includes the amino acid with a hydrogen atom removed from its amino group and/or a hydroxyl group removed from its carboxyl group and/or a hydrogen atom removed from its thiol group and/or its amino group being protected and/or its carboxyl group being protected and/or its thiol group being protected. Imprecisely, an amino acid residue can also be termed an amino acid, including residues formed by losing a carboxylic hydroxyl group (including every carboxylic hydroxyl group at the C-terminus as well as the carboxylic hydroxyl group in the pendant group of aspartic acid or glutamic acid), a hydrogen atom in a hydroxyl group, a hydrogen atom in a phenolic hydroxyl group (e.g., tyrosine), a hydrogen atom in a thiol group (e.g., cysteine), a hydrogen atom at a nitrogen atom (including every hydrogen atom at the N-terminus as well as the hydrogen atom in an amino group of a pendant group, e.g., the hydrogen atom in the ε-amino group of lysine or ornithine, and the hydrogen atom in the amino group of the pendant ring of histidine or tryptophan), an amino group in an amide (e.g., asparagine, glutamine, etc.), an amino group in a pendant group of an guanidine, or a hydrogen atom in an amino group. Besides having the essential characteristics of an amino acid, a residue of amino acid derivative also possess the essential characteristics of a non-amino acid.

In the present invention, the term "bio-related substance" includes but is not limited to the substances described, listed, or referred to in documents including CN104877127A, WO/2016/206540A, CN106967213A, CN108530637A, CN108530617A, and all cited documents. In general, bio-related substances include but are not limited to the following substances: drugs, proteins, polypeptides, oligopeptides, protein mimics, protein fragments, =====protein analogs, enzymes, antigens, antibodies, antibody fragments, receptors, small molecule drugs, nucleosides, nucleotides, oligonucleotides, antisense oligonucleotides, polynucleotides, nucleic acids, aptamers, polysaccharides, proteoglycans, glycoproteins, steroid compounds, lipid compounds, hormones, vitamins, phospholipids, glycolipids, dyes, fluorescent substances, targeting factors, targeting molecules, cytokines, neurotransmitters, extracellular matrix substances, plant or animal extracts, viruses, vaccines, cells, vesicles, liposomes, micelles, etc. The bio-related substances also include their precursors, activated states, derivatives, isomers, mutants, analogs, mimics, polymorphs, pharmaceutically acceptable salts, fusion proteins, chemically modified substances, genetically recombined substances, etc., as well as the corresponding agonists, activating agents, activators, inhibitors, antagonists, modulators, receptors, ligands or ligand groups, antibodies, antibody fragments, acting enzymes (e.g., kinases, hydrolases, lyases, oxoreductases, isomerases, transferases, deaminases, deiminases, invertases, synthetases, etc.), enzyme substrates (e.g., coagulation cascade protease substrates, etc.), etc. The derivatives include but are not limited to the derivatives of glycosides, nucleosides, amino acids, and polypeptides. Chemically modified products with newly formed reactive groups include, the chemically modified products obtained by changing the types of reactive groups via modification, and the chemically modified products formed after introducing extra functional groups, reactive groups, amino acids, amino acid derivatives, or polypeptides, etc., all of which are within the scope of chemically modified substances of bio-related substances. Before or after bio-related substances combine with functionalized compounds (including polyethylene glycol derivatives), they are allowed to form modified or complex bio-related substances with the bound target molecules, appendages, or delivery vectors. Wherein, the pharmaceutically acceptable salts can be inorganic salts such as hydrochlorides, sulfates, and phosphates, or organic salts such as oxalates, malates, citrates, etc.

**In** the present invention, the term "drug" includes any agents, compounds, compositions, and mixtures with physiological or pharmacological effects in vivo or in vitro, and the effects are usually beneficial. The "drug" is not particularly limited with respect to the type, including but not limited to pharmaceuticals, vaccines, antibodies, vitamins, food, food additives, nutritional supplements, nutraceuticals, and other agents providing beneficial effects. The "drug" is not particularly limited with respect to the range of physiological or pharmacological effects in vivo, which may be systemic or localized. The "drug" is not particularly limited with respect to the activity, which is mainly an active substance capable of interacting with other substances, and may also be an inert substance that is non-interactive; wherein, the inert substance can be transformed into an active form by in vivo processes or certain stimulations. Wherein, the term "small molecule drug" refers to a bio-related substance with a molecular weight not exceeding 1000 Da, or a small molecule mimic or active fragment of any bio-related substance.

In the present invention, the term "oligopeptide" refers to a peptide molecule formed by two or more amino acids, and the number of amino acid residues constituting the oligopeptide is preferably 2 to 20. An "oligopeptide residue" can be monovalent, divalent, trivalent, or have a higher valence state.

In the present invention, unless otherwise specified, the term "monosaccharide group" refers to a monosaccharide residue, i.e., the monosaccharide skeleton, including open-chain monosaccharide groups and cyclic monosaccharide groups (e.g., furanose rings and pyranose rings).

In the present invention, monosaccharide groups can be selected from the residues of compounds including but not limited to monosaccharides, sugar alcohols, deoxy sugars, amino sugars, amino sugar derivatives (e.g., amide derivatives), sugar acids, and glycosides, with open-chain or cyclic structures. Examples include the amino residue formed by removing an amino hydrogen atom of an amino sugar, the acyl group formed by removing a carboxylic hydroxyl group of a sugar acid, etc. The monosaccharides include but are not limited to aldoses (polyhydroxy aldehydes) and ketoses (polyhydroxy ketones). The monosaccharides may be alkyl ether derivatives, specifically, e.g., methyl ether derivatives, and more specifically, e.g., quebrachitol. In the present invention, monosaccharide groups are not particularly limited with respect to the number of carbon atoms, including but not limited to tetroses, pentoses, hexoses, and heptoses, preferably pentoses and hexoses. Exemplary tetroses, pentoses, hexoses, heptoses, sugar alcohols, deoxy sugars, amino sugars, amide derivatives of amino sugars, sugar acids, glycosides, etc., include but are not limited to the structures disclosed in CN106967213A.

In the present invention, bio-related substances can be delivered to one or more of the following physiological sites in a patient: liver or liver cells, kidney or kidney cells, tumor or tumor cells, CNS (central nervous system, such as brain and/or spinal cord) or CNS cells, PNS (peripheral nervous system) or PNS cells, lung or lung cells, blood vessels or vascular cells, skin (such as dermis and follicles) or skin cells, eye (such as macula, fovea, cornea, retina) or eye cells, ear (such as inner ear, middle ear, and outer ear) or ear cells.

In the present invention, the term "minor modification" refers to a chemical modification process that can be realized by simple chemical reactions. The simple chemical reactions mainly include protection, deprotection, salt complexation and decomplexation, ionization, protonation, deprotonation, changing of leaving groups, etc.

In the present invention, the "minor modified form" corresponds to the "minor modification", referring to a structural form that can be transformed into the target reactive group after simple chemical reactions such as protection, deprotection, salt complexation and decomplexation, ionization, protonation, deprotonation or changing of leaving groups, etc. The changing of leaving groups, i.e., transformation of leaving groups, includes but is not limited to the transformation of an ester form to an acyl chloride form.

In the present invention, a preparation process usually requires that selectively only the target reactive group proceeds to react. To avoid the influence of irrelevant reactive groups on the target reaction, the irrelevant reactive groups are usually protected. The "protection" of a reactive group refers to the strategy for reversibly transforming the reactive group in need of protection to an inert group (i.e., non-reactive group) using particular reagents. Within a protected group, the moiety that distinguishes the protected form from the unprotected form is termed the "protecting group". For example, -OTBS is a protected form of hydroxyl group (-OH), wherein the -TBS group is the hydroxyl protecting group. The protecting groups not only remain stable during the target reaction process but also, as needed, can be removed by conventional technical means in the art.

In the present invention, the term "deprotection" refers to the process of transforming a protected group to its unprotected form.

In the present invention, the term "hydroxyl protecting group" includes all commonly used hydroxyl protecting groups in the art, preferably alkanoyl (e.g., acetyl, tert-butyryl), aromatic alkanoyl (e.g., benzoyl), benzyl, triphenylmethyl, trimethylsilyl, t-butyldimethylsilyl, allyl, acetal, or ketal groups. The removal of acetyl groups is generally carried out under basic conditions, most commonly using the ammonolysis with NH₃/MeOH or the methanolysis catalyzed by methanol anions. Benzyl groups can be easily removed via palladium-catalyzed hydrogenolysis in a neutral solution at room temperature, and can also be removed via reduction reaction with metallic sodium in ethanol or liquid ammonia. Triphenylmethyl groups are generally removed via catalytic hydrogenolysis. Trimethylsilyl groups are generally removed using reagents containing fluoride ions (e.g., tetrabutylammonium fluoride/anhydrous THF, etc.). The t-butyldimethylsilyl ether is relatively stable, which can withstand ester hydrolysis conditions with alcoholic potassium hydroxide as well as mild reduction conditions (e.g., Zn/CH₃OH, etc.), but can be removed by fluoride ions (e.g., Bu₄N⁺F⁻) in a tetrahydrofuran solution or by aqueous acetic acid at room temperature. The protection of diols preferably forms dioxolanes, dioxanes, cyclic carbonates, or cyclic borates.

In the present invention, the term "thiol protecting group" includes all commonly used thiol protecting groups in the art. Similar to hydroxyl groups, thiol groups can be protected in the form of thioethers or thioesters. Thiol protecting groups are preferably tert-butyl, benzyl, substituted benzyl, benzhydryl, substituted benzhydryl, trityl, acetyl, benzoyl, tert-butoxycarbonyl, benzyloxycarbonyl, thioacetal, or thioketal groups. The deprotection of thioethers can be realized by acid-catalyzed reduction using Na/NH₃, or by reactions with heavy metal ions (e.g., Ag⁺, Hg⁺), followed by treatment with hydrogen sulfide. Groups such as hemi-thioacetals containing S-diphenylmethyl, S-triphenylmethyl, S-2-tetrahydropyranyl, or S-isobutoxymethyl groups can be oxidized to disulfides by (SCN)₂, iodine, or thionyl chloride, and further reduced to thiols. The formation of thioester and the corresponding deprotection methods are the same as those of carboxylates.

In the present invention, the "carboxyl protecting group" can be transformed into a carboxyl group via hydrolysis or deprotection reactions. Carboxyl protecting groups are preferably alkyl (e.g., methyl, ethyl, and tert-butyl) or aralkyl (e.g., benzyl) groups, and more preferably tert-butyl (tBu), methyl (Me), or ethyl (Et) groups. In the present invention, the "protected carboxyl group" is preferably a methoxycarbonyl group, an ethoxycarbonyl group, a t-butoxycarbonyl group, or a benzyloxycarbonyl group. The carboxyl protecting groups can be removed through pyrolysis, hydrogenolysis, or acid- or base-catalyzed hydrolysis. For example, t-butyl groups can be removed under mild acidic conditions, and benzyl groups can be removed by hydrogenolysis. The reagents for the removal of carboxyl protecting groups include but are not limited to TFA, H₂O, LiOH, NaOH, KOH, MeOH, EtOH, and combinations thereof, preferably the combination of TFA and H₂O, the combination of LiOH and MeOH, or the combination of LiOH and EtOH. The deprotection of a protected carboxyl group can be carried out in the presence of a base to produce the corresponding free acid, and the base can form a pharmaceutically acceptable salt with the free acid.

In the present invention, the term "amino protecting group" is synonymous with "amine protecting group", including all the amino/amine protecting groups that are commonly used in the art, such as aryl C₁₋₆ alkyl groups, C₁₋₆ alkoxy C₁₋₆ alkyl groups, C₁₋₆ alkoxycarbonyl groups, aryloxycarbonyl groups, C₁₋₆ alkylsulfonyl groups, arylsulfonyl groups, methylsilyl groups, etc., preferably Boc (t-butoxycarbonyl), Moz (p-methoxybenzyloxycarbonyl), or Fmoc (9-fluorenylmethoxycarbonyl). The reagents for the removal of amino protecting groups include but are not limited to TFA, H₂O, LiOH, MeOH, EtOH, and combinations thereof, preferably the combination of TFA and H₂O, the combination of LiOH and MeOH, or the combination of LiOH and EtOH. The reagent for the removal of Boc is preferably TFA or HCl/EA, more preferably TFA. The reagent for the removal of Fmoc is preferably the solution of 20% piperidine in N,N-dimethylformamide (DMF).

In the present invention, the term "alkynyl protecting group" includes all commonly used alkynyl protecting groups in the art, preferably the trimethylsilyl (TMS), triethylsilyl, tert-butyldimethylsilyl (TBS), or diphenyldimethylsilyl group. The TMS-protected alkynyl groups can be easily deprotected under basic conditions, for example, K₂CO₃/MeOH or KOH/MeOH. The TBS-protected alkynyl groups can be deprotected in the solution of tetra-n-butylammonium fluoride in tetrahydrofuran (TBAF/THF).

In the present invention, the hydroxyl groups that can be protected are not particularly limited, e.g., alcoholic hydroxyl groups, phenolic hydroxyl groups, etc. The amino/amine groups that can be protected are not particularly limited, e.g., the amino/amine groups in primary amines, secondary amines, hydrazines, or amides, etc.

In the present invention, the deprotection of protected hydroxyl groups is related to the type of hydroxyl protecting group. The type of hydroxyl protecting group is not particularly limited. For example, terminal hydroxyl groups protected as benzyl ethers, silyl ethers, acetals, or tert-butyl ethers can be deprotected by corresponding methods, including but not limited to the following:

### A: Deprotection of Benzyl Ethers

The deprotection of benzyl ethers can be realized by utilizing hydrogenation with hydrogenation reducing agents and hydrogen donors. The water content in this reaction system should be less than 1% so that the reaction can proceed smoothly.

The catalyst for hydrogenation reduction is not particularly limited, preferably palladium or nickel. The catalyst carrier is not particularly limited, preferably aluminum oxide or carbon, more preferably carbon. The amount of palladium used is 1 to 100 wt% relative to the compounds containing protected hydroxyl groups, preferably 1 to 20 wt% relative to the compounds containing protected hydroxyl groups.

The reaction solvent is not particularly limited, as long as it can dissolve both the starting materials and the products, but is preferably methanol, ethanol, ethyl acetate, tetrahydrofuran, or acetic acid, more preferably methanol. The hydrogen donor is not particularly limited, but is preferably hydrogen, cyclohexene, 2-propanol, or ammonium formate, etc. The reaction temperature is preferably 25 to 40°C. The reaction time is not particularly limited, which is negatively correlated with the amount of catalyst used and is preferably 1 to 5 hours.

### B: Deprotection of Acetals and Ketals

The protection of diols preferably forms cyclic acetal or ketal compounds including but not limited to methylene acetals, ethylene acetals, 1-tert-butyl methylene acetals, 1-tert-butyl ethylene ketals, 1-phenylethylene ketals, 1-(4-methoxyphenyl)ethylene ketals, 2,2,2-trichloroethylene acetals, allyl acetals, acetonides (isopropylidene ketals), etc. The deprotection of such acetals or ketals is usually realized under acidic conditions wherein the pH of the solution is preferably 0 to 4. The acid used is not particularly limited, but is preferably acetic acid, phosphoric acid, sulfuric acid, hydrochloric acid, or nitric acid, more preferably hydrochloric acid. The reaction solvent is not particularly limited, as long as it can dissolve the reactants and the products, but is preferably water. The reaction temperature is preferably 0 to 30°C.

### C: Deprotection of Silyl Ethers

The protected hydroxyl groups in the form of silyl ethers include trimethylsilyl ether, triethylsilyl ether, tert-butyldimethylsilyl ether, tert-butyldiphenylsilyl ether, etc. The deprotection of such silyl ethers generally uses compounds containing fluoride ions, preferably tetrabutylammonium fluoride, tetraethylammonium fluoride, hydrofluoric acid, or potassium fluoride, more preferably tetrabutylammonium fluoride or potassium fluoride. The molar equivalent of the fluorine-containing compound used is 5 to 20 times that of the protected hydroxyl group, preferably 8 to 15 times. If the molar equivalent of the fluorine-containing compound used is less than 5 times that of the protected hydroxyl group, the deprotonation may be incomplete. If the molar equivalent of the deprotection reagent used exceeds 20 times that of the protected hydroxyl group, the excess reagent will complicate purification and may interfere with subsequent steps, potentially causing side reactions. The reaction solvent is not particularly limited, as long as it can dissolve the reactants and the products, but is preferably an aprotic solvent, more preferably tetrahydrofuran or dichloromethane. The reaction temperature is preferably 0 to 30°C; if the temperature is lower than 0°C, the reaction rate will be relatively slow, and the protecting group may not be completely removed.

### D: Deprotection of tert-Butyl Ethers

The deprotection of tert-butyl ethers is carried out under an acidic condition wherein the pH of the solution is preferably 0 to 4. The acid used is not particularly limited, but is preferably acetic acid, phosphoric acid, sulfuric acid, hydrochloric acid, or nitric acid, more preferably hydrochloric acid. The reaction solvent is not particularly limited, as long as it can dissolve the reactants and the products, but is preferably water. The reaction temperature is preferably 0 to 30°C.

In the present invention, the term "activation of carboxyl group" refers to the activation of carboxyl groups with carboxyl activating agents, which facilitates condensation reactions by, for example, inhibiting the generation of racemic impurities during the condensation reactions, accelerating the reactions through catalysis, etc. The "carboxyl activating group" is a residue of a carboxyl activating agent. The carboxyl activating agent is selected from the group consisting of *N*-hydroxysuccinimide (NHS), 1-ethyl-(3-dimethylaminopropyl)carbodiimide hydrochloride (EDCI HCl), *N*-hydroxy-5-norbornene-2,3-dicarboximide (HONb), *N*,*N*'-dicyclohexylcarbodiimide (DCC), and combinations thereof, preferably the combination of NHS/EDCI, NHS/DCC, or HONb/DCC, more preferably the combination of NHS/EDCI or NHS/DCC.

In the present invention, the condensing agents used in reactions are not particularly limited, preferably selected from the group consisting of *N*,*N*'-dicyclohexylcarbodiimide (DCC), 1-ethyl-(3-dimethylaminopropyl)carbodiimide hydrochloride (EDCI·HCl), *o*-(7-azabenzotriazol-1-yl)-*N*,*N*,*N*',*N*'-tetramethyluronium hexafluorophosphate (HATU), and *o*-(benzotriazol-1-yl)-*N*,*N*,*N*',*N*'-tetramethyluronium hexafluorophosphate (HBTU), most preferably DDC. Generally, the molar equivalent of the condensing agent used is 1 to 20 times that of carboxylic acids, preferably 5 to 10 times. Appropriate catalysts (e.g., 4-dimethylaminopyridine (DMAP)) can be added to condensation reactions.

In the present invention, the obtained products can be purified via methods including but not limited to extraction, recrystallization, adsorption treatment, precipitation, anti-solvent precipitation, membrane dialysis, supercritical extraction, etc.

In the present invention, reactions may be solvent-free or use aprotic solvents. The aprotic solvent includes toluene, benzene, xylene, acetonitrile, ethyl acetate, diethyl ether, methyl tert-butyl ether, tetrahydrofuran, chloroform, dichloromethane, dimethyl sulfoxide, dimethylformamide, dimethylacetamide, etc., and is preferably tetrahydrofuran, dichloromethane, dimethyl sulfoxide, or dimethylformamide.

In the present invention, the bases used in reactions can be organic bases (e.g., triethylamine, pyridine, 4-dimethylaminopyridine, imidazole, or N,N-diisopropylethylamine, preferably triethylamine or pyridine), or inorganic bases (e.g., K₂CO₃).

In the present invention, the repeat unit of polyethylene glycol is the oxyethylene unit, i.e., -CH₂CH₂O- or -OCH₂CH₂-, also termed the EO unit. The number of repeat units is also termed the number of EO units (i.e., degree of polymerization). The average number of repeat units is also termed the average number of EO units (i.e., average degree of polymerization), which refers to the number average number of EO units (number average degree of polymerization) by default, unless otherwise specified.

In the present invention, for polydisperse polymers, when terms such as "equal", "identical" or other forms of equivalent expressions are used to describe the molecular weight or degree of polymerization of a single molecule or a macroscopic component, unless otherwise specified, the terms do not indicate a strict numerical equality but a proximity or approximate equality of values; wherein, the proximity or approximate equality preferably corresponds to a deviation within ±10%. For example, if a certain polydisperse mPEG (methoxy polyethylene glycol) has a molecular weight of 5 kDa, then the molecular weight of a single molecule thereof may be in the range of 4500 to 5500 Da, the average molecular weight of a macroscopic component thereof is 4500 to 5500 Da, and its content is calculated based on the components having average molecular weights in the range of 4500 to 5500 Da.

In the present invention, for monodisperse polymers, when terms such as "equal", "identical" or other forms of equivalent expressions are used to describe the molecular weight or degree of polymerization (number of EO units) of a single molecule or a macroscopic component, a strict numerical equality is indicated. For example, if the number of EO units of a certain monodisperse PEG component is 11, then the same kind of PEG component with the number of EO units being 12 is not considered the same. However, in pursuit of a component having the target number of EO units, the macroscopic product could also contain components having other numbers of EO units due to limitations in preparation or purification methods; in such cases, if the deviation between the average number of EO units and the target number of EO units is within ±5% (for target number of EO units≥10) or within ±0.5 (for target number of EO units<10), it is considered to have obtained a monodisperse macroscopic product of the target component; moreover, with respect to a component having the target number of EO units or an average number of EO units that meets the aforementioned requirements, if its content reaches a specific percentage (preferably 90% or higher, more preferably higher than 95%, more preferably higher than 96%, more preferably higher than 98%, and most preferably 99-100%), it is also considered to have obtained a monodisperse macroscopic product of the target component; even if the aforementioned content in percentage is not met, any product obtained using the preparation method of this invention, or a similar method with essentially the same approach, is within the scope of this invention, regardless of whether the component is insufficient in content or appears as a main product, co-product, or by-product, whether or not it is separated and purified. For example, if a certain monodisperse mPEG has 22 oxyethylene units, then the actual product may be a mixture of compounds having 20, 21, 22, 23, or 24 EO units; meanwhile, if the average number of EO units is within the range of 22±2.2 (preferably within the range of 22±1.1), then the target component is considered obtained, and all the components having the number of EO units within the numerical range can be regarded as target components for calculation of purity.

In the present invention, the unit of polymer molecular weight is typically Da or kDa, and it can also be described in terms of degree of polymerization, number of repeat units, or number of EO units, etc. Generally, a specified molecular weight is a discrete value rather than a numerical range, but the actual product may have non-uniform molecular weights so that the average molecular weight (or average number of EO units, etc.) may deviate from the specified value within a certain range (as aforementioned, the deviation does not exceed ±10% for polydisperse components or ±5% for monodisperse components).

In the present invention, unless otherwise specified, the term "mPEG" refers to a polyethylene glycol chain capped with a methoxy group, having the structure of 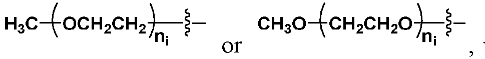 wherein nᵢ is the degree of polymerization of the polyethylene glycol chain.

In the present invention, a product with PDI<1.005 is regarded as monodisperse (PDI=1).

In the present invention, the number of repeat units in a "single-chain component" is at least 2.

In the present invention, drugs can act systemically and/or locally, and can be administered via appropriate routes, such as injection (e.g., intravenous, intraarterial, subcutaneous, intraperitoneal, and intramuscular injections, including infusion), or via transdermal, oral, buccal, transnasal, transmucosal, or topical routes, or in the form of ophthalmic preparations, or by inhalation. The pharmaceutical compositions of the present invention can be administered through these routes of administration in suitable dosage forms. The dosage forms include but are not limited to tablets, capsules, lozenges, hard sugar agents, powders, sprays, creams, ointments, suppositories, gels, pastes, lotions, ointments, aqueous suspensions, injectable solutions, elixirs, and syrups.

In the present invention, treatment refers to patient management and care in order to resist diseases, obstacles, or symptoms, which is intended to delay the development of diseases, obstacles, or symptoms, mitigate or alleviate symptoms and complications, and/or cure or eliminate diseases, obstacles, or symptoms. The patients to be treated are preferably mammals, especially humans.

### 1.2 Three-Arm Polyethylene Glycol Derivatives

### 1.2.1. Structures of General Formulas

### An embodiment of the present invention is:

A three-arm polyethylene glycol derivative having the structure represented by general formula (1): 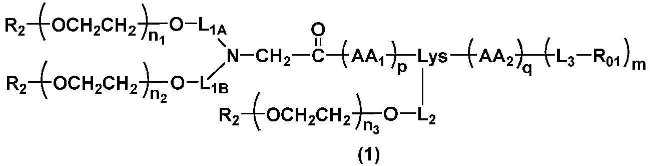
or a pharmaceutically acceptable salt, tautomer, stereoisomer or solvate thereof;
wherein,
n₁, n₂, and n₃ are the degrees of polymerization of the polyethylene glycol chains, each being independently an integer in the range of 30 to 1000;
R₂ is, at each occurrence, independently a C₁₋₆ alkyl group;
L_{1A}, L_{1B}, and L₂ are each independently a divalent linking group;
Lys is a residue of lysine;
AA₁ and AA₂ are each independently a residue of amino acid or amino acid derivative; p is an integer in the range of 1 to 5, and -(AA₁)ₚ- represents a divalent residue formed by the combination of p units of AA₁; q is 0 or 1, indicating the absence of AA₂ or the presence of one AA₂;
L₃ is a linking bond or a divalent linking group;
R₀₁ is H, a functional group capable of reacting with bio-related substances, or a protected form of the functional group; m is 1 or 2; when m is 2, the structures of two L₃ are identical or different, and the structures of two R₀₁ are identical or different;
the three-arm polyethylene glycol derivative is monodisperse or polydisperse.

In the present invention, the compound of general formula (1) may exist in unsolvated or solvated forms, including hydrated forms. In general, for the purpose of the present disclosure, solvated forms with pharmaceutically acceptable solvents (e.g., water, ethanol, etc.) are equivalent to the unsolvated form.

In the present invention, the compound of general formula (1), as well as its salts and solvates, can exist in their tautomeric forms, including those from keto-enol tautomerism, amide-imidic acid tautomerism, lactam-lactim tautomerism, enamine-imine tautomerism, proton transfer tautomerism, and valence tautomerism.

In the present invention, the compound of general formula (1) should be considered as including its pharmaceutically acceptable salts, tautomers, stereoisomers, and solvates.

In one specific embodiment of the present invention, the number average molecular weight of each PEG chain is 1000, 1500, 2000, 2500, 3000, 3350, 3500, 4000, 5000, 5500, 6000, 6500, 7000, 7500, 8000, 8500, 9000, 9500, 10000, 11000, 12000, 13000, 14000, 15000, 16000, 17000, 18000, 19000, or 20000 Da.

In one specific embodiment of the present invention, the n₁, n₂, and n₃ are each independently an integer in the range of 30 to 500, preferably in the range of 100 to 500; in a more specific embodiment of the present invention, the aforementioned n₁ and n₂ have the same value and are integers in the range of 180 to 270, preferably in the range of 200 to 250, more preferably in the range of 210 to 240, most preferably in the range of 220 to 230; the aforementioned n₃ has the same value as n₁ and n₂; or, n₃ is an integer in the range of 360 to 540, preferably in the range of 410 to 490, more preferably in the range of 430 to 470, most preferably in the range of 440 to 460.

In one specific embodiment of the present invention, the number average molecular weight of the three-arm polyethylene glycol derivative is 20 to 50 kDa, preferably 20 kDa, 25 kDa, 30 kDa, 35 kDa, 40 kDa, 45 kDa, or 50 kDa, more preferably 30 kDa, 35 kDa, or 40 kDa.

### A specific embodiment of the present invention is:

A three-arm polyethylene glycol derivative, wherein p is 1 and AA₁ is a residue of glycine, and the structure of the derivative is represented by general formula (2): 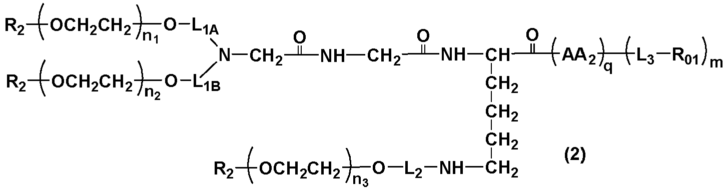 wherein,
L_{1A} and L_{1B} are each independently a C₁₋₆ alkylene group or -(CH₂)₁₋₆C(=O)-, wherein the left end of each linking group is connected to a PEG chain; more preferably, L_{1A} and L_{1B} are each independently selected from the group consisting of -CH₂CH₂-, -CH₂-, -CH₂C(=O)-, and -CH₂CH₂C(=O)-; more preferably, L_{1A} and L_{1B} conform to any one of the following cases:
   Case (1): L_{1A} and L_{1B} are each independently -CH₂CH₂- or -CH₂-;
   Case (2): one of L_{1A} and L_{1B} is -CH₂CH₂- or -CH₂-, and the other is -CH₂C(=O)- or -CH₂CH₂C(=O)-;
the rest parameters are defined as in general formula (1).

### A specific embodiment of the present invention is:

A three-arm polyethylene glycol derivative of general formula (2), wherein q is 0 and AA₂ is absent, and the structure of the derivative is represented by general formula (3): 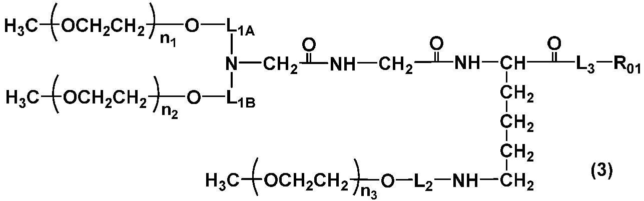
wherein, L₃ is preferably selected from the group consisting of a linking bond, -O-, -O(CH₂)ₜₚOC(=O)O-, -NH(CH₂)ₜₚ-, -NH(CH₂)ₜₚC(=O)NH(CH₂)ₜₚ-, and -NH(CH₂)ₜₚNHC(=O)(CH₂)ₜₚ-, and its right end is connected to R₀₁; tp is an integer in the range of 1 to 4, preferably 1 or 2;
R₀₁ is preferably selected from the group consisting of -OH, -CHO, -COOH, -NH₂, 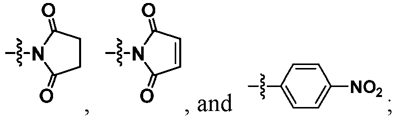
the structure of the three-arm polyethylene glycol derivative is more preferably selected from the group consisting of the following: 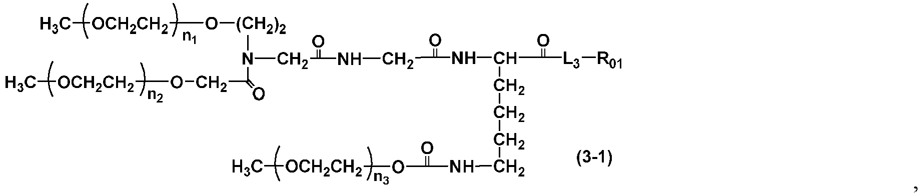 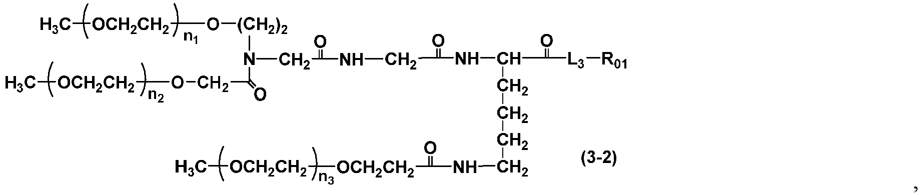 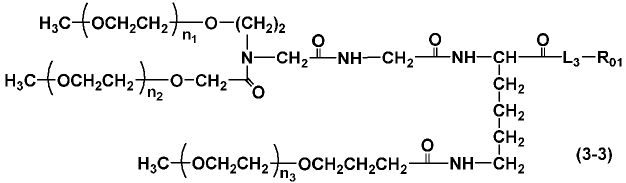 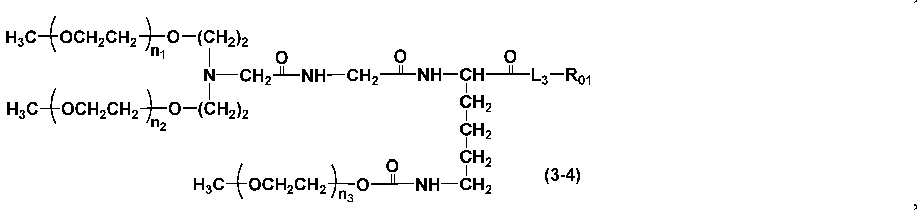 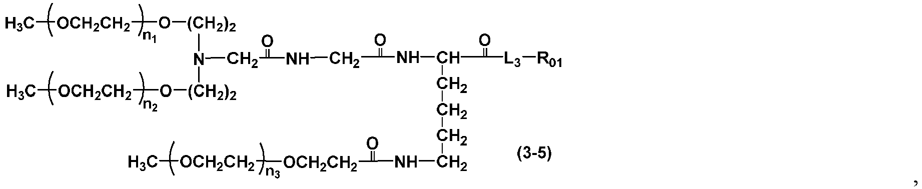 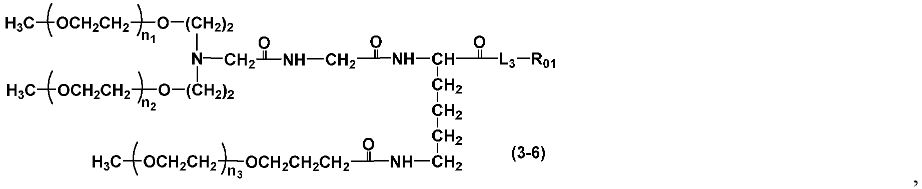 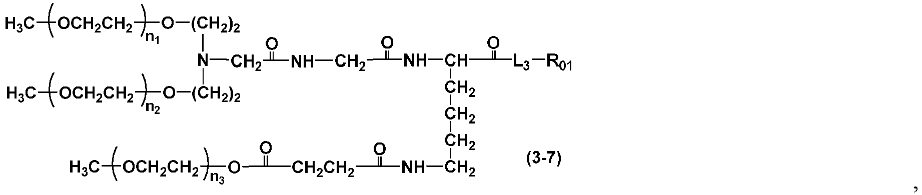 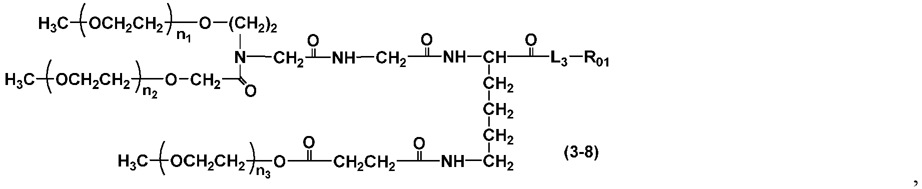 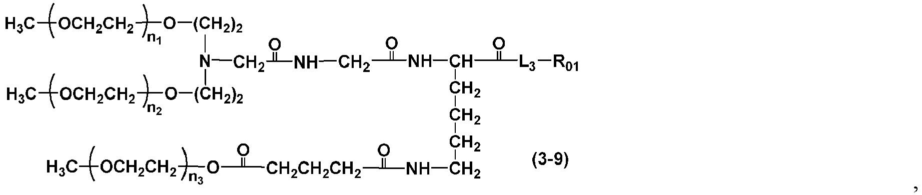 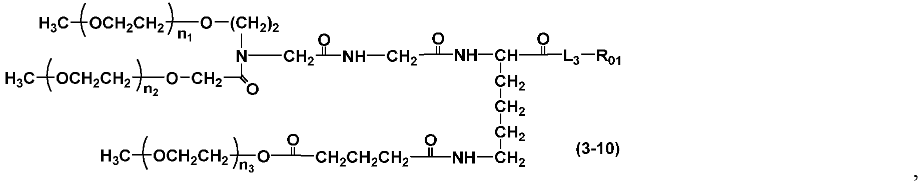 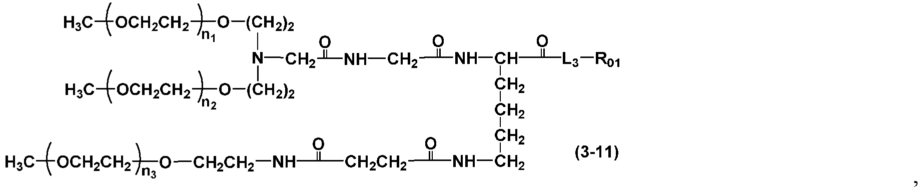 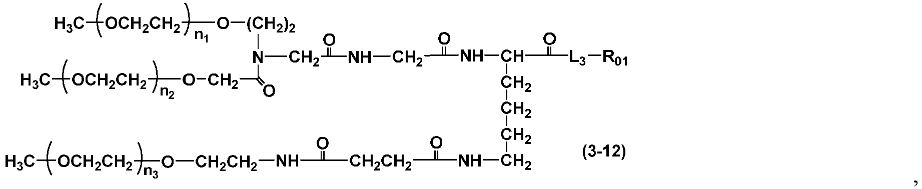 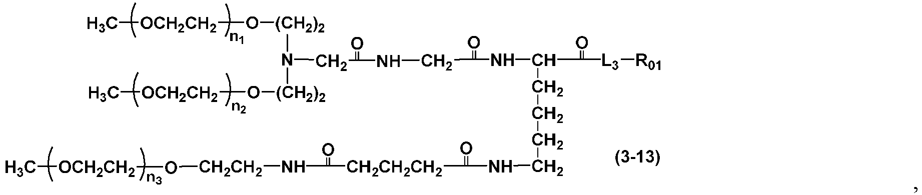 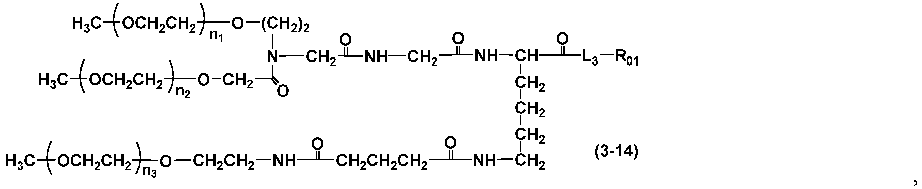 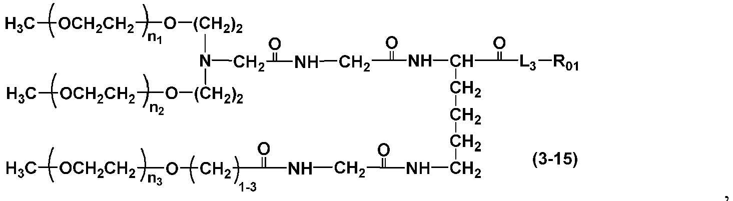 and 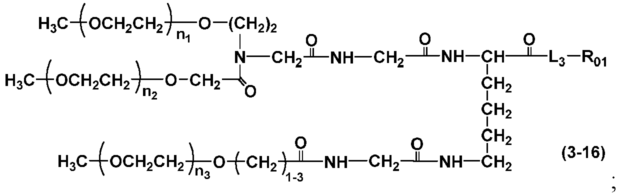
the rest parameters are defined as in general formula (2).

### A specific embodiment of the present invention is:

A three-arm polyethylene glycol derivative of general formula (2), wherein q is 1 and one AA₂ is present, and the structure of the derivative is represented by general formula (4): 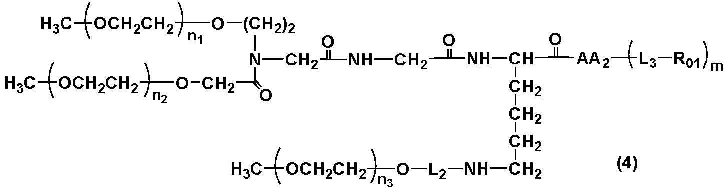 wherein,
R₀₁ is preferably -OH or -CHO;
L₃ is preferably a linking bond or -NH(CH₂)ₜₚ-, and its right end is connected to R₀₁; tp is an integer in the range of 1 to 4, preferably 1 or 2;
AA₂ is preferably a trivalent residue of amino acid or amino acid derivative, more preferably a trivalent residue of glutamic acid or aspartic acid;
the structure of the three-arm polyethylene glycol derivative is more preferably selected from the group consisting of the following structural formulas: 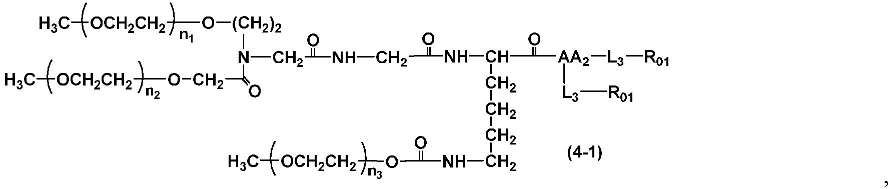 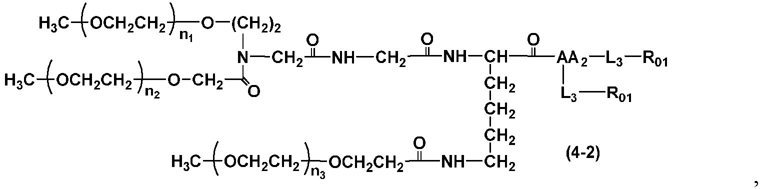 and 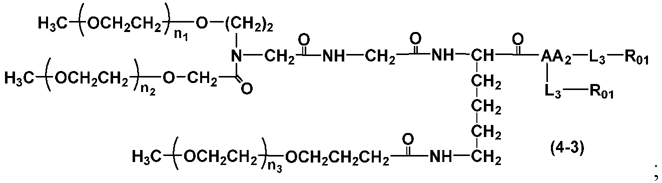
the rest parameters are defined as in general formula (2).

### A specific embodiment of the present invention is:

A three-arm polyethylene glycol derivative, wherein p is 2 and -(AA₁)₂- is a residue of phenylalanine-glycine dipeptide, and the structure of the derivative is represented by general formula (2-B): 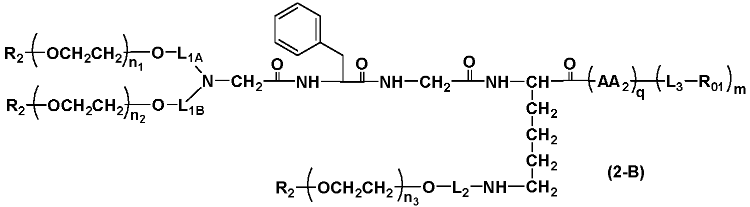 wherein,
L_{1A} and L_{1B} are each independently a C₁₋₆ alkylene group or -(CH₂)₁₋₆C(=O)-, wherein the left end of each linking group is connected to a PEG chain; more preferably, L_{1A} and L_{1B} are each independently selected from the group consisting of -CH₂CH₂-, -CH₂-, -CH₂C(=O)-, and -CH₂CH₂C(=O)-; more preferably, L_{1A} and L_{1B} conform to any one of the following cases:
   Case (1): L_{1A} and L_{1B} are each independently -CH₂CH₂- or -CH₂-;
   Case (2): one of L_{1A} and L_{1B} is -CH₂CH₂- or -CH₂-, and the other is -CH₂C(=O)- or -CH₂CH₂C(=O)-;
the rest parameters are defined as in general formula (1).

### 1.2.2 Divalent Linking Groups

In the present invention, L_{1A}, L_{1B}, and L₂ are each independently a divalent linking group; L₃ is a linking bond or a divalent linking group.

In one specific embodiment of the present invention, L₂ is preferably selected from the group consisting of -(CH₂)ₜₚ-, -(CH₂)_{tq}C(=O)-, -C(=O)(CH₂)_{tq}C(=O)-, -(CH₂)_{tq}NHC(=O)(CH₂)_{tq}C(=O)-, and -(CH₂)_{tq}(=O)NH(CH₂)_{tq}C(=O)-; wherein, tp is an integer in the range of 1 to 4; tq is, at each occurrence, independently an integer in the range of 0 to 4; the right end of the linking group is connected to the residue of lysine of general formula (1); L₂ is more preferably selected from the group consisting of -C(=O)-, -CH₂C(=O)-, -CH₂CH₂C(=O)-, -CH₂CH₂CH₂C(=O)-, -C(=O)CH₂CH₂C(=O)-, -C(=O)CH₂CH₂CH₂C(=O)-, -CH₂CH₂NHC(=O)CH₂CH₂C(=O)-, -CH₂CH₂CH₂NHC(=O)CH₂CH₂C(=O)-, -CH₂CH₂NHC(=O)CH₂CH₂CH₂C(=O)-, -CH₂CH₂CH₂NHC(=O)CH₂CH₂CH₂C(=O)-, -CH₂C(=O)NHCH₂C(=O)-, -CH₂CH₂C(=O)NHCH₂C(=O)-, and -CH₂CH₂CH₂C(=O)NHCH₂C(=O)-.

In one specific embodiment of the present invention, L₂ is more preferably -(CH₂)ₜₚ- or -(CH₂)_{tq}C(=O)-, tp is an integer in the range of 1 to 4, and tq is an integer in the range of 0 to 4; L₂ is preferably -C(=O)-, -CH₂CH₂C(=O)-, or -CH₂CH₂CH₂C(=O)-.

In one specific embodiment of the present invention, L₃ is preferably selected from the group consisting of a linking bond, an alkylene group, -C(=O)-, -OC(=O)-, -C(=O)O-, -OC(=O)O-, -O-, -S-, -S-S-, -C(=O)S-, -SC(=O)-, -NR_{c}-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, -NR_{c}C(=O)S-, and combinations thereof;
R_{c} is selected from the group consisting of H, a C₁₋₆ alkyl group, a carbocyclic group, and a heterocyclic group; preferably, R_{c} is H;
the alkylene group is preferably -(CRₐR_{b})ₜ-; wherein, t is, at each occurrence, independently an integer in the range of 1 to 12; Rₐ and R_{b} are each independently H, a C₁₋₆ alkyl group, a carbocyclic group, or a heterocyclic group; t instances of Rₐ and t instances of R_{b} are identical, or a combination of two or more different structures; preferably, Rₐ and R_{b} are, at each occurrence, independently -CH₃ or -H; more preferably, Rₐ and R_{b} are both H; preferably, t is an integer in the range of 1 to 4; more preferably, t is 1 or 2.

In one specific embodiment of the present invention, L_{1A} and L_{1B} are preferably, each independently a C₁₋₆ alkylene group, more preferably -CH₂CH₂- or -CH₂-.

In one specific embodiment of the present invention, the stability of the aforementioned divalent linking groups L_{1A}, L_{1B}, and L₃ is not particularly limited. L_{1A}, L_{1B}, and L₃, as well as the divalent linking groups formed by L_{1A}, L_{1B}, or L₃ with adjacent heteroatom-containing groups, are each independently a linking bond, a stable linking group, or a degradable linking group. The stable linking group can remain stable in both physiological environments in vivo and simulated physiological environments in vitro, and is preferably selected from the group consisting of a linking bond, an alkylene group, an amide bond, an ether bond, a carbamate bond, a secondary amino group, a carbonyl group, and combinations thereof.

### 1.2.3. Amino Acids/Oligopeptides

In the present invention, AA₁ and AA₂ are each independently a residue of amino acid or amino acid derivative.

In one specific embodiment of the present invention, p instances of AA₁ and q instances of AA₂ are each independently selected from the group consisting of the residues of glycine, alanine, β-alanine, valine, leucine, isoleucine, phenylalanine, tryptophan, tyrosine, aspartic acid, histidine, asparagine, glutamic acid, lysine, glutamine, methionine, arginine, serine, threonine, cysteine, ornithine, citrulline, and derivatives thereof;
when p=1, -AA₁- is preferably a residue of glycine;
when p≥2, -(AA₁)ₚ- is a residue of oligopeptide or oligopeptide derivative; the oligopeptide is a dipeptide, tripeptide, tetrapeptide, or pentapeptide, and is preferably selected from the group consisting of glycine-phenylalanine, histidine-β-alanine, glycine-glycine, lysine-glycine, lysine-glutamic acid, glycine-glycine-glycine, glycine-glycine-phenylalanine, glycine-phenylalanine-glycine, glycine-lysine-glycine, glycine-histidine-lysine, glycine-cysteine-glutamic acid, glycine-leucine-glycine, valine-citrulline-glycine, valine-alanine-glycine, arginine-glycine-aspartic acid, glycine-histidine-proline, glycine-leucine-phenylalanine-glycine, glycine-glycine-phenylalanine-glycine, glycine-lysine-glycine-lysine, glycine-phenylalanine-glutamic acid-phenylalanine-glycine, and arginine-lysine-aspartic acid-valine-tyrosine, more preferably selected from the group consisting of glycine-phenylalanine, histidine-β-alanine, glycine-glycine, lysine-glycine, glycine-glycine-phenylalanine, glycine-cysteine-glutamic acid, and glycine-leucine-phenylalanine-glycine;
when q is 1, AA₂ is preferably a residue of glutamic acid or aspartic acid.

### 1.2.4. R₀₁

In the present invention, R₀₁ is H, a functional group capable of reacting with bio-related substances, or a protected form of the functional group.

In one specific embodiment of the present invention, R₀₁ is preferably selected from the group consisting of an epoxy group, a hydroxyl group, a thiol group, a carboxyl group, an amino group, an aldehyde group, an active ester group, an active carbonate group, a carbamate group, an isocyanate group, an isothiocyanate group, a succinimide group, a maleimide group, an alkenyl group, an alkynyl group, an enoate group, an azido group, a cyano group, a dithiopyridyl group, an α-haloacetyl alkynyl group, a folic acid group, a rhodamine group, a biotin group, a monosaccharide group, a polysaccharide group, and protected forms thereof; more preferably, R₀₁ is selected from the group consisting of the following structures:

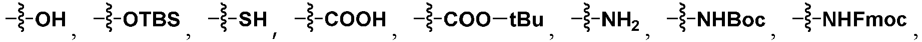

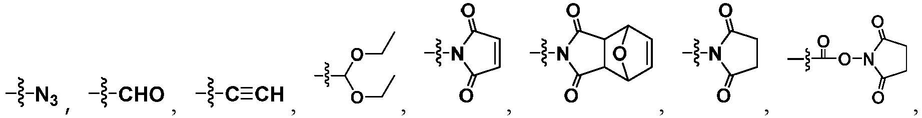

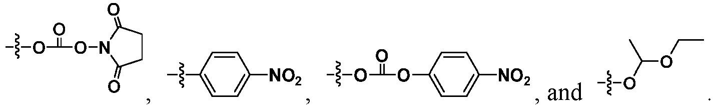

### 1.2.6. Preparation Methods

In one specific embodiment of the present invention, the three-arm polyethylene glycol derivative is obtained through a preparation process involving coupling reactions and/or polymerization reactions.

The coupling reaction is not particularly limited with respect to the selectable range, as long as two identical or different reactive groups can form a covalent linking group through the reaction. The preparation process may include coupling reactions in a single step or multiple steps, and the coupling reaction in each step is preferably, independently selected from the group consisting of alkylation reaction, condensation reaction, amidation reaction, esterification reaction, thioesterification reaction, ring-opening reaction, ring-closing condensation reaction, addition reaction, cycloaddition reaction, addition reaction of α,β-unsaturated bonds, addition reaction of alkynes, Schiff base reaction combined with reduction reaction, click reaction, azide-alkyne addition reaction, 1,3-dipolar cycloaddition reaction, Diels-Alder addition reaction, thiol-yne reaction, thiol-ene reaction, thiol-vinyl reaction, and condensation reaction. The reaction conditions for the coupling reactions are related to the type of the covalent linking groups formed in the reactions, which may include the prior art. The valence state of the covalent linking group obtained through the coupling reactions may be divalent or trivalent, preferably divalent. The coupling reactions may produce stable or degradable groups.

The polymerization reaction includes at least the following two steps: deprotonation of small molecule initiator, and polymerization of ethylene oxide; the small molecule initiator may be a readily available starting material, or an intermediate in the preparation process.

In the preparation process, when coupling and polymerization reactions are both present, the order in which the coupling and polymerization reactions are conducted is not limited.

In the present invention, the starting materials used in each preparation method can be obtained by purchase or synthesis.

In the present invention, with respect to the preparation method of monodisperse polyethylene glycol derivatives, the monodisperse starting materials containing polyethylene glycol components can be replaced with polydisperse starting materials containing the same components to obtain the corresponding polydisperse products. Similarly, with respect to the preparation method of polydisperse polyethylene glycol derivatives, the polydisperse starting materials containing polyethylene glycol components can be replaced with monodisperse starting materials containing the same components to obtain the corresponding monodisperse products. With respect to the structures provided in the present invention, the corresponding monodisperse and polydisperse derivatives are all within the scope of the present invention.

In one specific embodiment of the present invention, polyethylene glycol derivative starting materials, including but not limited to linear polyethylene glycol derivatives and non-linear polyethylene glycol derivatives, can be prepared by referring to the methods in CN108530637B, CN110591079A, CN108659227A, CN108530617B, or CN1243779C.

In the present invention, the intermediates and final products can be purified using purification methods including, but not limited to, extraction, recrystallization, adsorption treatment, precipitation, anti-solvent precipitation, membrane dialysis, supercritical extraction, etc. The structure and molecular weight of the final products can be characterized and confirmed using methods including, but not limited to, NMR, electrophoresis, UV-visible spectrophotometry, FTIR, AFM, GPC, HPLC, MALDI-TOF, circular dichroism, etc.

### 1.2.6.1. Preparation of Three-Arm Polyethylene Glycol Derivatives

In the present invention, the reaction routes described in the preparation methods do not represent the actual complete preparation process which may also include any necessary procedures familiar to those skilled in the art, such as minor modification (e.g., protection, deprotection), intermediate preparation, post-processing, purification, etc., and such methods for preparing polyethylene glycol derivatives would still fall within the scope of the present disclosure.

In the present invention, the preparation method of the three-arm polyethylene glycol derivative represented by general formula (1) may utilize one or more of the following processes, and the order in which each process is implemented is not particularly limited, as long as the implementation is smooth; wherein, RPEG is 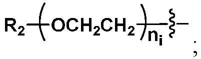 the processes include:

**Introduction of -(AA₁)_{P}-:** The two-arm polyethylene glycol derivative **2arm-PEG** is used as a starting material to be coupled with a small molecule of amino acid or oligopeptide H₂N-(AA₁)ₖ-COO-PG₁, wherein k is an integer in the range of 1 of 5; when k is smaller than p, two or more small molecules of amino acid or oligopeptide are needed for further coupling until the sum of all k is equal to p, therefore obtaining intermediate **IM-1.** Wherein, PG₁ is a carboxyl protecting group, preferably tBu (tert-butyl group).

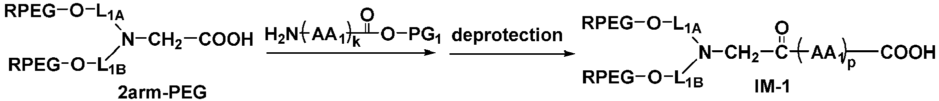

A specific example is the preparation of S1-3 in Example 1.1:

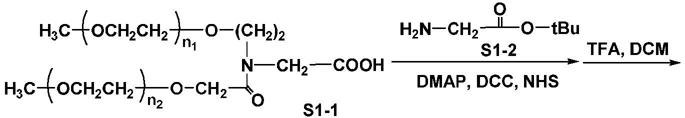

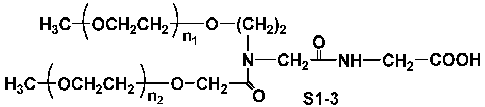

**Introduction of the lysine branching structure: IM-1** is coupled with the small molecule N-1 containing a Lys residue to obtain intermediate **IM-2.** Wherein, T is a linear or non-linear structure which does not contain any polyethylene glycol component, and its terminal contains a reactive group or minor modified form thereof (e.g., -COOH or -COOtBu, aldehyde or acetal groups); the structure of T at each occurrence is the same as or different from one another.

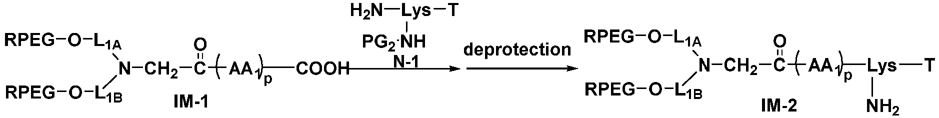

A specific example is the preparation of **S1-9** in **Example 1.2:**

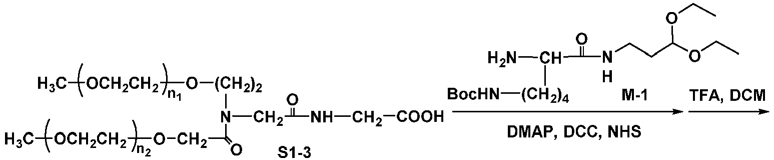

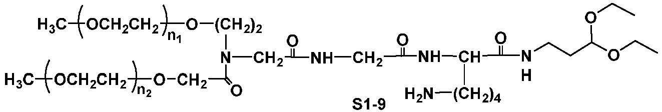

**Introduction of PEG single-chain components:** This process is divided into two cases: (1) when a two-arm PEG has already been used as a starting material, a third PEG single-chain component is introduced; (2) only polyethylene glycol derivatives containing a single-chain component are used as the PEG source.

Case (1): Intermediate **IM-2** is coupled with the polyethylene glycol derivative RPEG-L_{F}-F_{G} containing one single-chain component to obtain intermediate **IM-3,** wherein L_{F} is a divalent linking group, preferably a linking bond, -OCH₂CH₂-, or -OCH₂CH₂CH₂; F_{G} is a reactive group, preferably selected from the group consisting of -COOH, -F, -Cl, -Br, -OMs, -OTs, -CHO, -COCl, -CONHS, and an active ester group, more preferably -COOH or 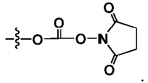 Wherein, the definition of T is the same as the aforementioned.

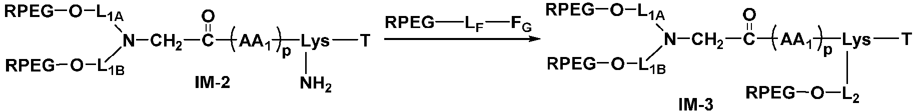

A specific example is the preparation of **E1-1** in **Example 1.1** (before removing the tBu protection):

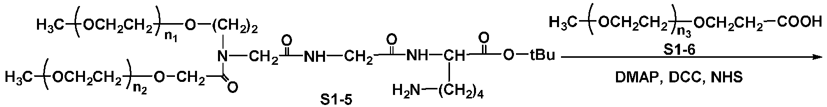

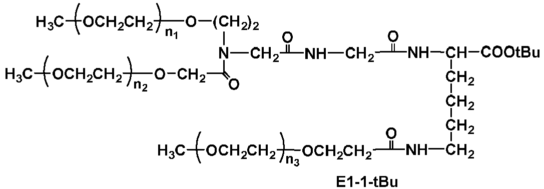

**Case (2):** An amino group of the small molecule **N-2** undergoes a single-step or stepwise reaction with the reactive group W of the polyethylene glycol derivative RPEG-L_{F}-W containing a PEG single-chain component to obtain the two-arm polyethylene glycol intermediate **IM-4.** Wherein, W is prefer ably a leaving group, more preferably -Cl, -Br, -OMs, or -OTs. Wherein, RPEG and L_{F} are the same as those in Case (1); PG₂ is an amino protecting group. After the removal of PG₂, a third PEG single-chain component is introduced by referring to Case (1).

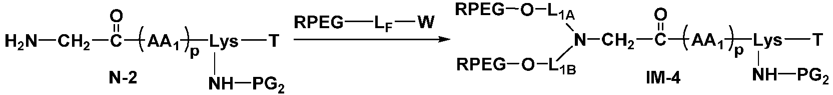

A specific example is the preparation of E9-1 in Example 9, wherein S9-5 corresponds to IM-4:

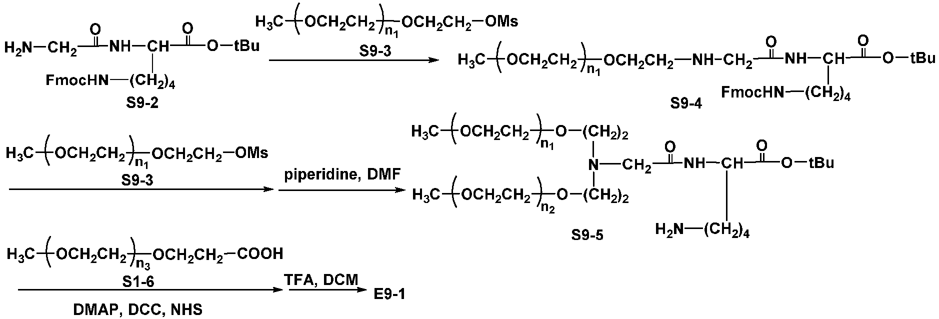

**End-functionalization:** The structure of 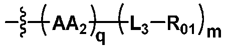 is obtained through terminal minor modification and/or further coupling with small molecules; such process corresponds to the transformation of T. Wherein, the terminal minor modification is selected from one or more of the following chemical reaction processes: protection, deprotection, salt complexation and decomplexation, ionization, protonation, deprotonation, and changing of leaving groups. A complete preparation example may contain multiple consecutive or non-consecutive transformations of T. When q is 1 and AA₂ is present, the transformation of T includes coupling with a small molecule containing the AA₂ residue, and the small molecule preferably contains a residue of glutamic acid. In a specific example such as **Example 1.1,** T undergoes the transformation from -COOtBu to -COOH; or in **Example 1.4,** T undergoes the transformation from -COOH to -C(=O)O(CH₂)₂OH, and further to 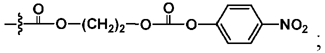 or in **Example 7,** T undergoes the transformation from -COOH to

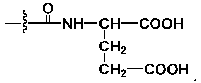

The parameters not specifically described above have the same definitions as those in general formula (1).

**End-functionalization based on R₀₁:** The end-functionalization based on R₀₁ may be carried out for a polyethylene glycol derivative of general formula (1), and the obtained structure after the end-functionalization still belongs to general formula (1). The method for the end-functionalization is not particularly limited but is related to the type of the final functional group or its protected form, mainly including the functionalization of the terminal hydroxyl group and the transformation from the terminal reactive group to the target functional group or its protected form. In the present application, specific preparation methods for the functionalization of the terminal hydroxyl group include but are not limited to those described in paragraphs [0960] to [1205] of CN104530417A. In the present application, the transformation from the terminal reactive group to the target functional group or its protected form can be realized by any of the following methods:
Method 1: The direct modification of the terminal reactive group produces the target functional group or its protected form. Examples include the transformation of a carboxyl group into an acyl halide group, a hydrazide group, an ester group, a thioester group, or a dithioester group, the transformation of a hydroxyl group, a thiol group, an alkynyl group, an amino group, or a carboxyl group, etc., into the corresponding protected form, and the modification of a hydroxyl group, or an amino group, etc., with an anhydride.
Method 2: The target functional group or its protected form is introduced through a coupling reaction between two reactive groups. The coupling reaction is based on the terminal reactive group and a reactive group from a heterofunctional reagent, and the heterofunctional reagent also contains the target functional group or its protected form. The reaction method of the two reactive groups is not particularly limited, and the reaction conditions are related to the type of the divalent linking group formed in the reaction, which may include the prior arts. The reaction includes but is not limited to alkylation, alkene addition, alkyne addition, Schiff base reaction combined with reduction reaction, condensation, etc. Wherein, the alkylation reaction is preferably based on thiol group or amino group, corresponding to the formation of thioether bond and secondary or tertiary amino group, respectively. Wherein, the condensation reaction includes but is not limited to those producing ester groups, thioester groups, amide groups, imine bonds, hydrazone bonds, carbamate groups, etc. The reaction also includes click reactions, and the heterofunctional reagent used contains not only the target functional group or its protected form, but also a reactive group such as an azido group, an alkynyl group, an alkenyl group, a tri-thioester group, a thiol group, a diene group, a furyl group, a 1,2,4,5-tetrazinyl group, a cyanate group, etc. The reaction of the two reactive groups is accompanied by the formation of new bonds. Typical representatives of newly formed divalent linking groups include amide bond, urethane bond, ester group, secondary amine bond, thioether bond, triazole group, etc.

### 1.2.7. Examples of Structures

In one specific embodiment of the present invention, the structure of three-arm polyethylene glycol derivative is selected from the group consisting of the following structural formulas: 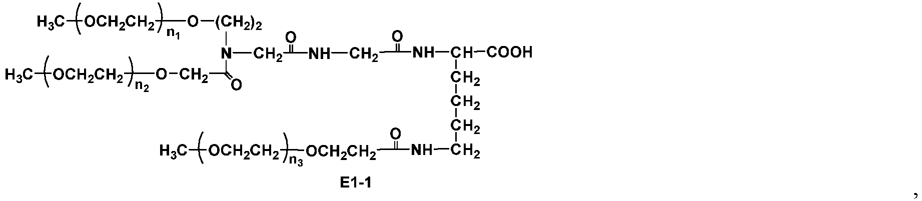 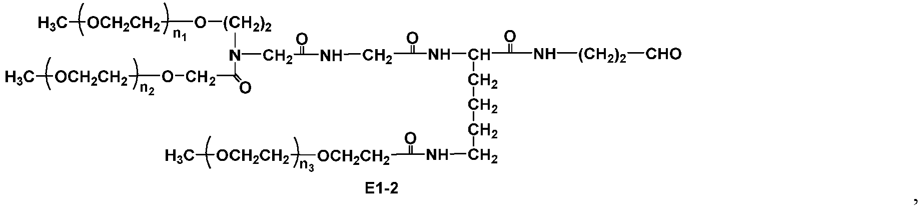 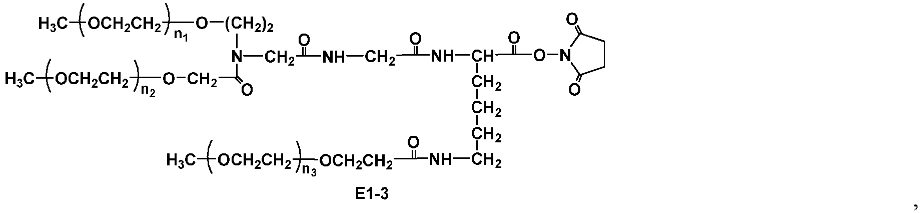 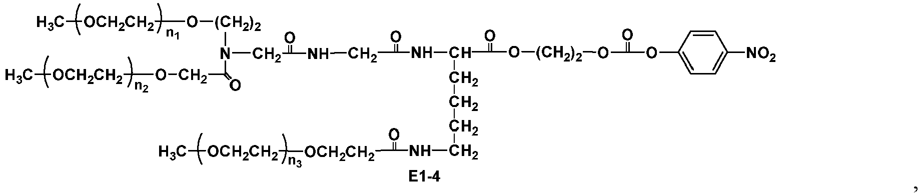 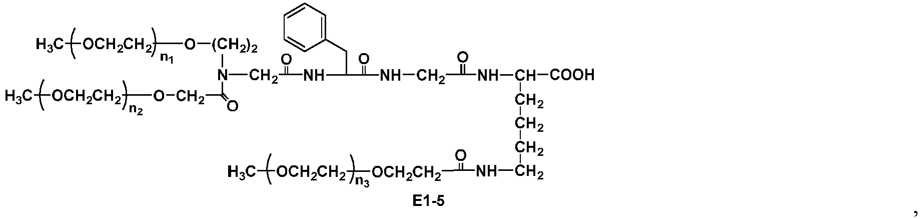 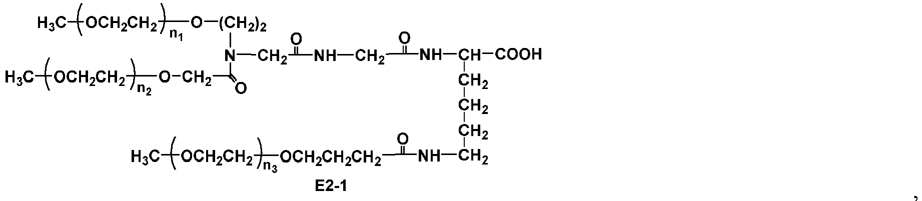 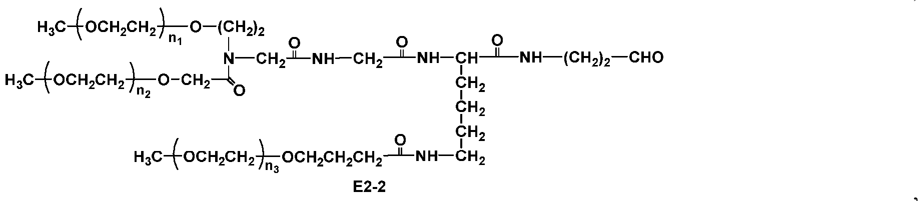 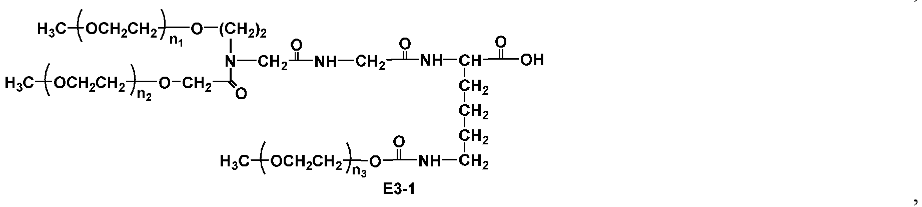 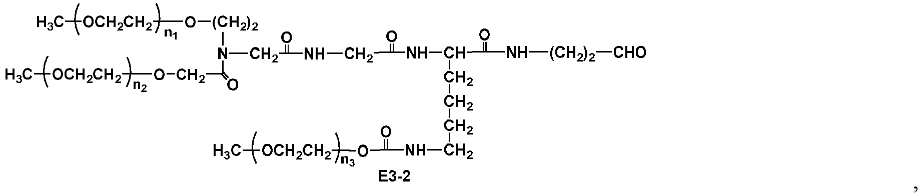 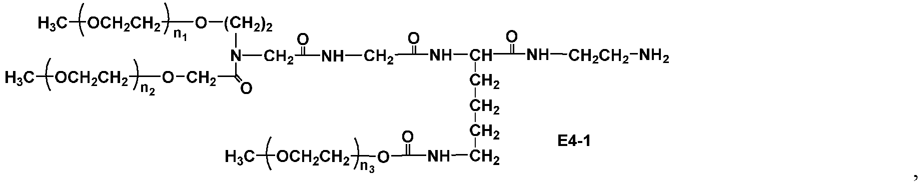 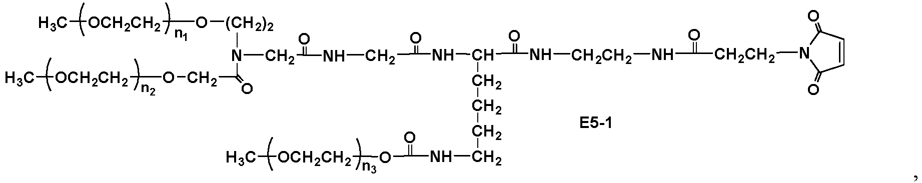 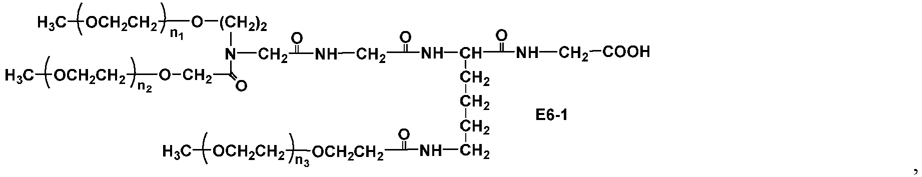 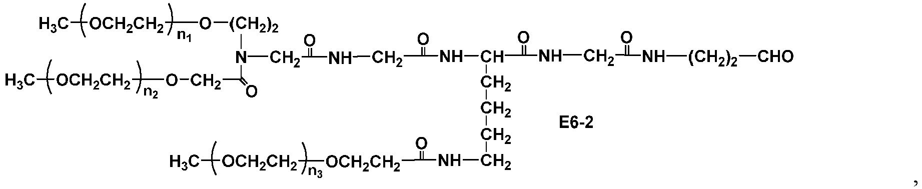 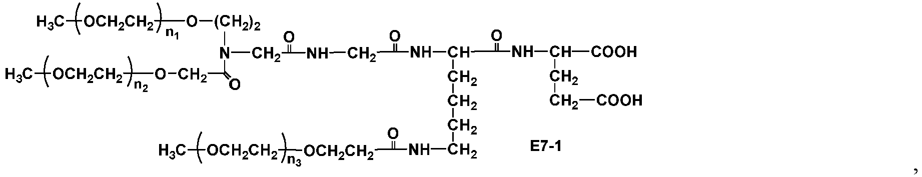 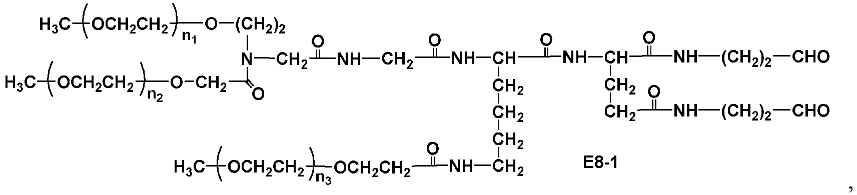 and

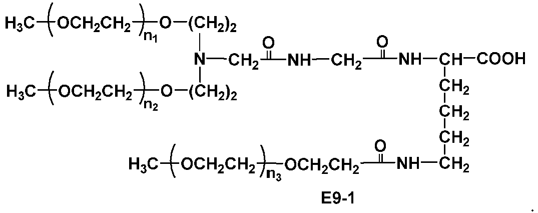

### 1.2.8. Bio-Related Substance Modified with a Three-Arm Polyethylene Glycol Derivative

An embodiment of the present invention is:
A bio-related substance modified with a three-arm polyethylene glycol derivative, having the structure represented by general formula (5):

   P-L-D (5)
wherein, P is a residue of any aforementioned three-arm polyethylene glycol derivative, D is a residue of bio-related substance, and L is a divalent linking group formed by the reacting the functional group of the three-arm polyethylene glycol derivative with the bio-related substance;
the bio-related substance is selected from the group consisting of drugs, proteins, polypeptides, oligopeptides, protein mimics, protein fragments, enzymes, antigens, antibodies, antibody fragments, receptors, aptamers of gene-related substances, polysaccharides, proteoglycans, glycoproteins, lipid compounds, hormones, vitamins, vesicles, liposomes, dyes, fluorescent substances, targeting factors, cytokines, neurotransmitters, extracellular matrix substances, plant or animal extracts, viruses, vaccines, cells, and micelles; preferably, the bio-related substance is selected from the group consisting of small molecule drugs, nucleic acids, steroids, phospholipids, and glycolipids; more preferably, the bio-related substance is selected from the group consisting of small molecule drugs, nucleosides, nucleotides, oligonucleotides, antisense oligonucleotides, polynucleotides, and steroids; furthermore, the small molecule drugs are preferably selected from the group consisting of flavonoids, terpenoids, carotenoids, saponins, steroids, sterols, quinones, anthraquinones, fluoroquinones, coumarins, alkaloids, porphyrins, polyphenols, macrolides, monobactams, phenylpropanoid phenols, anthracyclines, and aminoglycosides.

In one specific embodiment of the present invention, the bio-related substance is preferably a genetically engineered drug, selected from the group consisting of antibodies, antibody fragments, interleukins, lysozymes, interferons, growth hormones, erythropoietins, and granulocyte colony-stimulating factors, wherein the interferons are preferably α-, β- or γ-interferons.

### EXAMPLES

The following specific examples are for further description of the preparation of three-arm polyethylene glycol derivatives, the preparation of three-arm polyethylene glycol derivative-modified drugs, and biological activity assays. The specific examples are for further detailed illustration of the present invention, not limiting the scope of the present invention.

### Example 1: Three-arm monofunctional polyethylene glycol derivatives E1-1 and E1-2

### Example 1.1: Preparation of three-arm monofunctional polyethylene glycol-carboxylic acid derivative E1-1

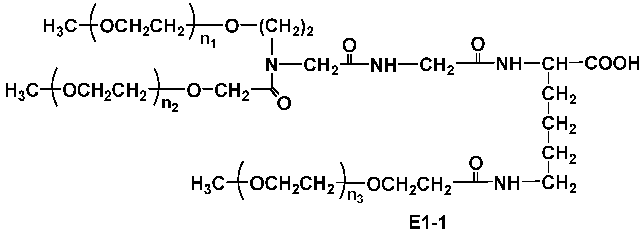

**E1-1** corresponds to general formula (3-2); wherein, L₃ is a linking bond, and R₀₁ is -OH. The designed total molecular weight is approximately 40.2 kDa, with n₁≈226, n₂≈226, and n₃≈450.

The preparation method is as follows:
Step a: Two-arm polyethylene glycol derivative **S1-1** (30.15 g, 1.5 mmol, Mₙ≈20.1 kDa, n₁≈n₂≈226, PDI=1.04) was dissolved in 150 mL anhydrous dichloromethane, followed by successively adding *N*-hydroxysuccinimide (NHS, 0.26 g, 2.3 mmol) and *N*,*N*'-dicyclohexylcarbodiimide (DCC, 0.46 g, 2.3 mmol). 4-(Dimethylamino)pyridine (DMAP, 0.04 g, 0.3 mmol) was added to the solution of glycine (**S1-2,** 1.97 g, 15.0 mmol) containing a tBu-protected carboxyl group in 30 mL dichloromethane. After mixing the two aforementioned solutions, the reaction was carried out at room temperature for 24 hours while stirring. After the reaction was over, the solution was filtered to remove insoluble substances and then concentrated under reduced pressure. The TFA/DCM mixed solution (1:1 v/v) was used to remove the tBu protection. After washing with purified water and extraction with dichloromethane, the extract was dried over anhydrous sodium sulfate, and then filtered, concentrated, and purified by column chromatography to obtain **S1-3** (18.27 g).
Step b: **S1-3** (16.16 g, 0.8 mmol, Mₙ≈20.2 kDa, n₁≈n₂≈226, PDI=1.04) was dissolved in 100 mL anhydrous dichloromethane, followed by successively adding NHS (0.14 g, 1.2 mmol) and DCC (0.25 g, 1.2 mmol). DMAP (0.02 g, 0.2 mmol) was added to the solution of lysine (**S1-4,** 3.40 g, 8.0 mmol) containing a Fmoc-protected amino group in 40 mL dichloromethane. After mixing the two aforementioned solutions, the reaction was carried out at room temperature for 24 hours while stirring. After the reaction was over, the solution was filtered to remove insoluble substances and then concentrated under reduced pressure. The 20% piperidine/DMF solution was used to remove the Fmoc protecting group, and then the solvent was removed by rotary evaporation. Purification by column chromatography was carried out to obtain **S1-5** (8.43 g).
Step c: Methoxy polyethylene glycol-propionic acid derivative **S1-6** (7.96 g, 0.4 mmol, Mₙ≈19.9 kDa, n₃≈450, PDI=1.04) was dissolved in 50 mL anhydrous dichloromethane, followed by successively adding NHS (0.07 g, 0.6 mmol) and DCC (0.12 g, 0.6 mmol). DMAP (0.01 g, 0.1 mmol) was added to the solution of **S1-5** (8.12 g, 0.4 mmol, Mₙ≈20.3 kDa, n₁≈n₂≈226, PDI=1.04) in 50 mL dichloromethane. After mixing the two aforementioned solutions, the reaction was carried out at room temperature for 24 hours while stirring. After the reaction was over, the solution was filtered to remove insoluble substances and then concentrated under reduced pressure. The TFA/DCM mixed solution (1:1 v/v) was used to remove the tBu protection. After washing with purified water and extraction with dichloromethane, the extract was dried over anhydrous sodium sulfate, and then filtered, concentrated, and purified by column chromatography to obtain **E1-1** (5.44 g). ¹H NMR (400 MHz, CDCl₃) δ: 4.27-4.21 (m, 1H, Lys-α-C*H*<), 4.12 (s, 2H, -OC*H*₂C(=O)N<), 3.74-3.43 (m, PEG; 2H, Gly-C*H*₂-; 4H, -O(C*H*₂)₂N<; 2H, >NC*H*₂C(=O)NH-; 2H, -OC*H*₂CH₂C(=O)NH-), 3.37 (s, 9H, -OC*H*₃), 3.23-3.16 (m, 2H, Lys-ε-C*H*₂-), 2.52-2.47 (t, 2H, -OCH₂C*H*₂C(=O)NH-), 1.87-1.34 (m, 6H, Lys-β-C*H*₂-, Lys-γ-C*H*₂-, Lys-α-C*H*₂-). The molecular weight determined by GPC was approximately 40.2 kDa, with PDI=1.05.

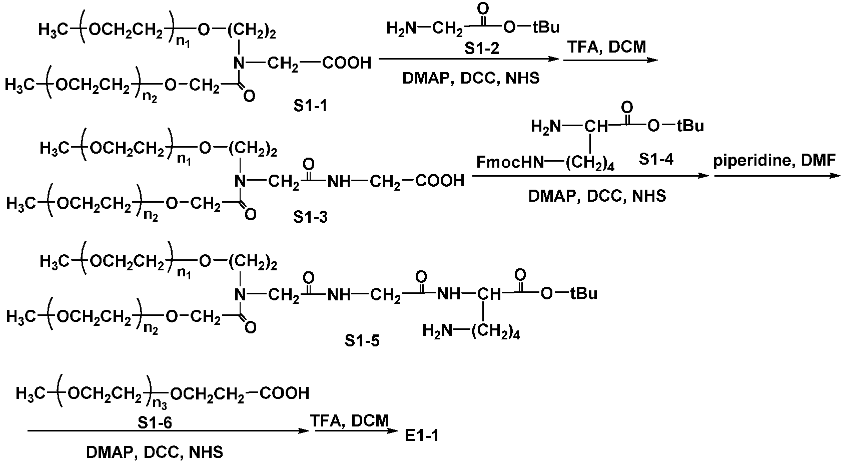

### Example 1.2: Preparation of three-arm monofunctional polyethylene glycol-aldehyde derivative E1-2

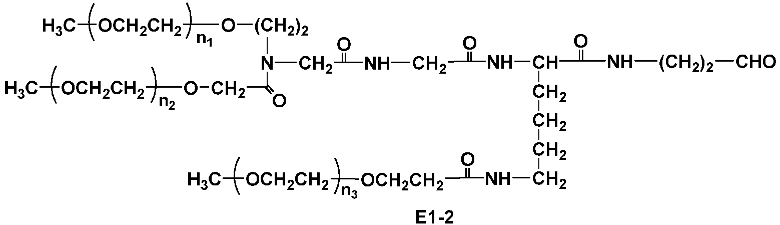

**E1-2** corresponds to general formula (3-2); wherein, L₃ is -NH(CH₂)₂-, and R₀₁ is -CHO. The designed total molecular weight is approximately 40.3 kDa, with n₁≈226, n₂≈226, and n₃≈450.

Step a: Lysine **S1-7** (5.03 g, 10.0 mmol) containing a Fmoc-protected amino group and a Cbz-protected amino group was dissolved in 80 mL anhydrous dichloromethane, followed by successively adding NHS (1.27 g, 11.0 mmol) and DCC (2.27 g, 11.0 mmol). DMAP (0.24 g, 2.0 mmol) was added to the solution of 3-aminopropionaldehyde diethyl acetal **S1-8** (1.76 g, 12.0 mmol) in 30 mL dichloromethane. After mixing the two aforementioned solutions, the reaction was carried out at room temperature for 24 hours while stirring. After the reaction was over, the solution was filtered to remove insoluble substances and then concentrated under reduced pressure. The 20% piperidine/DMF solution was used to remove the Fmoc protecting group, and then the solvent was removed by rotary evaporation. Purification by column chromatography was carried out to obtain **M-1** (3.73 g).

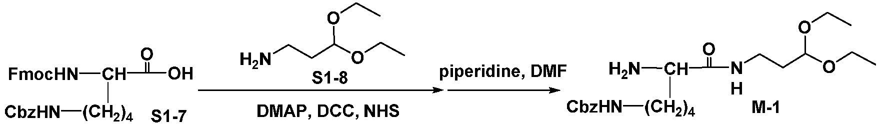

Step b: **S1-3** (16.16 g, 0.8 mmol, Mₙ≈20.2 kDa, n₁≈n₂≈226, PDI=1.04) was dissolved in 100 mL anhydrous dichloromethane, followed by successively adding NHS (0.14 g, 1.2 mmol) and DCC (0.25 g, 1.2 mmol). DMAP (0.02 g, 0.2 mmol) was added to the solution of **M-1** (3.28 g, 8.0 mmol) in 50 mL dichloromethane. After mixing the two aforementioned solutions, the reaction was carried out at room temperature for 24 hours while stirring. After the reaction was over, the solution was filtered to remove insoluble substances and then concentrated under reduced pressure. The crude product was dissolved with methanol, and then a reduction reaction was performed using Pd/C. After the reaction was completed, purification by column chromatography was carried out to obtain **S1-9** (8.70 g) containing a free amino group.

Step c: **S1-6** (7.96 g, 0.4 mmol, Mₙ≈19.9 kDa, n₃≈450, PDI=1.04) was dissolved in 50 mL anhydrous dichloromethane, followed by successively adding NHS (0.07 g, 0.6 mmol) and DCC (0.12 g, 0.6 mmol). DMAP (0.01 g, 0.1 mmol) was added to the solution of **S1-9** (8.16 g, 0.4 mmol, Mₙ≈20.4 kDa, n₁≈n₂≈226, PDI=1.04) in 50 mL dichloromethane. After mixing the two aforementioned solutions, the reaction was carried out at room temperature for 24 hours while stirring. After the reaction was over, the solution was filtered to remove insoluble substances and then concentrated. The crude product was dissolved with water, and the pH of the solution was adjusted to 2.0 with hydrochloric acid. The reaction was carried out for 16 hours while stirring. After the reaction was completed, 10% sodium chloride was added, and the pH of the solution was adjusted to 6.4±0.2 with sodium bicarbonate. The solution was extracted with dichloromethane. The organic phases were combined, dried over anhydrous magnesium sulfate, filtered, and recrystallized with anhydrous isopropanol to obtain **E1-2** (5.96 g). ¹H NMR (400 MHz, CDCl₃) δ: 4.26-4.19 (m, 1H, Lys-α-C*H*<), 4.12 (s, 2H, -OC*H*₂C(=O)N<), 3.75-3.38 (m, PEG; 2H, Gly-C*H*₂-; 4H, -O(C*H*₂)₂N<; 2H, >NC*H*₂C(=O)NH-; 2H, -OC*H*₂CH₂C(=O)NH-; 2H, -C*H*₂CH₂CHO), 3.36 (s, 9H, -OC*H*₃), 3.22-3.13 (m, 2H, Lys-ε-C*H*₂-), 2.74-2.64 (m, 2H, -CH₂C*H*₂CHO), 2.52-2.47 (t, 2H, -OCH₂C*H*₂C(=O)NH-), 1.80-1.35 (m, 6H, Lys-β-C*H*₂-, Lys-γ-C*H*₂-, Lys-α-C*H*₂-). The molecular weight determined by GPC was approximately 40.3 kDa, with PDI=1.06.

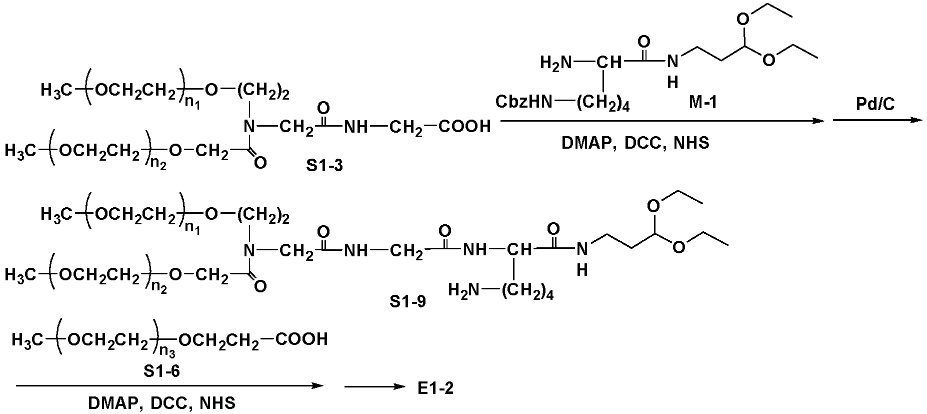

### Example 1.3: Preparation of three-arm monofunctional polyethylene glycol-succinimide active ester derivative E1-3

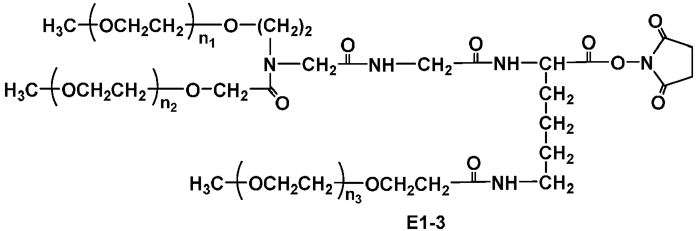

**E1-3** corresponds to general formula (3-2); wherein, L₃ is -O-, and R₀₁ is a succinimide group. The designed total molecular weight is approximately 40.3 kDa, with n₁≈226, n₂≈226, and n₃≈450.

The preparation method is as follows:
**E1-1** (8.04 g, 0.2 mmol, Mₙ≈40.2 kDa, n₁≈n₂≈226, n₃≈450, PDI=1.05) was dissolved in 100 mL anhydrous dichloromethane, followed by successively adding NHS (0.12 g, 1.0 mmol) and DCC (0.21 g, 1.0 mmol). The reaction was carried out at room temperature for 24 hours while stirring. After the reaction was over, the solution was filtered to remove insoluble substances, concentrated under reduced pressure, and purified by column chromatography to obtain **E1-3** (7.70 g). ¹H NMR (400 MHz, CDCl₃) δ: 4.47-4.40 (m, 1H, Lys-α-C*H*<), 4.12 (s, 2H, -OC*H*₂C(=O)N<), 3.75-3.43 (m, PEG; 2H, Gly-C*H*₂-; 4H, -O(C*H*₂)₂N<; 2H, >NC*H*₂C(=O)NH-; 2H, -OC*H*₂C*H*₂C(=O)NH-), 3.37 (s, 9H, -OC*H*₃), 3.19-3.13 (m, 2H, Lys-ε-C*H*₂-), 2.77 (s, 4H, NHS-C*H*₂-), 2.52-2.47 (t, 2H, -OCH₂C*H*₂C(=O)NH-), 1.92-1.31 (m, 6H, Lys-β-C*H*₂-, Lys-γ-C*H*₂-, Lys-σ-C*H*₂-). The molecular weight determined by GPC was approximately 40.3 kDa, with PDI=1.05.

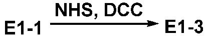

### Example 1.4: Preparation of three-arm monofunctional polyethylene glycol-p-nitrophenyl active ester derivative E1-4

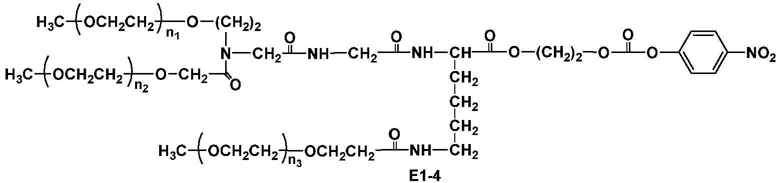

**E1-4** corresponds to general formula (3-2); wherein, L₃ is -O(CH₂)₂OC(=O)O-, and R₀₁ is a *p*-nitrophenyl group. The designed total molecular weight is approximately 40.4 kDa, with n₁≈226, n₂≈226, and n₃≈450.

The preparation method is as follows:
Step a: Under argon atmosphere, **E1-1** (20.10 g, 0.5 mmol, Mₙ≈40.2 kDa, n₁≈n₂≈226, n₃≈450, PDI=1.05), ethylene glycol (**S1-10,** 0.31 g, 5.0 mmol), and DMAP (0.01 g, 0.1 mmol) were successively dissolved in dichloromethane (200 mL), followed by the dropwise addition of the dichloromethane solution (3 mL) of DCC (0.12 g, 0.6 mmol) in an ice bath. After the dropwise addition, the reaction temperature was raised to room temperature, and the reaction was carried out for 24 h while stirring. After the reaction was over, the solution was filtered to remove precipitates, concentrated, and purified by column chromatography to obtain **S1-11** (10.42 g).
Step b: In an ice bath, 1 mL dichloromethane solution of *p*-nitrophenyl chloroformate (**S1-12,** 0.05g, 0.2 mmol) was slowly added dropwise into the solution of **S1-11** (8.06 g, 0.2 mmol, Mₙ≈40.2 kDa, n₁≈n₂≈226, n₃≈450, PDI=1.05) and triethylamine (TEA, 0.04 g, 0.4 mmol) in dichloromethane (80 mL). After stirring for one hour, the reaction temperature was raised to room temperature, and the reaction was continued for 24 hours. After the reaction was over, the solution was extracted, concentrated, and purified by column chromatography to obtain **E1-4** (6.71 g). ¹H NMR (400 MHz, CDCl₃) δ: 8.28 (d, 4H, Ar), 7.38 (d, 4H, Ar), 4.48-4.40 (m, 4H, -C(=O)O(CH₂)₂OC(=O)O-), 4.26-4.17 (m, 1H, Lys-α-CH<), 4.13 (s, 2H, -OCH₂C(=O)N<), 3.75-3.41 (m, PEG; 2H, Gly-CH₂-; 4H, -O(CH₂)₂N<; 2H, >NCH₂C(=O)NH-; 2H, -OCH₂CH₂C(=O)NH-), 3.37 (s, 9H, -OCH₃), 3.22-3.15 (m, 2H, Lys-ε-C*H*₂-), 2.52-2.48 (t, 2*H*, -OCH₂CH₂C(=O)NH-), 1.89-1.35 (m, 6H, Lys-β-C*H*₂-, Lys-γ-C*H*₂-, Lys-σ-C*H*₂-). The molecular weight determined by GPC was approximately 40.4 kDa, with PDI=1.05.

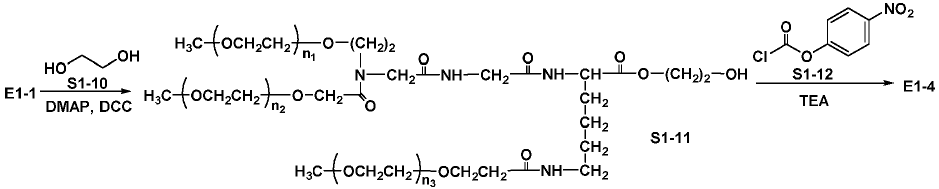

### Example 1.5: Preparation of three-arm monofunctional polyethylene glycol-carboxylic acid derivative E1-5 (containing a residue of phenylalanine-glycine dipeptide)

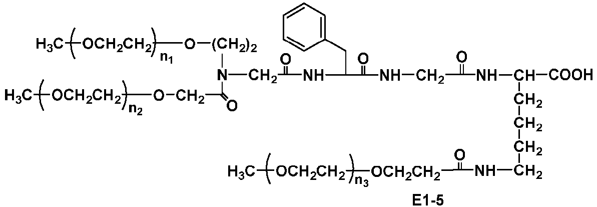

**E1-5** corresponds to general formula (2-B); wherein, R₂ is a methyl group, L_{1A} is an ethylene group, q is 0, m is 1, L₃ is a linking bond, and R₀₁ is -OH. The designed total molecular weight is approximately 40.3 kDa, with n₁≈226, n₂≈226, and n₃≈450.

Referring to the preparation method in Example 1.1, **S1-2** was replaced with phenylalanine-glycine dipeptide containing a tBu-protected carboxyl group, and the rest steps and molar amounts remained unchanged to obtain **E1-5** (5.26 g). ¹H NMR (400 MHz, CDCl₃) δ: 7.29-7.17 (m, 5H, Phe-Ar), 4.51-4.41 (m, 1H, Phe-α-C*H*<), 4.28-4.23 (m, 1H, Lys-α-C*H*<), 4.12 (s, 2H, -OC*H*₂C(=O)N<), 3.76-3.43 (m, PEG; 2H, Gly-C*H*₂-; 4H, -O(C*H*₂)₂N<; 2H, >NC*H*₂C(=O)NH-; 2H, -OC*H*₂CH₂C(=O)NH-), 3.36 (s, 9H, -OC*H*₃), 3.31-2.92 (m, 2H, Phe-C*H*₂-; 2H, Lys-ε-C*H*₂-), 2.53-2.38 (t, 2H, -OCH₂C*H*₂C(=O)NH-), 1.89-1.29 (m, 6H, Lys-β-C*H*₂-, Lys-γ-C*H*₂-, Lys-σ-C*H*₂-). The molecular weight determined by GPC was approximately 40.3 kDa, with PDI=1.05.

### Example 2: Three-arm monofunctional polyethylene glycol derivatives E2-1 and E2-2

### Example 2.1: Preparation of three-arm monofunctional polyethylene glycol-carboxylic acid derivative E2-1

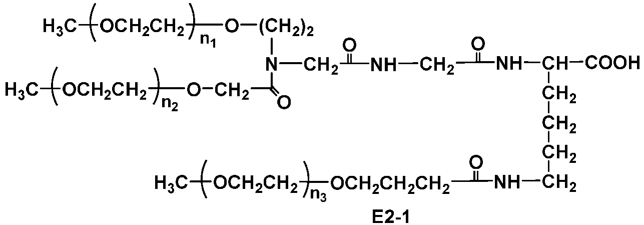

**E2-1** corresponds to general formula (3-3); wherein, L₃ is a linking bond, and R₀₁ is -OH. The designed total molecular weight is approximately 30.4 kDa, with n₁≈n₂≈n₃≈226.

The preparation method is as follows:
Methoxy polyethylene glycol-butyric acid derivative **S2-1** (10.10 g, 1.0 mmol, Mₙ≈10.1 kDa, n₃≈226, PDI=1.03) was dissolved in 50 mL anhydrous dichloromethane, followed by successively adding NHS (0.17 g, 1.5 mmol) and DCC (0.31 g, 1.5 mmol). DMAP (0.02 g, 0.2 mmol) was added to the solution of **S1-5** (20.3 g, 1.0 mmol, Mₙ≈20.3 kDa, n₁≈n₂≈226, PDI=1.04) in 100 mL dichloromethane. After mixing the two aforementioned solutions, the reaction was carried out at room temperature for 24 hours while stirring. After the reaction was over, the solution was filtered to remove insoluble substances, and then concentrated under reduced pressure. The TFA/DCM mixed solution (1:1 v/v) was used to remove the tBu protection. After washing with purified water and extraction with dichloromethane, the extract was dried over anhydrous sodium sulfate, and then filtered, concentrated, and purified by column chromatography to obtain **E2-1** (9.36 g). ¹H NMR (400 MHz, CDCl₃) δ: 4.27-4.21 (m, 1H, Lys-α-C*H*<), 4.12 (s, 2H, -OC*H*₂C(=O)N<), 3.72-3.40 (m, PEG; 2H, Gly-C*H*₂-; 4H, -O(C*H*₂)₂N<; 2H, >NC*H*₂C(=O)NH-; 2H, -OC*H*₂CH₂CH₂C(=O)NH-), 3.36 (s, 9H, -OC*H*₃), 3.23-3.14 (m, 2H, Lys-ε-C*H*₂-), 2.35-2.21 (m, 2H, -OCH₂CH₂C*H*₂C(=O)NH-), 1.89-1.32 (m, 6H, Lys-β-C*H*₂-, Lys-γ-C*H*₂-, Lys-α-C*H*₂-; 2H, -OCH₂C*H*₂CH₂C(=O)NH-). The molecular weight determined by GPC was approximately 30.4 kDa, with PDI=1.06.

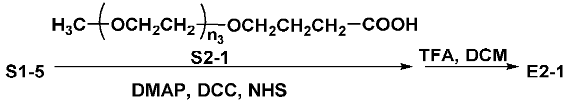

### Example 2.2: Preparation of three-arm monofunctional polyethylene glycol-aldehyde derivative E2-2

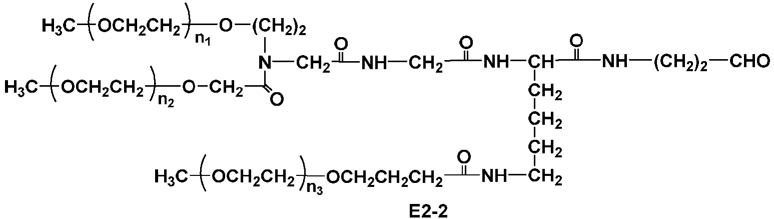

**E2-2** corresponds to general formula (3-3); wherein, L₃ is -NH(CH₂)₂-, and R₀₁ is -CHO. The designed total molecular weight is approximately 30.4 kDa, with n₁≈n₂≈n₃≈226.

The preparation method is as follows:
**S2-1** (10.10 g, 1.0 mmol, Mₙ≈10.1 kDa, n₃≈226, PDI=1.03) was dissolved in 50 mL anhydrous dichloromethane, followed by successively adding NHS (0.17 g, 1.5 mmol) and DCC (0.31 g, 1.5 mmol). DMAP (0.02 g, 0.2 mmol) was added to the solution of S1-9 (20.40 g, 1.0 mmol, Mₙ≈20.4 kDa, n₁≈n₂≈226, PDI=1.04) in 100 mL dichloromethane. After mixing the two aforementioned solutions, the reaction was carried out at room temperature for 24 hours while stirring. After the reaction was over, the solution was filtered to remove insoluble substances and then concentrated. The crude product was dissolved with water, and the pH of the solution was adjusted to 2.0 with hydrochloric acid. The reaction was carried out for 16 hours while stirring. After the reaction was completed, 10% sodium chloride was added, and the pH of the solution was adjusted to 6.4±0.2 with sodium bicarbonate. The solution was extracted with dichloromethane. The organic phases were combined, dried over anhydrous magnesium sulfate, filtered, and recrystallized with anhydrous isopropanol to obtain **E2-2** (9.70 g). ¹H NMR (400 MHz, CDCl₃) δ: 4.26-4.19 (m, 1H, Lys-α-C*H*<), 4.12 (s, 2H, -OC*H*₂C(=O)N<), 3.74-3.39 (m, PEG; 2H, Gly-C*H*₂-; 4H, -O(C*H*₂)₂N<; 2H, >NC*H*₂C(=O)NH-; 2H, -OC*H*₂CH₂CH₂C(=O)NH-; 2H, -C(=O)NHC*H*₂CH₂CHO; 9H, -OC*H*₃), 3.19-3.11 (m, 2H, Lys-ε-C*H*₂-), 2.73-2.66 (m, 2H, -C(=O)NHCH₂C*H*₂CHO), 2.34-2.22 (m, 2H, -OCH₂CH₂C*H*₂C(=O)NH-), 1.82-1.34 (m, 6H, Lys-β-C*H*₂-, Lys-γ-C*H*₂-, Lys-σ-C*H*₂-; 2H, -OCH₂C*H*₂CH₂C(=O)NH-). The molecular weight determined by GPC was approximately 30.4 kDa, with PDI=1.05.

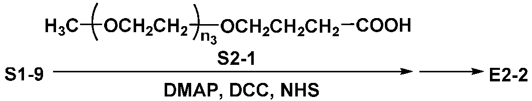

### Example 3: Three-arm monofunctional polyethylene glycol derivatives E3-1 and E3-2

### Example 3.1: Preparation of three-arm monofunctional polyethylene glycol-carboxylic acid derivative E3-1

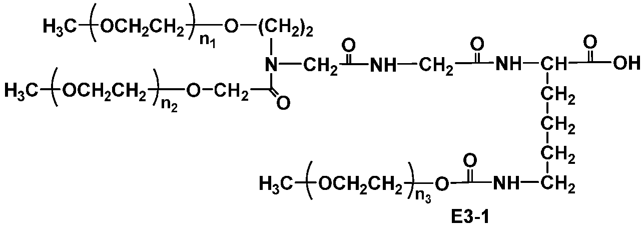

**E3-1** corresponds to general formula (3-1); wherein, L₃ is a linking bond, and R₀₁ is -OH. The designed total molecular weight is approximately 40.2 kDa, with n₁≈n₂≈226 and n₃≈450.

The preparation method is as follows:
Methoxy polyethylene glycol-succinimide carbonate **S3-1** (20.00 g, 1.0 mmol, Mₙ≈20.0 kDa, n₃≈450, PDI=1.04) was dissolved in 100 mL anhydrous dichloromethane, followed by successively adding NHS (0.17 g, 1.5 mmol) and DCC (0.31 g, 1.5 mmol). DMAP (0.02 g, 0.2 mmol) was added to the solution of S1-5 (20.3 g, 1.0 mmol, Mₙ≈20.3 kDa, n₁≈n₂≈226, PDI=1.04) in 100 mL dichloromethane. After mixing the two aforementioned solutions, the reaction was carried out at room temperature for 24 h while stirring. After the reaction was over, the solution was filtered to remove insoluble substances, and then concentrated under reduced pressure. The TFA/DCM mixed solution (1:1 v/v) was used to remove the tBu protection. After washing with purified water and extraction with dichloromethane, the extract was dried over anhydrous sodium sulfate, and then filtered, concentrated, and purified by column chromatography to obtain **E3-1** (10.65 g). The molecular weight determined by GPC was approximately 40.2 kDa, with PDI=1.06.

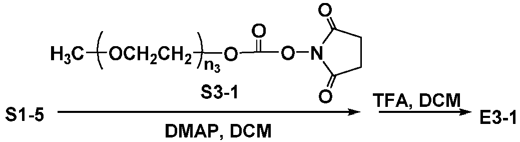

### Example 3.2: Preparation of three-arm monofunctional polyethylene glycol-aldehyde derivative E3-2

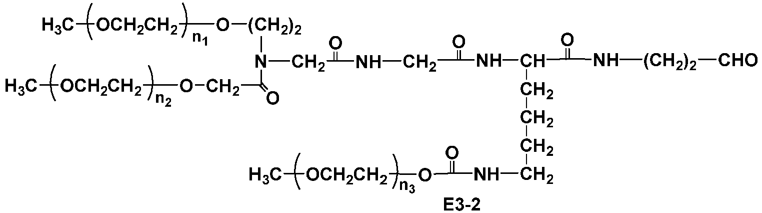

**E3-2** corresponds to general formula (3-1); wherein, L₃ is -NH(CH₂)₂-, and R₀₁ is -CHO. The designed total molecular weight is approximately 40.2 kDa, with n₁≈n₂≈226 and n₃≈450.

The preparation method is as follows:
**S3-1** (20.00 g, 1.0 mmol, Mₙ≈20.0 kDa, n₃≈450, PDI=1.04) was dissolved in 100 mL anhydrous dichloromethane, followed by successively adding NHS (0.17 g, 1.5 mmol) and DCC (0.31 g, 1.5 mmol). DMAP (0.02 g, 0.2 mmol) was added to the solution of **S1-9** (20.40 g, 1.0 mmol, Mₙ≈20.4 kDa, n₁≈n₂≈226, PDI=1.04) in 100 mL dichloromethane. After mixing the two aforementioned solutions, the reaction was carried out at room temperature for 24 h while stirring. After the reaction was over, the solution was filtered to remove insoluble substances and then concentrated. The crude product was dissolved with water, and the pH of the solution was adjusted to 2.0 with hydrochloric acid. The reaction was carried out for 16 hours while stirring. After the reaction was completed, 10% sodium chloride was added, and the pH of the solution was adjusted to 6.4±0.2 with sodium bicarbonate. The solution was extracted with dichloromethane. The organic phases were combined, dried over anhydrous magnesium sulfate, filtered, and recrystallized with anhydrous isopropanol to obtain **E3-2** (11.66 g). ¹H NMR (400 MHz, CDCl₃) δ: 4.28-4.16 (m, 1H, Lys-α-C*H*<; 2H, -OCH₂C*H*₂OC(=O)NH-), 4.12 (s, 2H, -OC*H*₂C(=O)N<), 3.75-3.38 (m, PEG; 2H, Gly-C*H*₂-; 4H, -O(C*H*₂)₂N<; 2H, >NC*H*₂C(=O)NH-; 2H, -OC*H*₂CH₂OC(=O)NH-; 2H, -C(=O)NHC*H*₂CH₂CHO), 3.36 (s, 9H, -OC*H*₃), 3.21-3.11 (m, 2H, Lys-ε-C*H*₂-), 2.73-2.62 (m, 2H, -C(=O)NHCH₂C*H*₂CHO), 1.78-1.34 (m, 6H, Lys-β-C*H*₂-, Lys-γ-C*H*₂-, Lys-σ-C*H*₂-). The molecular weight determined by GPC was approximately 40.2 kDa, with PDI=1.06.

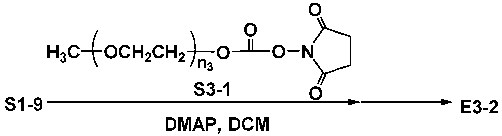

### Example 4: Three-arm monofunctional polyethylene glycol-amine derivative (E4-1)

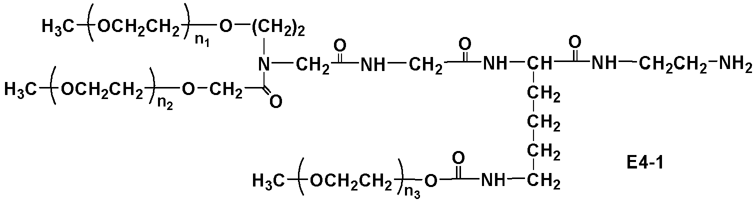

**E4-1** corresponds to general formula (3-1); wherein, L₃ is -NH(CH₂)₂-, and R₀₁ is -NH₂. The designed total molecular weight is approximately 40.2 kDa, with n₁≈n₂≈226 and n₃≈450.

The preparation method is as follows:
**E3-1** (20.10 g, 0.5 mmol, Mₙ≈40.2 kDa, n₁≈n₂≈226, n₃≈450, PDI=1.06) was dissolved in 200 mL anhydrous dichloromethane, followed by successively adding NHS (0.09 g, 0.8 mmol) and DCC (0.15 g, 0.8 mmol). DMAP (0.01 g, 0.1 mmol) was added to the solution of ethylenediamine (**S4-1,** 0.30 g, 5.0 mmol) in 5 mL dichloromethane. After mixing the two aforementioned solutions, the reaction was carried out at room temperature for 24 h while stirring. After the reaction was over, the solution was filtered to remove insoluble substances, concentrated under reduced pressure, and purified by column chromatography to obtain **E4-1** (7.04 g). ¹H NMR (400 MHz, CDCl₃) δ: 4.30-4.17 (m, 1H, Lys-α-C*H*<; 2H, -OCH₂C*H*₂OC(=O)NH-), 4.12 (s, 2H, -OC*H*₂C(=O)N<), 3.77-3.43 (m, PEG; 2H, Gly-C*H*₂-; 4H, -O(C*H*₂)₂N<; 2H, >NC*H*₂C(=O)NH-; 2H, -OC*H*₂CH₂OC(=O)NH-), 3.36 (s, 9H, -OC*H*₃), 3.33-3.25 (m, 2H, -C(=O)NHC*H*₂CH₂NH₂), 3.19-3.10 (m, 2H, Lys-ε-C*H*₂-), 2.84 (m, 2H, -C(=O)NHCH₂C*H*₂NH₂), 1.77-1.32 (m, 6H, Lys-β-C*H*₂-, Lys-γ-C*H*₂-, Lys-σ-C*H*₂-). The molecular weight determined by GPC was approximately 40.2 kDa, with PDI=1.06.

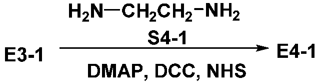

### Example 5: Three-arm monofunctional polyethylene glycol-maleimide derivative (E5-1)

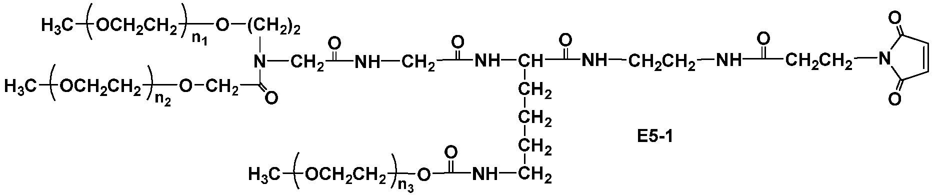

**E5-1** corresponds to general formula (3-1); wherein, L₃ is -NH(CH₂)₂NHC(=O)(CH₂)₂-, and R₀₁ is a maleimide group. The designed total molecular weight is approximately 40.4 kDa, with n₁≈n₂≈226 and n₃≈450.

The preparation method is as follows:
**E4-1** (20.10 g, 0.5 mmol, Mₙ≈40.2 kDa, n₁≈n₂≈226, n₃≈450, PDI=1.06) was dissolved in 200 mL anhydrous dichloromethane, followed by successively adding NHS (0.09 g, 0.8 mmol) and DCC (0.15 g, 0.8 mmol). DMAP (0.01 g, 0.1 mmol) was added to the solution of 3-maleimidopropionic acid containing a furan protecting group (**S5-1,** 1.19 g, 5.0 mmol) in 15 mL dichloromethane. After mixing the two aforementioned solutions, the reaction was carried out at room temperature for 24 h while stirring. After the reaction was over, the solution was filtered to remove insoluble substances, and then concentrated under reduced pressure. 50 mL toluene (Tol) and 0.50 g 2,6-di-tert-butyl-4-methylphenol (BHT) were successively added. After heating to 125°C, the reaction was carried out for 5 hours while stirring to remove the furan protecting group. The solution was concentrated and then recrystallized with anhydrous isopropanol to obtain **E5-1** (5.60 g). ¹H NMR (400 MHz, CDCl₃) δ: 6.72 (s, 2H, MAL), 4.28-4.17 (m, 1H, Lys-α-C*H*<; 2H, -OCH₂C*H*₂OC(=O)NH-), 4.13 (s, 2H, -OC*H*₂C(=O)N<), 3.85 (t, 2H, -CH₂C*H*₂-MAL), 3.78-3.43 (m, PEG; 2H, Gly-C*H*₂-; 4H, -O(C*H*₂)₂N<; 2H, >NC*H*₂C(=O)NH-; 2H, -OC*H*₂CH₂OC(=O)NH-), 3.37 (s, 9H, -OC*H*₃), 3.30-3.23 (m, 4H, -C(=O)NH(C*H*₂)₂NHC(=O)-), 3.19-3.11 (m, 2H, Lys-ε-C*H*₂-), 2.57 (t, 2H, -C*H*₂CH₂-MAL), 1.78-1.34 (m, 6H, Lys-β-C*H*₂-, Lys-γ-C*H*₂-, Lys-σ-C*H*₂-). The molecular weight determined by GPC was approximately 40.4 kDa, with PDI=1.06.

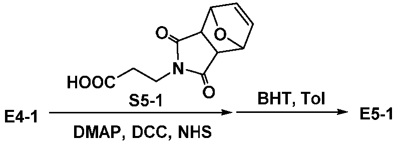

### Example 6: Three-arm monofunctional polyethylene glycol derivatives E6-1 and E6-2

### Example 6.1: Preparation of three-arm monofunctional polyethylene glycol-carboxylic acid derivative E6-1

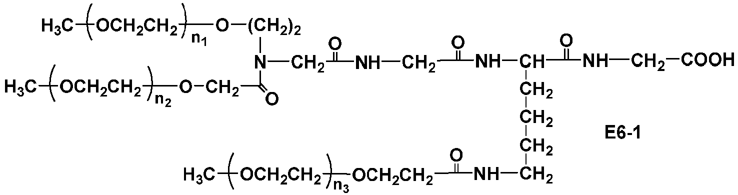

**E6-1** corresponds to general formula (3-2); wherein, L₃ is -NHCH₂-, and R₀₁ is -COOH. The designed total molecular weight is approximately 40.3 kDa, with n₁≈n₂≈226 and n₃≈450.

The preparation method is as follows:
**E1-1** (20.10 g, 0.5 mmol, Mₙ≈40.2 kDa, n₁≈n₂≈226, n₃≈450, PDI=1.05) was dissolved in 200 mL anhydrous dichloromethane, followed by successively adding NHS (0.09 g, 0.8 mmol) and DCC (0.15 g, 0.8 mmol). DMAP (0.01 g, 0.1 mmol) was added to the solution of **S1-2** (0.66 g, 5.0 mmol) in 10 mL dichloromethane. After mixing the two aforementioned solutions, the reaction was carried out at room temperature for 24 h while stirring. After the reaction was over, the solution was filtered to remove insoluble substances, and then concentrated under reduced pressure. The TFA/DCM mixed solution (1:1 v/v) was used to remove the tBu protection. After washing with purified water and extraction with dichloromethane, the extract was dried over anhydrous sodium sulfate, and then filtered, concentrated, and purified by column chromatography to obtain **E6-1** (8.28 g). ¹H NMR (400 MHz, CDCl₃) δ: 4.29-4.18 (m, 1H, Lys-α-C*H*<), 4.12 (s, 2H, -OC*H*₂C(=O)N<), 3.74-3.43 (m, PEG; 2H, Gly-CH₂-; 4H, -O(CH₂)₂N<; 2H, >NC*H*₂C(=O)NH-; 2H, -OC*H*₂CH₂C(=O)NH-; 2H, -C(=O)NHC*H*₂COOH), 3.36 (s, 9H, -OC*H*₃), 3.19-3.10 (m, 2H, Lys-ε-C*H*₂-), 2.53-2.45 (t, 2H, -OCH₂C*H*₂C(=O)NH-), 1.79-1.35 (m, 6H, Lys-β-C*H*₂-, Lys-γ-C*H*₂-, Lys-σ-C*H*₂-). The molecular weight determined by GPC was approximately 40.3 kDa, with PDI=1.05.

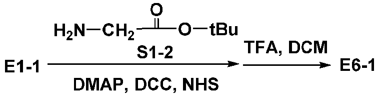

### Example 6.2: Preparation of three-arm monofunctional polyethylene glycol-aldehyde derivative E6-2

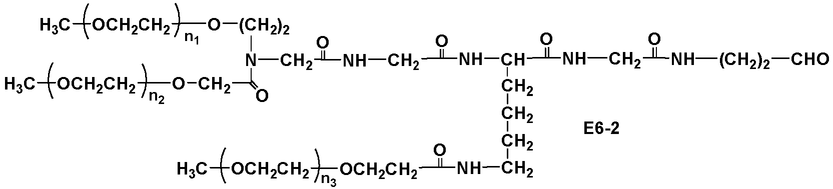

**E6-2** corresponds to general formula (3-2); wherein, L₃ is -NHCH₂C(=O)NH(CH₂)₂-, and R₀₁ is -CHO. The designed total molecular weight is approximately 40.3 kDa, with n₁≈n₂≈226 and n₃≈450.

The preparation method is as follows:
**E6-1** (20.15 g, 0.5 mmol, Mₙ≈40.3 kDa, n₁≈n₂≈226, n₃≈450, PDI=1.05) was dissolved in 200 mL anhydrous dichloromethane, followed by successively adding NHS (0.09 g, 0.8 mmol) and DCC (0.15 g, 0.8 mmol). DMAP (0.01 g, 0.1 mmol) was added to the solution of **S1-8** (0.74 g, 5.0 mmol) in 10 mL dichloromethane. After mixing the two aforementioned solutions, the reaction was carried out at room temperature for 24 h while stirring. After the reaction was over, the solution was filtered to remove insoluble substances and then concentrated. The crude product was dissolved with water, and the pH of the solution was adjusted to 2.0 with hydrochloric acid. The reaction was carried out for 16 hours while stirring. After the reaction was completed, 10% sodium chloride was added, and the pH of the solution was adjusted to 6.4±0.2 with sodium bicarbonate. The solution was extracted with dichloromethane. The organic phases were combined, dried over anhydrous magnesium sulfate, filtered, and recrystallized with anhydrous isopropanol to obtain **E6-2** (7.31 g). ¹H NMR (400 MHz, CDCl₃) δ: 4.31-4.20 (m, 1H, Lys-α-CH<), 4.12 (s, 2H, -OC*H*₂C(=O)N<), 3.74-3.45 (m, PEG; 4H, Gly-C*H*₂-; 4H, -O(C*H*₂)₂N<; 2H, >NC*H*₂C(=O)NH-; 2H, -OC*H*₂CH₂C(=O)NH-), 3.42-3.38 (m, 2H, -C(=O)NHCH₂C*H*₂CHO), 3.36 (s, 9H, -OC*H*₃), 3.18-3.10 (m, 2H, Lys-ε-C*H*₂-), 2.71-2.63 (m, 2H, -C(=O)NHCH₂C*H*₂CHO), 2.53-2.45 (t, 2H, -OCH₂C*H*₂C(=O)NH-), 1.79-1.32 (m, 6H, Lys-β-C*H*₂-, Lys-γ-C*H*₂-, Lys-σ-CH₂-). The molecular weight determined by GPC was approximately 40.3 kDa, with PDI=1.05.

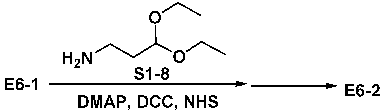

### Example 7: Three-arm monofunctional polyethylene glycol-dicarboxylic acid derivative E7-1

Three-arm monofunctional polyethylene glycol-carboxylic acid derivatives **E1-1**, **E2-1,** and **E3-1** can be further coupled with glutamic acid to obtain three-arm monofunctional polyethylene glycol-dicarboxylic acid derivatives containing two carboxyl groups. The following is an Example of the preparation of **E7-1** through the reaction between **E1-1** and glutamic acid containing tBu-protected carboxyl groups:

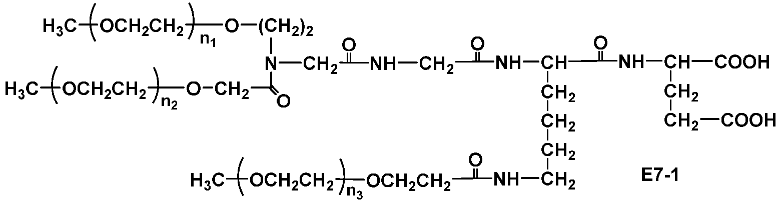

**E7-1** corresponds to general formula (4); wherein, L₂ is -CH₂CH₂C(=O)-, AA₂ is the trivalent residue of glutamic acid 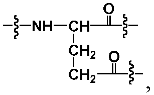 m is 2, two L₃ are both linking bonds, and two R₀₁ are both -OH. The designed total molecular weight is approximately 40.3 kDa, with n₁≈n₂≈226 and n₃≈450.

The preparation method is as follows:
**E1-1** (20.10 g, 0.5 mmol, Mₙ≈40.2 kDa, n₁≈n₂≈226, n₃≈450, PDI=1.05) was dissolved in 200 mL anhydrous dichloromethane, followed by successively adding NHS (0.09 g, 0.8 mmol) and DCC (0.15 g, 0.8 mmol). DMAP (0.01 g, 0.1 mmol) was added to the solution of glutamic acid **(S7-1,** 1.30 g, 5.0 mmol) containing two tBu-protected carboxyl groups in 20 mL dichloromethane. After mixing the two aforementioned solutions, the reaction was carried out at room temperature for 24 h while stirring. After the reaction was over, the solution was filtered to remove insoluble substances, and then concentrated under reduced pressure. The TFA/DCM mixed solution (1:1 v/v) was used to remove the tBu protection. After washing with purified water and extraction with dichloromethane, the extract was dried over anhydrous sodium sulfate, and then filtered, concentrated, and purified by column chromatography to obtain **E7-1** (10.46 g). ¹H NMR (400 MHz, CDCl₃) δ: 4.49-4.40 (m, 1H, Glu-α-C*H*), 4.27-4.19 (m, 1H, Lys-α-C*H*<), 4.12 (s, 2H, -OC*H*₂C(=O)N<), 3.76-3.42 (m, PEG; 2H, Gly-C*H*₂-; 4H, -O(C*H*₂)₂N<; 2H, >NC*H*₂C(=O)NH-; 2H, -OC*H*₂CH₂C(=O)NH-), 3.36 (s, 9H, -OC*H*₃), 3.22-3.15 (m, 2H, Lys-ε-C*H*₂-), 2.54-2.47 (t, 2H, -OCH₂C*H*₂C(=O)NH-), 2.42 (t, 2H, Glu-γ-C*H*₂-), 2.19-1.94 (m, 2H, Glu-β-C*H*₂-), 1.78-1.34 (m, 6H, Lys-β-C*H*₂-, Lys-γ-C*H*₂-, Lys-σ-C*H*₂-). The molecular weight determined by GPC was approximately 40.3 kDa, with PDI=1.05.

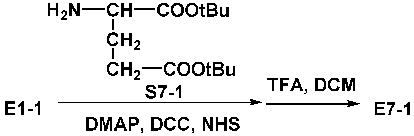

### Example 8: Three-arm monofunctional polyethylene glycol-dialdehyde derivative E8-1

The dicarboxylic acid derivative obtained through coupling **E1-1, E2-1** or **E3-1** with glutamic acid, by referring to **Example 7,** can be further used as the starting material in the preparation of the corresponding dialdehyde derivative. For example, dicarboxylic acid derivative **E7-1** can be used to prepare three-arm monofunctional polyethylene glycol-dialdehyde derivative **E8-1:**

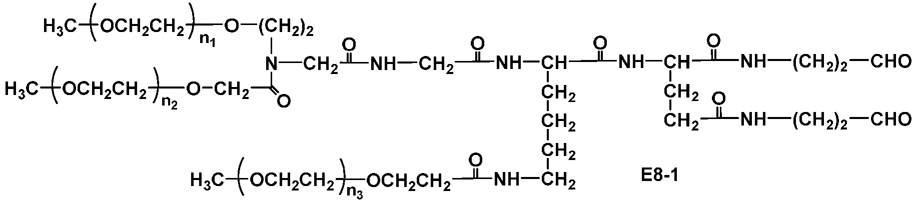

**E8-1** corresponds to general formula (4); wherein, L₂ is -CH₂CH₂C(=O)-, AA₂ is the trivalent residue of glutamic acid 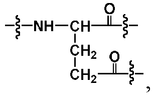 m is 2, two L₃ are both -NH(CH₂)₂-, and two R₀₁ are both -CHO. The designed total molecular weight is approximately 40.4 kDa, with n₁≈n₂≈226 and n₃≈450.

The preparation method is as follows:
**E7-1** (20.15 g, 0.5 mmol, Mₙ≈40.3 kDa, n₁≈n₂≈226, n₃≈450, PDI=1.05) was dissolved in 200 mL anhydrous dichloromethane, followed by successively adding NHS (0.09 g, 0.8 mmol) and DCC (0.15 g, 0.8 mmol). DMAP (0.01 g, 0.1 mmol) was added to the solution of **S8-1** (2.03 g, 5.0 mmol, **S8-1** was synthesized using **S1-8** and glutamic acid containing a Cbz-protected amino group as starting materials) in 30 mL dichloromethane. After mixing the two aforementioned solutions, the reaction was carried out at room temperature for 24 h while stirring. After the reaction was over, the solution was filtered to remove insoluble substances and then concentrated. The crude product was dissolved with water, and the pH of the solution was adjusted to 2.0 with hydrochloric acid. The reaction was carried out for 16 hours while stirring. After the reaction was completed, 10% sodium chloride was added, and the pH of the solution was adjusted to 6.4±0.2 with sodium bicarbonate. The solution was extracted with dichloromethane. The organic phases were combined, dried over anhydrous magnesium sulfate, filtered, and recrystallized with anhydrous isopropanol to obtain **E8-1** (8.04 g). ¹H NMR (400 MHz, CDCl₃) δ: 4.47-4.40 (m, 1H, Glu-α-C*H*), 4.29-4.20 (m, 1H, Lys-α-C*H*<), 4.14 (s, 2H, -OC*H*₂C(=O)N<), 3.75-3.44 (m, PEG; 2H, Gly-C*H*₂-; 4H, -O(C*H*₂)₂N<; 2H, >NC*H*₂C(=O)NH-; 2H, -OC*H*₂CH₂C(=O)NH-), 3.42-3.38 (m, 4H, -C*H*₂CH₂CHO), 3.36 (s, 9H, -OC*H*₃), 3.23-3.17 (m, 2H, Lys-ε-C*H*₂-), 2.74-2.63 (m, 4H, -CH₂C*H*₂CHO), 2.55-2.46 (t, 2H, -OCH₂C*H*₂C(=O)NH-), 2.41 (t, 2H, Glu-γ-C*H*₂-), 2.20-1.96 (m, 2H, Glu-β-C*H*₂-), 1.77-1.32 (m, 6H, Lys-β-C*H*₂-, Lys-γ-C*H*₂-, Lys-σ-C*H*₂-). The molecular weight determined by GPC was approximately 40.4 kDa, with PDI=1.05.

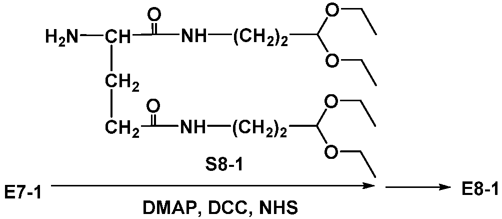

### Example 9: Three-arm monofunctional polyethylene glycol-carboxylic acid derivative E9-1

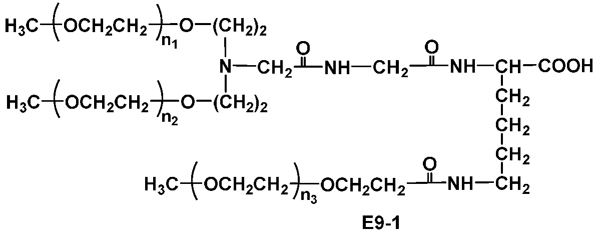

**E9-1** corresponds to general formula (3-5); wherein, L₃ is a linking bond, and R₀₁ is -OH. The designed total molecular weight is approximately 40.2 kDa, with n₁≈226, n₂≈226, and n₃≈450.

The preparation method is as follows:
Step a: First alkylation reaction: Methoxy polyethylene glycol-sulfonate derivative **S9-3** (12.12 g, 1.2 mmol, Mₙ≈10.1 kDa, n₁≈226, PDI=1.03) was dissolved in 60 mL dichloromethane, followed by adding small-molecule starting material **S9-2** (5.78 g, 12.0 mmol, prepared using glycine containing a Boc-protected amino group and **S1-4** as starting materials), and the reaction was carried out for 24 hours at room temperature. After the reaction was over, the reaction solution was concentrated, and then precipitated twice in ether to remove impurities to obtain intermediate **S9-4** (10.84 g).
Step b: Second alkylation reaction: **S9-4** (8.40 g, 0.8 mmol, Mₙ≈10.5 kDa, n₁≈226, PDI=1.03) and **S9-3** (12.12 g, 1.2 mmol) were dissolved in 100 mL dichloromethane. The reaction was carried out for 24 hours at room temperature. After the reaction was over, the reaction solution was concentrated, and then a phosphate buffer of pH=7.0 was added. The reaction was stirred for 16 h. Purification by column chromatography was performed to obtain intermediate **S9-5** (8.61 g) containing a symmetric nitrogen-branched two-arm polyethylene glycol structure.
Step c: **S1-6** (7.96 g, 0.4 mmol, Mₙ≈19.9 kDa, n₃≈450, PDI=1.04) was dissolved in 50 mL anhydrous dichloromethane, followed by successively adding NHS (0.07 g, 0.6 mmol) and DCC (0.12 g, 0.6 mmol). DMAP (0.01 g, 0.1 mmol) was added to the solution of S9-5 (8.12 g, 0.4 mmol, Mₙ≈20.3 kDa, n₁≈n₂≈226, PDI=1.04) in 50 mL dichloromethane. After mixing the two aforementioned solutions, the reaction was carried out at room temperature for 24 h while stirring. After the reaction was over, the solution was filtered to remove insoluble substances, and then concentrated under reduced pressure. The TFA/DCM mixed solution (1:1 v/v) was used to remove the tBu protection. After washing with purified water and extraction with dichloromethane, the extract was dried over anhydrous sodium sulfate, and then filtered, concentrated, and purified by column chromatography to obtain **E9-1** (5.50 g). ¹H NMR (400 MHz, CDCl₃) δ: 4.26-4.18 (m, 1H, Lys-α-C*H*<), 3.72-3.43 (m, PEG; 2H, Gly-C*H*₂-; 2H, -OC*H*₂CH₂C(=O)NH-; 4H, -OC*H*₂CH₂N<), 3.37 (s, 9H, -OC*H*₃), 3.27 (s, 2H, >NC*H*₂C(=O)NH-), 3.21-3.11 (m, 2H, Lys-ε-C*H*₂-), 2.80 (t, 2H, -OCH₂C*H*₂N<), 2.52-2.44 (t, 2H, -OCH₂C*H*₂C(=O)NH-), 1.90-1.34 (m, 6H, Lys-β-C*H*₂-, Lys-γ-C*H*₂-, Lys-σ-C*H*₂-). The molecular weight determined by GPC was approximately 40.2 kDa, with PDI=1.07.

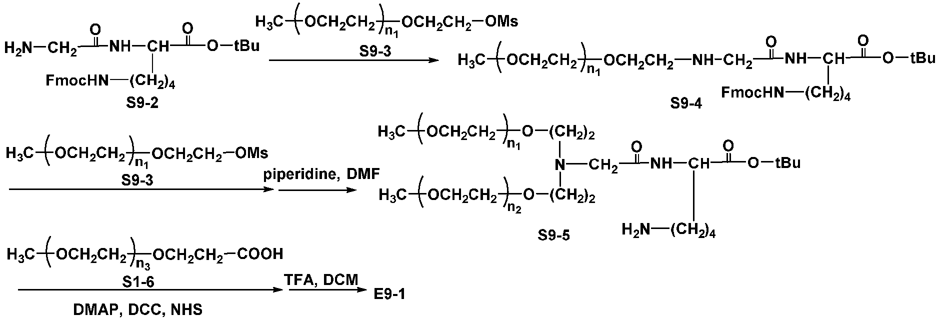

### Example 10: Three-arm monofunctional polyethylene glycol-modified irinotecan derivative E10-1

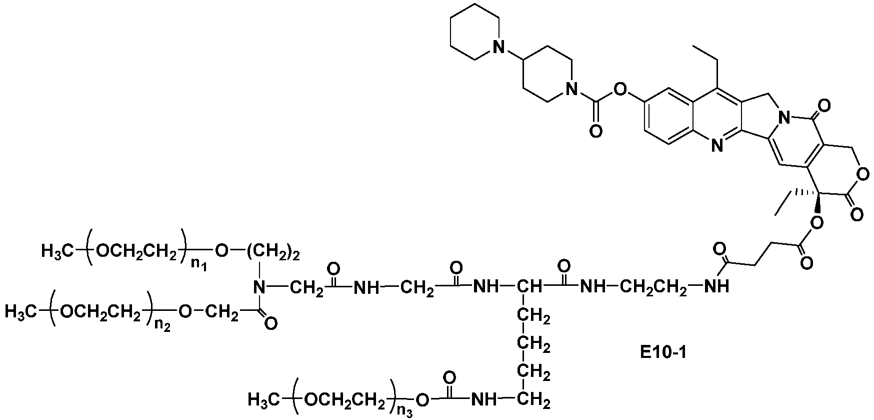

E10-1 corresponds to general formula (5); wherein, P is the residue of E4-1, L is a linking bond, and D is the residue of propionic acid derivative of irinotecan. The designed total molecular weight is approximately 40.9 kDa, with n₁≈226, n₂≈226, and n₃≈450.

The preparation method is as follows:
E4-1 (20.10 g, 0.5 mmol, Mₙ≈40.2 kDa, n₁≈n₂≈226, n₃≈450, PDI=1.06) was dissolved in 200 mL anhydrous dichloromethane, followed by successively adding NHS (0.09 g, 0.8 mmol) and DCC (0.15 g, 0.8 mmol). DMAP (0.01 g, 0.1 mmol) was added to the solution of propionic acid derivative of irinotecan **(S10-1,** 3.44 g, 5.0 mmol) in 50 mL dichloromethane. After mixing the two aforementioned solutions, the reaction was carried out at room temperature for 24 h while stirring. After the reaction was over, the solution was filtered to remove insoluble substances, concentrated, and purified by column chromatography to obtain **E10-1** (6.93 g). ¹H NMR (400 MHz, CDCl₃) δ: 8.19-8.17 (d, 1H), 7.84 (s, 1H), 7.59 (dd, 1H), 7.17 (s, 1H), 5.70-5.39 (m, 2H), 5.24 (s, 2H), 4.50-4.39 (m, 2H), 4.26-4.23 (m, 3H), 4.13 (s, 2H), 3.76-3.43 (m, PEG+10H), 3.36 (s, 9H), 3.30-3.24 (m, 4H), 3.18-3.06 (m, 6H), 2.95-2.88 (m, 1H), 2.82-2.74 (m, 4H), 2.72-2.56 (m, 4H), 2.52-2.43 (m, 2H), 2.08-1.32 (m, 19H), 0.97 (t, 3H). The molecular weight determined by GPC was approximately 40.9 kDa, with PDI=1.06.

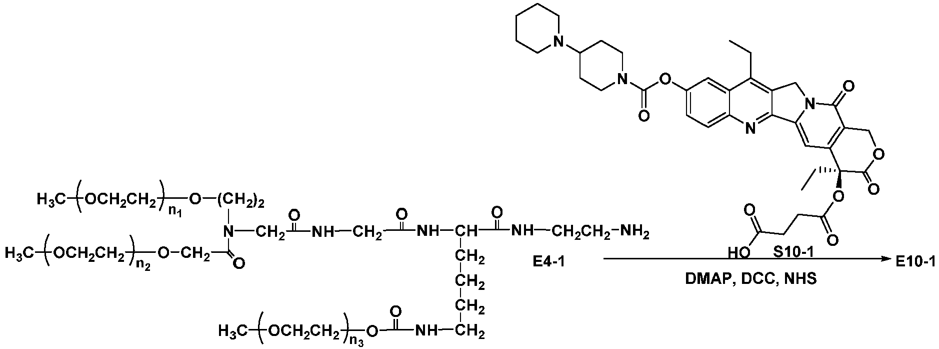

### Example 11: Biological activity assays

### (1) Evaluation of stability in serum

1 mL of mouse serum was added to each of 18 microcentrifuge tubes, each with a volume of 1.3 mL; wherein, 15 tubes were added with polyethylene glycol derivatives of the present invention, 1 tube was added with the polyethylene glycol derivative of the comparative example **C-1** (30.2 kDa) containing no degradable groups, 1 tube was added with the three-arm polyethylene glycol derivative of the comparative example **C-2** (40.4 kDa) containing a residue of lysine-lysine dipeptide, and 1 tube was added with the three-arm polyethylene glycol derivative of the comparative example **C-3** (40.5 kDa) containing a residue of glycine-lysine-lysine tripeptide. The concentration of polyethylene glycol derivative was set at 5.0 mg/mL for each tube. After 96 hours of incubation at 37°C, 200 µL samples were taken. Removing proteins from serum: acetonitrile was added, and a vortexer was used to stir for 1 minute to precipitate the proteins in serum, followed by centrifugation to recover the supernatant. Removing fatty acids and other hydrophobic substances: hexane was added to the recovered liquid, and a vortexer was used to stir for 1 minute, followed by centrifugation for separation. The lower layer was recovered and then concentrated under vacuum condition to recover the polyethylene glycol derivatives. The molecular weight was determined by GPC, and the degradation rate was calculated according to the following formula: Degradation Rate = (Peak Area % of Mn before the assay - Peak Area % of Mn after the assay)/(Peak Area % of Mn before the assay) × 100% wherein, Mₙ is the average molecular weight of polyethylene glycol derivative; for example, the average molecular weight Mₙ **of E1-1** is 40.2 kDa.

The comparative examples **C-1, C-2,** and **C-3** were synthesized by referring to CN1243779C, CN1995094A, and CN101168594A, respectively, and the structures are as follows: 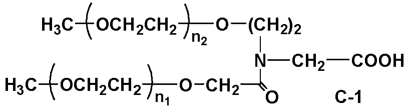 n₁ is about 340, n₂ is about 340, Mₙ is about 30.2 kDa, PDI=1.04 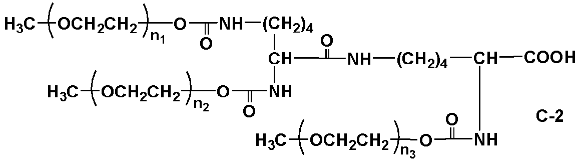
n₁ is about 226, n₂ is about 226, n₃ is about 454, Mₙ is about 40.4 kDa, PDI=1.05 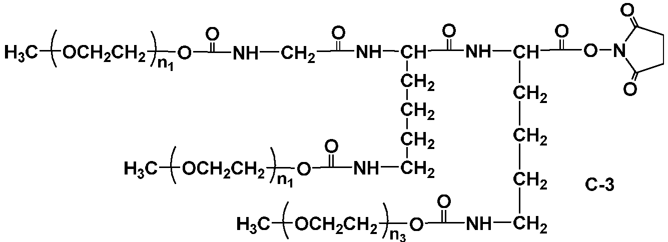
n₁ is about 226, n₂ is about 226, n₃ is about 454, Mₙ is about 40.5 kDa, PDI=1.05

The measured results of the degradation rate in mouse serum are shown in Table 1. The results indicate that the three-arm monofunctional polyethylene glycol derivatives of the present invention, with nitrogen-branched structures, all have a degradation rate not exceeding 5%, which is slightly lower than those of the three-arm polyethylene glycol derivatives of the comparative examples with branched structures that are all based on carbon cores (the degradation rates of **C-2** and **C-3** in serum are 9% and 7%, respectively), indicating that the three-arm monofunctional polyethylene glycol derivatives of the present invention exhibit better stability in serum.

**Table 1. Degradation Rate of Polyethylene Glycol Derivatives in Mouse Serum**

| PEG | **E1-1** | **E1-1** | **E1-3** | **E1-4** | **E1-5** | **E2-1** | **E2-2** |
|---|---|---|---|---|---|---|---|
| Degradation Rate /% | 0 | 0 | 0 | 2 | 0 | 0 | 0 |

| PEG | **E3-1** | **E3-2** | **E4-1** | **E5-1** | **E6-1** | **E6-2** | **E7-1** |
|---|---|---|---|---|---|---|---|
| Degradation Rate /% | 3 | 5 | 2 | 3 | 0 | 0 | 0 |

| PEG | **E8-1** | **E9-1** | **C-1** | **C-2** | **C-3** | | |
|---|---|---|---|---|---|---|---|
| Degradation Rate /% | 0 | 0 | 0 | 9 | 7 | | |

### (2) Evaluation of intracellular degradability

Experimental groups: 10 mL of RPMI-1640 (10% FBS Pn/St) culture medium was used, to inoculate 10×10⁶ cells of RAW264.7 in a 100 mm culture dish. After 24 hours of incubation at 37°C, a culture medium containing 10.0 mg/mL of the polyethylene glycol derivative of the present invention or the polyethylene glycol derivative of the comparative example was used for replacement. After further incubation for 96 hours at 37°C, the cells were dissolved with 1% SDS solution and diluted with PBS. Removing proteins: acetonitrile was added, and a vortexer was used to stir for 1 minute to precipitate proteins, followed by centrifugation to recover the supernatant. Removing fatty acids and other hydrophobic substances: hexane was added to the recovered liquid, and a vortexer was used to stir for 1 minute, followed by centrifugation for separation. The lower layer was recovered and then concentrated under vacuum condition to recover the polyethylene glycol derivatives.

Control groups: A culture medium containing 10.0 mg/mL of the polyethylene glycol derivative of the present invention or the polyethylene glycol derivative of the comparative example was left for 96 hours at 37°C without inoculating cells. The polyethylene glycol derivatives were recovered following the same procedure as the experimental groups.

The recovered polyethylene glycol derivatives were analyzed by GPC, and the degradation rate was calculated according to the aforementioned formula. The results are summarized in Table 2. The results indicate that the three-arm monofunctional polyethylene glycol derivatives with nitrogen-branched structures have high degradation rates within cells, wherein the degradation rates of **E3-1** and **E3-2** are above 98% which is significantly higher than those of the comparative examples.

**Table 2. Degradation Rates of Polyethylene Glycol Derivatives in Cells/Culture Media**

| PEG | **E1-1** | **E1-1** | **E1-3** | **E1-4** | **E1-5** | **E2-1** | **E2-2** |
|---|---|---|---|---|---|---|---|
| Degradation rate of experimental group/% | 87 | 88 | 84 | 90 | 95 | 84 | 86 |
| Degradation rate of control group /% | 0 | 0 | 0 | 0 | 0 | 0 | 0 |

| PEG | **E3-1** | **E3-2** | **E4-1** | **E5-1** | **E6-1** | **E6-2** | **E7-1** |
|---|---|---|---|---|---|---|---|
| Degradation rate of experimental group/% | 98 | 100 | 94 | 97 | 93 | 96 | 93 |
| Degradation rate of control group /% | 0 | 0 | 0 | 0 | 0 | 0 | 0 |

| PEG | **E8-1** | **E9-1** | **C-1** | **C-2** | **C-3** | | |
|---|---|---|---|---|---|---|---|
| Degradation rate of experimental group/% | 92 | 85 | 0 | 68 | 81 | | |
| Degradation rate of control group /% | 0 | 0 | 0 | 0 | 0 | | |

### (3) Evaluation of cell vacuole formation

Three-arm polyethylene glycol derivative **E3-2** (40.4 kDa) of the present invention, which contains a residue of glycine-glycine-lysine tripeptide, as well as the comparative example **C-1** (30.2 kDa) which contains no degradable groups, the three-arm polyethylene glycol derivative of the comparative example **C-2** (40.4 kDa) which contains a residue of lysine-lysine dipeptide, and the three-arm polyethylene glycol derivative of the comparative example **C-3** (40.5 kDa) which contains a residue of glycine-lysine-lysine tripeptide, were evaluated for cellular vacuole formation. Balb/c mice (8 weeks old, male) were divided into a blank group, three control groups **(C-1, C-2,** and **C-3)** and an experimental group **(E3-2),** and each group contained four mice. The blank group received a tail vein injection of 20 µL of normal saline, while the control groups received a tail vein injection of 20 µL of the normal saline solution of the polyethylene glycol derivatives of the comparative examples (200 mg/mL), and the experimental group received a tail vein injection of 20 µL of the normal saline solution of **E3-2** (200 mg/mL). The injections were administered once every 3 days for 10 consecutive doses. After administration, the mice were perfused and fixed with 4% paraformaldehyde aqueous solution to prepare paraffin sections. Hematoxylin and eosin (HE) staining and immunostaining using anti-PEG antibodies were performed to evaluate the vacuole formation in the choroid plexus epithelial cells of the brain. The results showed that obvious cell vacuoles were generated in the mice of the control groups injected with **C-1** and **C-2,** while no vacuole formation was observed for the blank group, the control group injected with **C-3,** and the experimental group injected with **E3-2.** Referring to the intracellular degradability test results, it indicates that the three-arm polyethylene glycol derivative of the present invention can significantly inhibit the cell vacuole formation.

### (4) Biological assays of PEGylated irinotecan

### Cytotoxicity assay:

Cytotoxicity assay of PEGylated irinotecan **E10-1** (wherein the polyethylene glycol component is derived from **E4-1)** was performed using MTT staining. A blank control group containing no drugs but only culture medium, a positive control group containing irinotecan, and an experimental group containing PEGylated irinotecan **(E10-1)** were respectively set up. The drug concentration of the positive control group and experimental group was 10 nM, with 6 replicate wells each, and the assay was repeated three times. Six types of cancer cells were selected as in vitro cancer cell models: human colon cancer cell COLO205, human colon adenocarcinoma cell HT29, human lung adenocarcinoma cell A549, pancreatic cancer cell MiaPaCa-2, human ovarian cancer cell A2780, and human ovarian adenocarcinoma cell OVCAR-3.

Cells were inoculated onto a 96-well plate at a density of 1×10⁴ cells/well, with 100 µL of cell suspension added to each well. After the inoculation, the cells were incubated in a cell culture incubator at 37°C, 4% CO₂ for 24 h. The old culture medium was aspirated, and then 100 µL of culture medium containing 10 nM **E10-1** was added to each experimental group, 100 µL of culture medium containing 10 nM irinotecan was added to each positive control group, and 100 µL of fresh culture medium was added to the blank control group. After further incubation for 48 h, 20 µL of 5 mg/mL MTT in PBS buffer was added to each well. After incubating the cancer cells with MTT for 4 h, the mixture of culture medium and MTT buffer was aspirated, and then DMSO (150 µL/well) was added to dissolve the blue-purple crystalline product of formazan in viable cells. The 96-well plate was gently shaken until the crystalline product was fully dissolved, and then a microplate reader was used to measure the absorbance at 490 nm. Cell Viability = (Absorbance of the drug group/Absorbance of the blank group) × 100%.

The data of cytotoxicity assay are as follows:

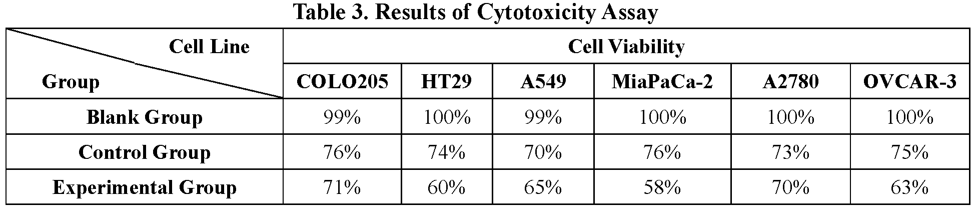

The above results show that both the positive control group (irinotecan) and the experimental group **(E10-1)** have a significant inhibitory effect on the proliferation of the six types of cancer cells mentioned above, and the cell viability of the experimental group is even slightly lower than that of the positive control group, indicating that the three-arm polyethylene glycol derivative of the present invention retains the drug activity of the modified irinotecan.

### Anti-tumor efficacy in vivo:

The transplantation of animal tumors was adopted as the experimental method, with 6 mice per group, and H22 mouse liver cancer cells were inoculated subcutaneously into the right axilla of the mice to form solid tumors. Single-dose administrations via tail vein injection were performed on days 2 and 7 post-inoculation, respectively. After 2 weeks of administration, the mice were sacrificed via cervical dislocation, and the tumors were excised and weighed.

Tumor Inhibition Rate = [G-H/G]*100% (G represents the average tumor weight of the blank control group, H represents the average tumor weight of the treatment group).

**Table 4. Results of Anti-tumor Effect**

| | Blank Control Group | Irinotecan Group | **E10-1** Group |
|---|---|---|---|
| Tumor Inhibition Rate | / | 30.3% | 64.7% |

The results indicate that, compared with the blank control, both irinotecan and the two-arm polyethylene glycol-modified irinotecan derivative **(E10-1)** have significant anti-tumor effects, and the tumor inhibition rate of **E10-1** is significantly higher than that of irinotecan alone, indicating that the three-arm polyethylene glycol derivative of the present invention can effectively protect the drug and enable the drug to fully exert its therapeutic effects in vivo.

Those described above are only embodiments of the present invention, not limiting the patent scope of the present invention. Any transformation of equivalent structure or equivalent process based on the specification content of the present invention, directly or indirectly applied in other related arts, should be similarly included in the protected patent scope of the present invention.

For those skilled in the art, without deviating from the purpose and the scope of the present invention and without unnecessary experimentation, the present invention can be implemented within a broad range with equivalent parameters, concentrations, and conditions. Although specific embodiments are provided in the present invention, it should be understood that the present invention may be further modified. In conclusion, in accordance with the principles of the present invention, the present application is intended to encompass any alterations, uses, or improvements to the present invention, including those that extend beyond the scope disclosed in this application and involve modifications using conventional techniques known in the art.

## Claims

1. A three-arm polyethylene glycol derivative having the structure represented by general formula (1): 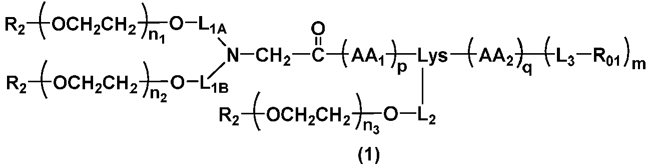
or a pharmaceutically acceptable salt, tautomer, stereoisomer or solvate thereof;
wherein,
n₁, n₂, and n₃ are the degrees of polymerization of the polyethylene glycol chains, each being independently an integer in the range of 30 to 1000;
R₂ is, at each occurrence, independently a C₁₋₆ alkyl group;
L_{1A}, L_{1B}, and L₂ are each independently a divalent linking group;
Lys is a residue of lysine;
AA₁ and AA₂ are each independently a residue of amino acid or amino acid derivative;
p is an integer in the range of 1 to 5, and -(AA₁)ₚ- represents a divalent residue formed by the combination of p units of AA₁; q is 0 or 1, indicating the absence of AA₂ or the presence of one AA₂;
L₃ is a linking bond or a divalent linking group;
R₀₁ is H, a functional group capable of reacting with bio-related substances, or a protected form of the functional group; m is 1 or 2; when m is 2, the structures of two L₃ are identical or different, and the structures of two R₀₁ are identical or different;
the three-arm polyethylene glycol derivative is monodisperse or polydisperse.

2. The three-arm polyethylene glycol derivative according to claim 1, wherein the number average molecular weight of each PEG chain is 1000, 1500, 2000, 2500, 3000, 3350, 3500, 4000, 5000, 5500, 6000, 6500, 7000, 7500, 8000, 8500, 9000, 9500, 10000, 11000, 12000, 13000, 14000, 15000, 16000, 17000, 18000, 19000, or 20000 Da.

3. The three-arm polyethylene glycol derivative according to claim 1, wherein n₁, n₂, and n₃ are each independently an integer in the range of 30 to 500, preferably in the range of 100 to 500.

4. The three-arm polyethylene glycol derivative according to claim 1, wherein R₀₁ is selected from the group consisting of an epoxy group, a hydroxyl group, a thiol group, a carboxyl group, an amino group, an aldehyde group, an active ester group, an active carbonate group, a carbamate group, an isocyanate group, an isothiocyanate group, a succinimide group, a maleimide group, an alkenyl group, an alkynyl group, an enoate group, an azido group, a cyano group, a dithiopyridyl group, an α-haloacetyl alkynyl group, a folic acid group, a rhodamine group, a biotin group, a monosaccharide group, a polysaccharide group, and protected forms thereof; preferably, R₀₁ is selected from the group consisting of the following structures:

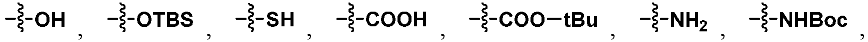

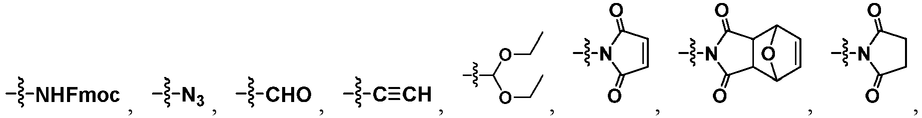

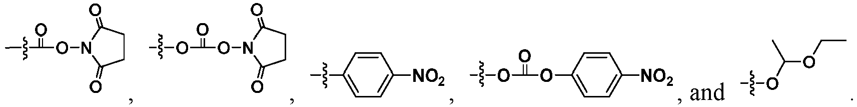

5. The three-arm polyethylene glycol derivative according to claim 1, wherein L₂ is selected from the group consisting of -(CH₂)ₜₚ-, -(CH₂)_{tq}C(=O)-, -C(=O)(CH₂)_{tq}C(=O)-, -(CH₂)_{tq}NHC(=O)(CH₂)_{tq}C(=O)-, and -(CH₂)_{tq}C(=O)NH(CH₂)_{tq}C(=O)-; wherein, tp is an integer in the range of 1 to 4; tq is, at each occurrence, independently an integer in the range of 0 to 4; the right end of the linking group is connected to the residue of lysine of general formula (1); L₂ is preferably selected from the group consisting of -C(=O)-, -CH₂C(=O)-, -CH₂CH₂C(=O)-, -CH₂CH₂CH₂C(=O)-, -C(=O)CH₂CH₂C(=O)-, -C(=O)CH₂CH₂CH₂C(=O)-, -CH₂CH₂NHC(=O)CH₂CH₂C(=O)-, -CH₂CH₂CH₂NHC(=O)CH₂CH₂C(=O)-, -CH₂CH₂NHC(=O)CH₂CH₂CH₂C(=O)-, -CH₂CH₂CH₂NHC(=O)CH₂CH₂CH₂C(=O)-, -CH₂C(=O)NHCH₂C(=O)-, -CH₂CH₂C(=O)NHCH₂C(=O)-, and -CH₂CH₂CH₂C(=O)NHCH₂C(=O)-.

6. The three-arm polyethylene glycol derivative according to claim 1, wherein L₃ is selected from the group consisting of a linking bond, an alkylene group, -C(=O)-, -OC(=O)-, -C(=O)O-, -OC(=O)O-, -O-, -S-, -S-S-, -C(=O)S-, -SC(=O)-, -NR_{c}-, -NR_{c}C(=O)-, -C(=O)NR_{c}-, -NR_{c}C(=O)NR_{c}-, -OC(=O)NR_{c}-, -NR_{c}C(=O)O-, -SC(=O)NR_{c}-, -NR_{c}C(=O)S-, and combinations thereof;
R_{c} is selected from the group consisting of H, a C₁₋₆ alkyl group, a carbocyclic group, and a heterocyclic group; preferably, R_{c} is H;
the alkylene group is preferably -(CRₐR_{b})ₜ-; wherein, t is, at each occurrence, independently an integer in the range of 1 to 12; Rₐ and R_{b} are each independently H, a C₁₋₆ alkyl group, a carbocyclic group, or a heterocyclic group; t instances of Rₐ and t instances of R_{b} are identical, or a combination of two or more different structures; preferably, Rₐ and R_{b} are, at each occurrence, independently -CH₃ or -H; more preferably, Rₐ and R_{b} are both H; preferably, t is an integer in the range of 1 to 4; more preferably, t is 1 or 2.

7. The three-arm polyethylene glycol derivative according to claim 1, wherein p instances of AA₁ and q instances of AA₂ are each independently selected from the group consisting of the residues of glycine, alanine, β-alanine, valine, leucine, isoleucine, phenylalanine, tryptophan, tyrosine, aspartic acid, histidine, asparagine, glutamic acid, lysine, glutamine, methionine, arginine, serine, threonine, cysteine, ornithine, citrulline, and derivatives thereof;
when p=1, -AA₁- is preferably a residue of glycine;
when p≥2, -(AA₁)ₚ- is a residue of oligopeptide or oligopeptide derivative; the oligopeptide is a dipeptide, tripeptide, tetrapeptide, or pentapeptide, and is preferably selected from the group consisting of glycine-phenylalanine, histidine-β-alanine, glycine-glycine, lysine-glycine, lysine-glutamic acid, glycine-glycine-glycine, glycine-glycine-phenylalanine, glycine-phenylalanine-glycine, glycine-lysine-glycine, glycine-histidine-lysine, glycine-cysteine-glutamic acid, glycine-leucine-glycine, valine-citrulline-glycine, valine-alanine-glycine, arginine-glycine-aspartic acid, glycine-histidine-proline, glycine-leucine-phenylalanine-glycine, glycine-glycine-phenylalanine-glycine, glycine-lysine-glycine-lysine, glycine-phenylalanine-glutamic acid-phenylalanine-glycine, and arginine-lysine-aspartic acid-valine-tyrosine, more preferably selected from the group consisting of glycine-phenylalanine, histidine-β-alanine, glycine-glycine, lysine-glycine, glycine-glycine-phenylalanine, glycine-cysteine-glutamic acid, and glycine-leucine-phenylalanine-glycine;
when q is 1, AA₂ is preferably a residue of glutamic acid or aspartic acid.

8. The three-arm polyethylene glycol derivative according to claim 7, wherein p is 1 and AA₁ is a residue of glycine; the structure of the three-arm polyethylene glycol derivative is represented by general formula (2): 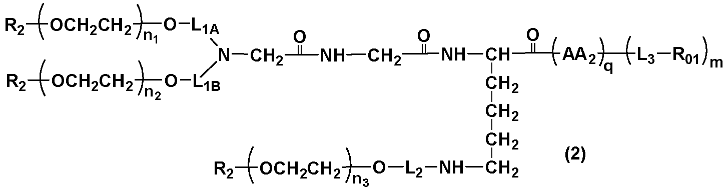 wherein,
L_{1A} and L_{1B} are each independently a C₁₋₆ alkylene group or -(CH₂)₁₋₆C(=O)-, wherein the left end of each linking group is connected to a PEG chain; more preferably, L_{1A} and L_{1B} are each independently selected from the group consisting of -CH₂CH₂-, -CH₂-, -CH₂C(=O)-, and -CH₂CH₂C(=O)-; more preferably, L_{1A} and L_{1B} conform to any one of the following cases:
Case (1): L_{1A} and L_{1B} are each independently -CH₂CH₂- or -CH₂-;
Case (2): one of L_{1A} and L_{1B} is -CH₂CH₂- or -CH₂-, and the other is -CH₂C(=O)- or -CH₂CH₂C(=O)-.

9. The three-arm polyethylene glycol derivative according to claim **8,** wherein q is 0 and AA₂ is absent; the structure of the three-arm polyethylene glycol derivative is represented by general formula (3): 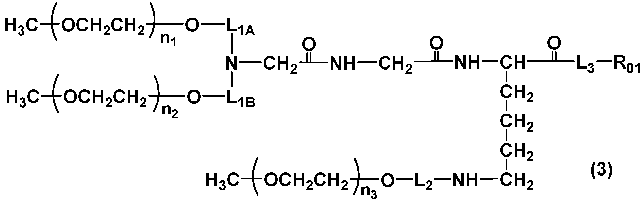
wherein, L₃ is preferably selected from the group consisting of a linking bond, -O-, -O(CH₂)ₜₚOC(=O)O-, -NH(CH₂)ₜₚ-, -NH(CH₂)ₜₚC(=O)NH(CH₂)ₜₚ-, and -NH(CH₂)ₜₚNHC(=O)(CH₂)ₜₚ-, and the right end of L₃ is connected to R₀₁; tp is an integer in the range of 1 to 4, preferably 1 or 2;
R₀₁ is preferably selected from the group consisting of -OH, -CHO, -COOH, -NH₂, 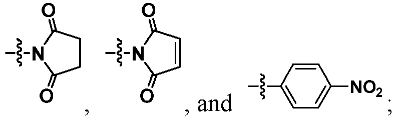
the structure of the three-arm polyethylene glycol derivative is more preferably selected from the group consisting of the following structural formulas: 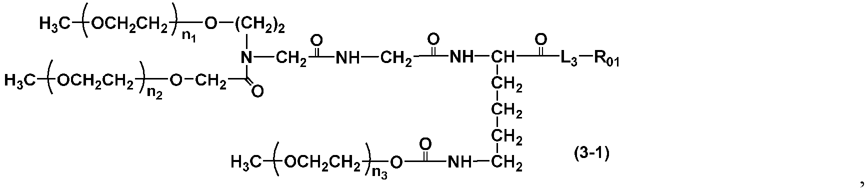 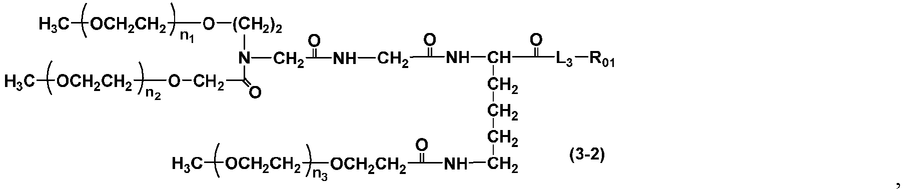 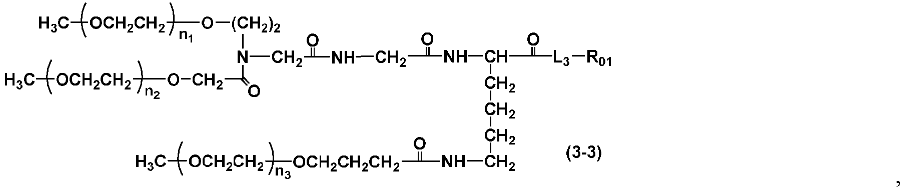 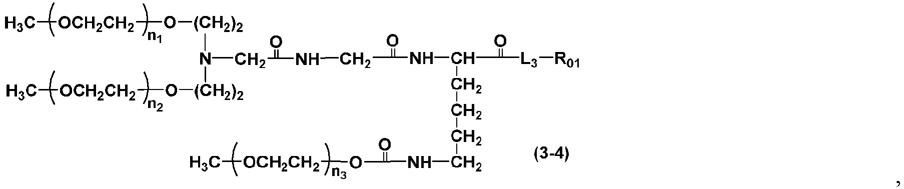 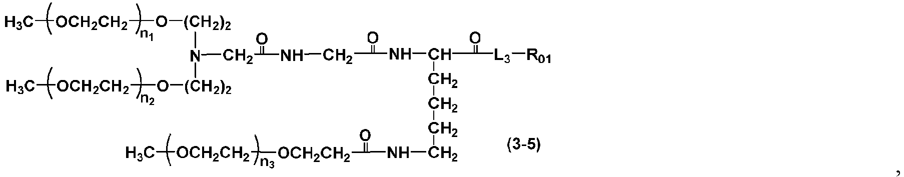 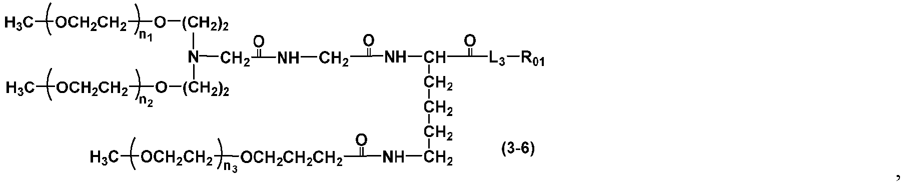 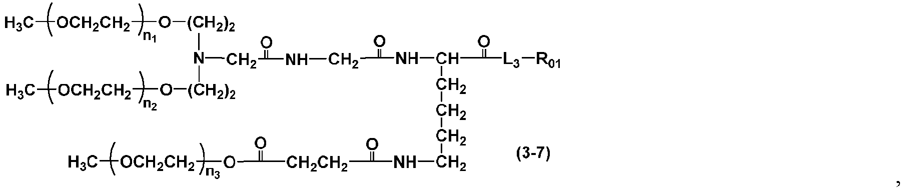 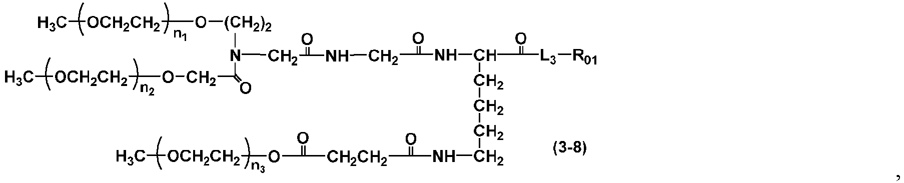 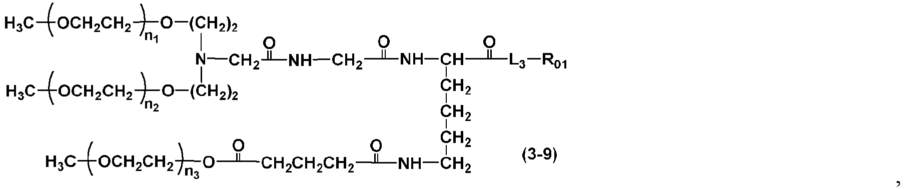 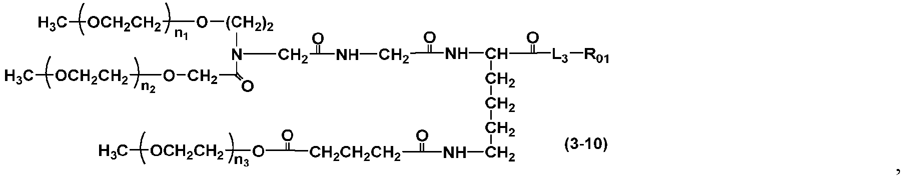 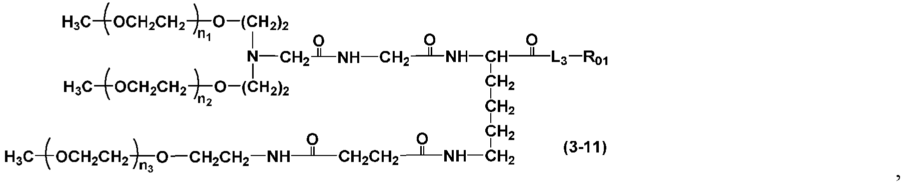 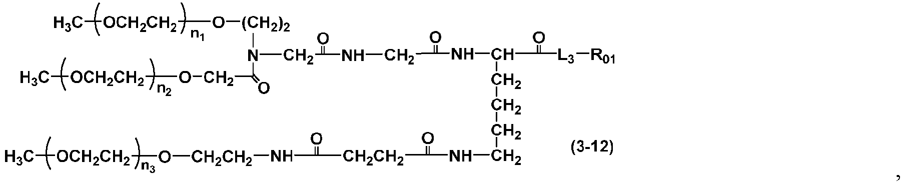 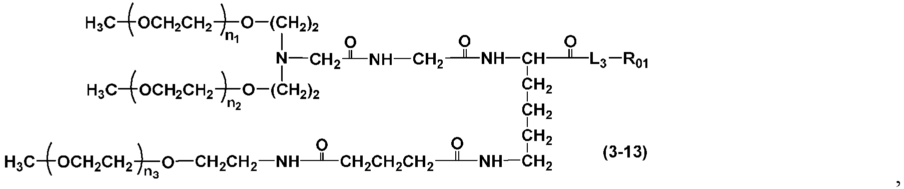 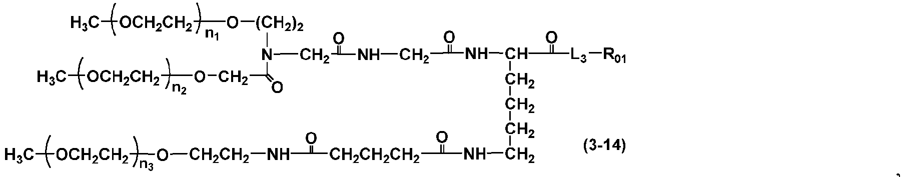 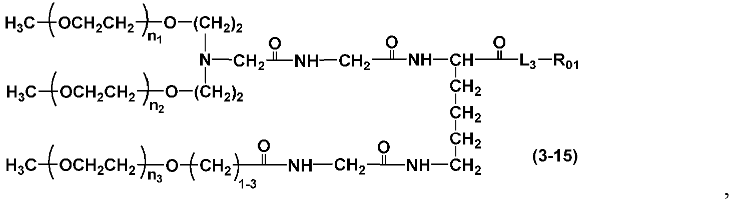 and

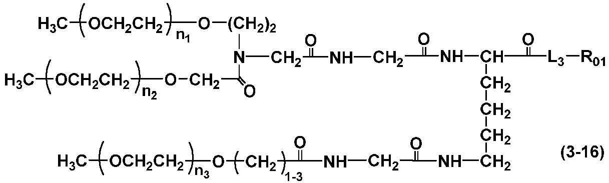

10. The three-arm polyethylene glycol derivative according to claim **8,** wherein q is 1 and one AA₂ is present; the structure of the three-arm polyethylene glycol derivative is represented by general formula (4): 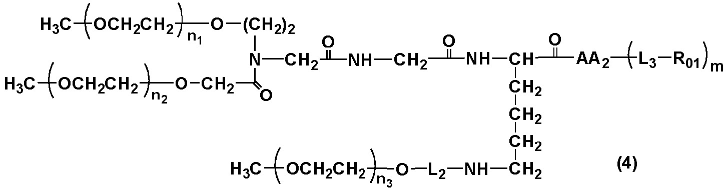 wherein,
R₀₁ is preferably -OH or -CHO;
L₃ is preferably a linking bond or -NH(CH₂)ₜₚ-, and the right end of L₃ is connected to R₀₁; tp is an integer in the range of 1 to 4, preferably 1 or 2;
AA₂ is preferably a trivalent residue of amino acid or amino acid derivative, more preferably a trivalent residue of glutamic acid or aspartic acid;
the structure of the three-arm polyethylene glycol derivative is more preferably selected from the group consisting of the following structural formulas: 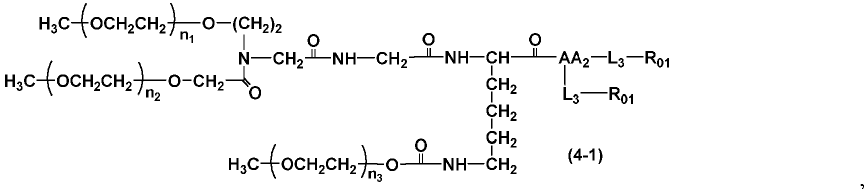 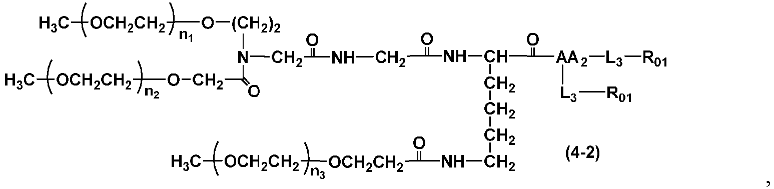 and

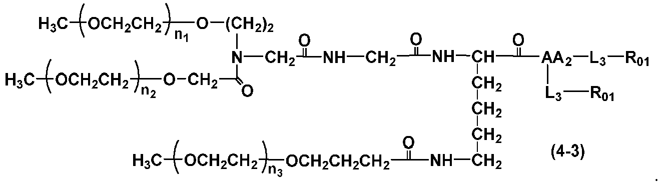

11. The three-arm polyethylene glycol derivative according to claim 1, wherein the structure is selected from the group consisting of the following structural formulas: 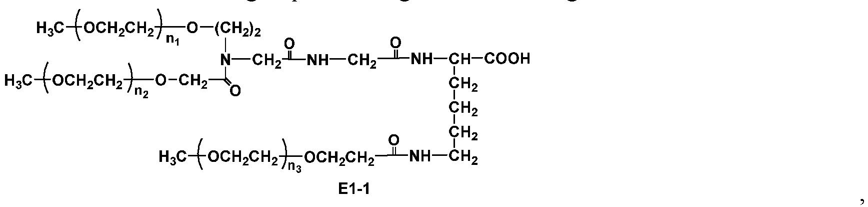 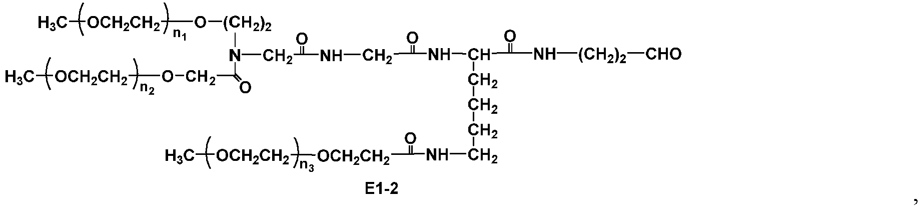 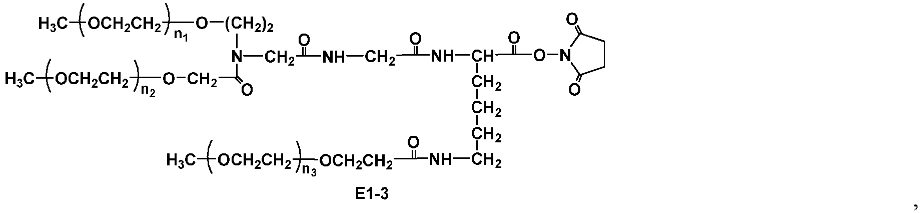 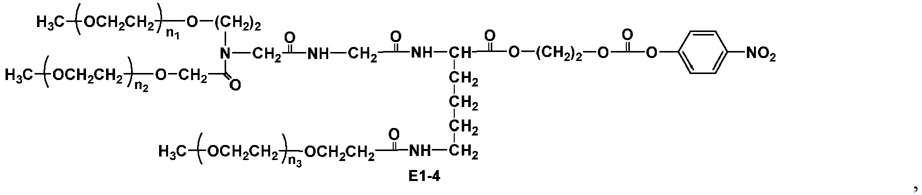 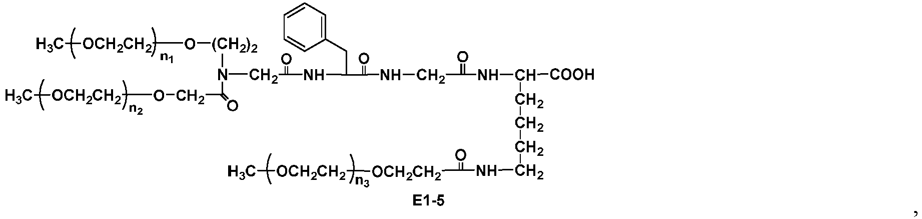 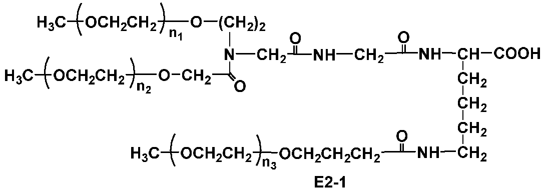 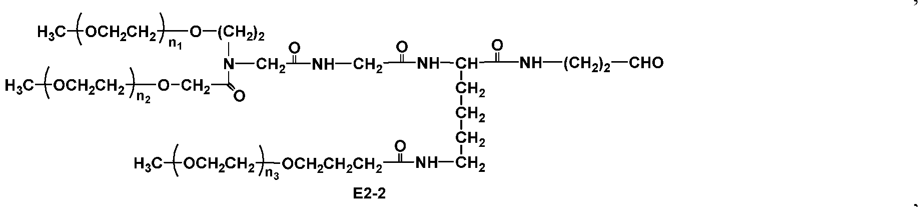 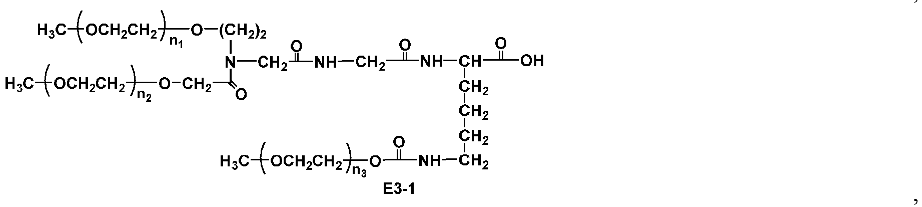 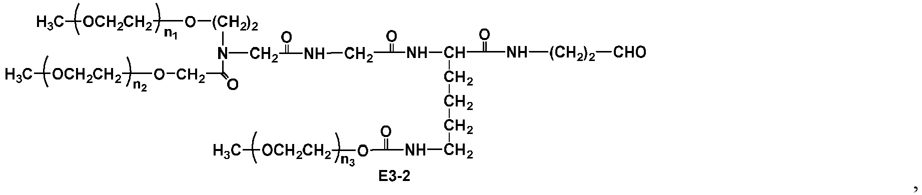 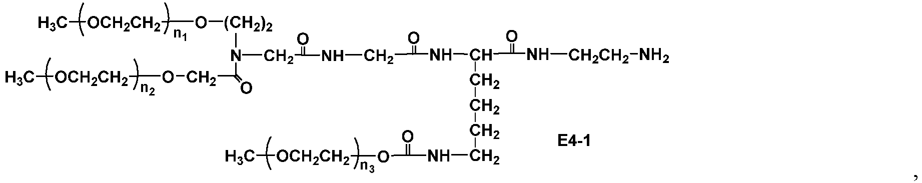 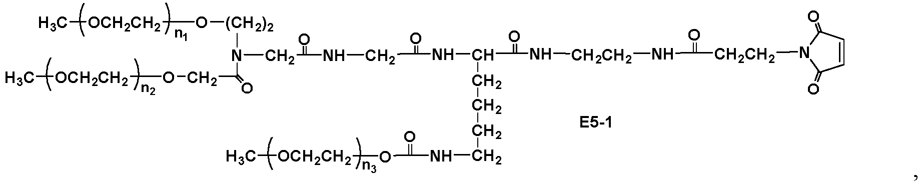 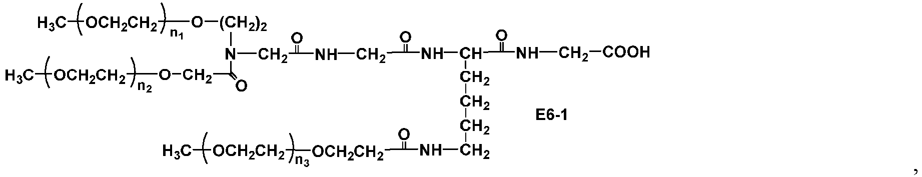 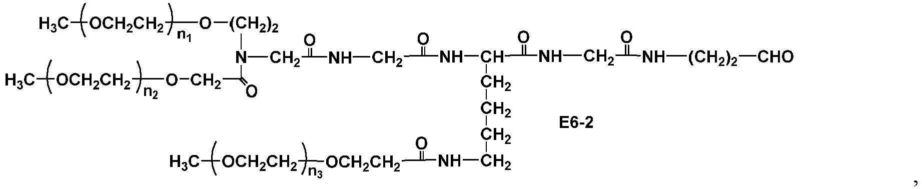 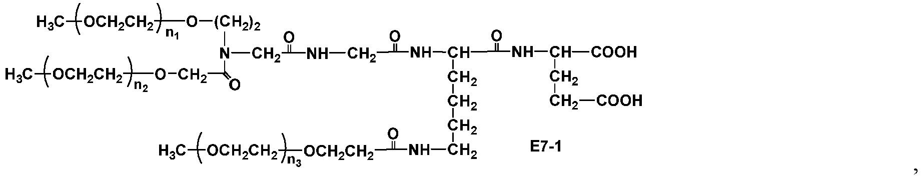 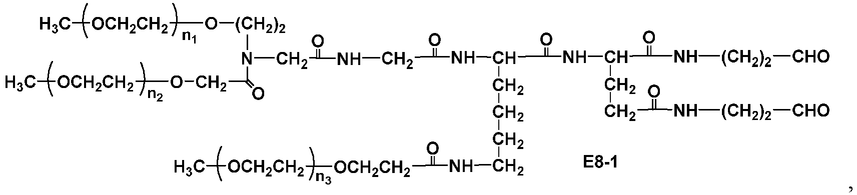 and

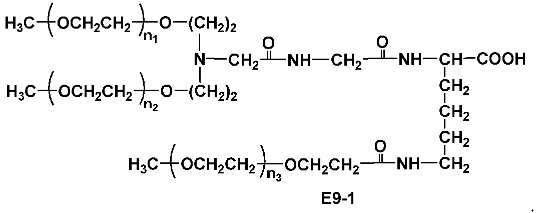

12. The three-arm polyethylene glycol derivative according to claim 7, wherein p is 2 and -(AA₁)₂- is a residue of phenylalanine-glycine dipeptide; the structure of the three-arm polyethylene glycol derivative is represented by general formula (2-B): 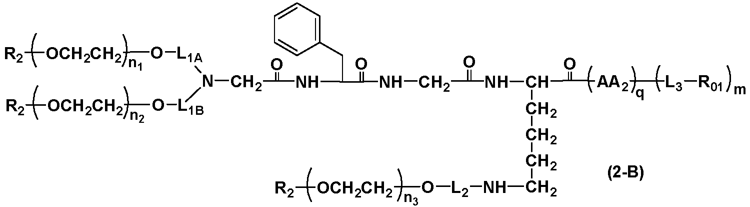 wherein,
L_{1A} and L_{1B} are each independently a C₁₋₆ alkylene group or -(CH₂)₁₋₆C(=O)-, wherein the left end of each linking group is connected to a PEG chain; more preferably, L_{1A} and L_{1B} are each independently selected from the group consisting of -CH₂CH₂-, -CH₂-, -CH₂C(=O)-, and -CH₂CH₂C(=O)-; more preferably, L_{1A} and L_{1B} conform to any one of the following cases:
Case (1): L_{1A} and L_{1B} are each independently -CH₂CH₂- or -CH₂-;
Case (2): one of L_{1A} and L_{1B} is -CH₂CH₂- or -CH₂-, and the other is -CH₂C(=O)- or -CH₂CH₂C(=O)-.

13. A bio-related substance modified with a three-arm polyethylene glycol derivative, having the structure represented by general formula (5):
P-L-D (5)
wherein, **P** is a residue of the three-arm polyethylene glycol derivative of any one of claims **1 to 12, D** is a residue of bio-related substance, and L is a divalent linking group formed by the reacting the functional group of the three-arm polyethylene glycol derivative with the bio-related substance;
the bio-related substance is selected from the group consisting of drugs, proteins, polypeptides, oligopeptides, protein mimics, protein fragments, enzymes, antigens, antibodies, antibody fragments, receptors, aptamers of gene-related substances, polysaccharides, proteoglycans, glycoproteins, lipid compounds, hormones, vitamins, vesicles, liposomes, dyes, fluorescent substances, targeting factors, cytokines, neurotransmitters, extracellular matrix substances, plant or animal extracts, viruses, vaccines, cells, and micelles; preferably, the bio-related substance is selected from the group consisting of small molecule drugs, nucleic acids, steroids, phospholipids, and glycolipids; more preferably, the bio-related substance is selected from the group consisting of small molecule drugs, nucleosides, nucleotides, oligonucleotides, antisense oligonucleotides, polynucleotides, and steroids; furthermore, the small molecule drugs are preferably selected from the group consisting of flavonoids, terpenoids, carotenoids, saponins, steroids, sterols, quinones, anthraquinones, fluoroquinones, coumarins, alkaloids, porphyrins, polyphenols, macrolides, monobactams, phenylpropanoid phenols, anthracyclines, and aminoglycosides.

14. The bio-related substance modified with a three-arm polyethylene glycol derivative according to claim **13,** wherein the bio-related substance is a genetically engineered drug, selected from the group consisting of antibodies, antibody fragments, interleukins, lysozymes, interferons, growth hormones, erythropoietins, and granulocyte colony-stimulating factors, wherein the interferons are preferably α-, β- or γ-interferons.
